# EUROPEAN PATENT APPLICATION

(11) **EP 4 830 719 A2**
(43) Date of publication of application: **28.10.2026**
(21) Application number: 26204963.8
(22) Date of filing: 11.07.2014
(51) Int. Cl.: A61K 39/39

(54) **METHODS FOR TREATING OR PREVENTING OPTHALMOLOGICAL CONDITIONS**

(30) Priority: 12.07.2013 US 201361845938 P; 12.07.2013 US 201361845935 P; 12.07.2013 US 201361845936 P; 15.08.2013 US 201361866502 P; 15.08.2013 US 201361866503 P; 15.08.2013 US 201361866507 P; 04.12.2013 US 201361911854 P; 04.12.2013 US 201361911860 P; 04.12.2013 US 201361911894 P; 13.01.2014 US 201461926848 P; 13.01.2014 US 201461926825 P; 13.01.2014 US 201461926812 P; 24.01.2014 US 201461931116 P; 24.01.2014 US 201461931125 P; 24.01.2014 US 201461931135 P
(62) Document number(s) of the earlier application(s) in accordance with Art. 76 EPC: 19192389.5 / 3 628 373; 14823713.4 / 3 019 243
(71) Applicant: Astellas US LLC, Northbrook, IL 60062 (US)
(72) Inventor: PATEL, Samir, Princeton, 08540 (US)
(74) Representative: Carpmaels & Ransford LLP

(57) **Abstract**

The present invention relates to methods for treating and preventing ophthalmological disease and disorders, comprising administering Antagonist A or another pharmaceutically acceptable salt thereof, optionally in combination with another treatment, to a subject in need thereof. The present invention also relates to methods for treating and preventing ophthalmological disease and disorders, comprising administering an anti-C5 agent (e.g., ARC1905), optionally in combination with another treatment, to a subject in need thereof.

## Description

### CROSS REFERENCE TO RELATED APPLICATIONS

This application claims the benefit of U.S. provisional application nos. 61/845,938, filed July 12, 2013, 61/845,935, filed July 12, 2013, 61/845,936, filed July 12, 2013, 61/866,502, filed August 15, 2013, 61/866,503, filed August 15, 2013, 61/866,507, filed August 15, 2013, 61/911,854, filed December 4, 2013, 61/911,860, filed December 4, 2013, 61/911,894, filed December 4, 2013, 61/926,812, filed January 13, 2014, 61/926,825, filed January 13, 2014, 61/926,848, filed January 13, 2014, 61/931,116, filed January 24, 2014, 61/931,125, filed January 24, 2014, and 61/931,135, filed January 24, 2014, each of which is incorporated by reference herein in its entirety.

### SEQUENCE LISTING

The Sequence Listing associated with this application is provided in text format in lieu of a paper copy, and is hereby incorporated by reference into the specification. The name of the text file containing the Sequence Listing is OPHT_012_06WO_SeqList_ST25.txt. The text file is about 372 KB, was created on July 10, 2014, and is being submitted electronically via EFS-Web.

### FIELD OF THE INVENTION

This invention relates to methods and compositions useful for the treatment or prevention of an ophthalmological disease or disorder, comprising administration of an effective amount of Antagonist A or another pharmaceutically acceptable salt thereof.

### BACKGROUND OF THE INVENTION

Various disorders of the eye are characterized, caused by, or result in choroidal, retinal or iris neovascularization or retinal edema. One of these disorders is macular degeneration. Age-related macular degeneration (AMD) is a disease that affects approximately one in ten Americans over the age of 65. One type of AMD, "wet-AMD," accounts only for approximately 10% of age-related macular degeneration cases but results in approximately 90% of cases of legal blindness from macular degeneration in the elderly. Another disorder of the eye is diabetic retinopathy. Diabetic retinopathy can affect up to 80% of all patients having diabetes for 10 years or more and is the third leading cause of adult blindness, accounting for almost 7% of blindness in the USA. Other disorders include hypertensive retinopathy, central serous chorioretinopathy, cystoid macular edema, Coats disease and ocular or adnexal neoplasms such as choroidal hemangioma, retinal pigment epithelial carcinoma, retinal vein occlusions and intraocular lymphoma.

Therefore, although advances in the understanding of the molecular events accompanying neovascularization have been made, there exists a need to utilize this understanding to develop improved methods for treating or preventing neovascular diseases disorders, including ocular neovascular diseases and disorders such as the neovascularization that occurs with AMD, diabetic retinopathy, and retinal vein occlusions.

### SUMMARY OF THE INVENTION

The present invention relates to methods and compositions useful for the treatment or prevention of an ophthalmological disease or disorder.

The present invention provides a method for treating or preventing wet age-related macular degeneration (wet AMD), comprising administering to a subject in need thereof (a) Antagonist A or another pharmaceutically acceptable salt thereof and (b) an VEGF antagonist, wherein (a) and (b) are administered in an amount that is effective for treating or preventing wet AMD, and wherein the administering occurs once every month, ± about seven days, for a first administration period of at least 3 consecutive months, followed by administering (a) and (b) for a second administration period at a frequency of at least every other month ± about seven days beginning at two months ± about seven days after the day of the last month of the first administration period on which (a) and (b) are administered.

Also provided herein is a method for treating or preventing sub-retinal fibrosis, comprising administering to a subject in need thereof (a) Antagonist A or another pharmaceutically acceptable salt thereof in an amount that is effective for treating or preventing sub-retinal fibrosis.

A method for treating or preventing von Hippel-Lindau (VHL) disease, comprising administering to a subject in need thereof Antagonist A or another pharmaceutically acceptable salt thereof in an amount that is effective for treating or preventing VHL disease is also provided herein.

### BRIEF DESCRIPTION OF THE DRAWINGS

Reference is made to the following detailed description, which sets forth illustrative embodiments and the accompanying drawings of which:
**FIGS. 1A-F** show the chemical structure of Antagonist A, wherein the 5' end of its aptamer (SEQ ID NO: 1) is modified with Me(OCH₂CH₂)ₙOC(O)NH(CH₂)₄CH(NHC(O)O(CH₂CH₂O)ₙMe)C(O)NH(CH₂)₆-, where n is about 450. The designations Ⓑ-Ⓕ indicate a continuation from a previous panel.
**FIG. 2** shows a graph depicting the mean change in visual acuity in wet AMD patients in a phase 2b clinical trial, who were treated with 0.5 mg of Lucentis^{®} alone or with 0.5 mg of Lucentis^{®} and either 1.5 mg of Antagonist A or 0.3 mg of Antagonist A.
**FIG. 3** shows a bar graph showing comparative visual-acuity benefit in wet AMD patients with treatment with 0.5 mg of Lucentis^{®} and either 1.5 mg or 0.3 mg of Antagonist A as compared to treatment with Lucentis^{®} monotherapy (0.5 mg).
**FIG. 4** shows a graph depicting the early and sustained visual-acuity improvement over time in wet AMD patients treated with Lucentis^{®} monotherapy (0.5 mg) or with 0.5 mg of Lucentis^{®} and either 1.5 mg of Antagonist or 0.3 mg of Antagonist A.
**FIGS. 5A and 5A** provide bar graphs showing that the increased efficacy of treatment with 0.5 mg of Lucentis^{®} and either 1.5 mg or 0.3 mg of Antagonist A as compared to treatment with Lucentis^{®} monotherapy (0.5 mg) in patients with wet AMD is independent of baseline lesion size or baseline vision. **FIG. 5A** shows the mean change in visual acuity for patients in each of the indicated baseline lesion quartiles, and **FIG. 5B** shows the mean change in visual acuity for patients with the indicated baseline vision.
**FIGS. 6A and 6B** provide bar graphs showing that the cohort of patients treated with a combination of 0.5 mg of Lucentis^{®} and 1.5 mg of Antagonist A included a greater proportion of patients with significant visual gain (**FIG. 6A**) and fewer patients with visual loss (**FIG. 6B**) as compared to the cohort of patients with treated Lucentis^{®} monotherapy (0.5 mg).
**FIGS. 7A-C** provide bar graphs showing that patients treated with 0.5 mg of Lucentis^{®} and 1.5 mg of Antagonist A exhibited a greater mean improvement in final visual acuity as compared to patients treated with Lucentis^{®} monotherapy (0.5 mg). **FIG. 7A** shows the percentage of patients who demonstrated a visual acuity of 20/40 or better; **FIG. 7B** shows the percentage of patients who demonstrated a visual acuity of 20/25 or better; and **FIG. 7C** shows the percentage of patients who demonstrated a visual acuity of 20/200 or worse.
**FIGS. 8A and 8B** provide bar graphs showing increased reduction in choroidal neovascularization (CNV) lesion size in small and large baseline CNV lesions in wet AMD patients treated with both 0.5 mg of Lucentis^{®} and 1.5 mg of Antagonist A as compared to patients treated with Lucentis^{®} monotherapy (0.5 mg). **FIG. 8A** shows the results in all patients, and **FIG. 8B** shows the results in patients with a visual outcome >3-lines.
**FIG. 9** shows a graph depicting the mean change in geographic atrophy (GA) lesion area in dry AMD patients measured at 24 weeks in patients treated with either a 0.3 mg or 1 mg dose of ARC 1905 monthly from weeks 0 to 24 in a phase 2a trial.
**FIG. 10** shows a graph depicting the mean change in GA lesion area in dry AMD patients measured at 24 weeks and 48 weeks in patients treated with either a 0.3 mg or 1 mg dose of ARC1905 monthly from weeks 0 to 48 in a phase 2a trial.
**FIG. 11** shows Early Treatment for Diabetic Retinopathy Study ("ETDRS") Chart 1.
**FIG. 12** shows Early Treatment for Diabetic Retinopathy Study ("ETDRS") Chart 2.
**FIG. 13** shows Early Treatment for Diabetic Retinopathy Study ("ETDRS") Chart R.

### DETAILED DESCRIPTION OF THE INVENTION

In certain aspects, the present invention provides new and improved methods and compositions for treating and preventing ophthalmological diseases and disorders, including, e.g., new uses, combination therapies, treatment and dosing regimens, and coformulations.

In one aspect, the invention provides methods for treating or preventing an ophthalmological disease or disorder, comprising administering to a subject in need thereof an effective amount of Antagonist A or another pharmaceutically acceptable salt thereof. In particular embodiments, the subject is administered Antagonist A or another pharmaceutically acceptable salt thereof and not administered an anti-C5 agent. In some embodiments, the subject is administered Antagonist A or another pharmaceutically acceptable salt thereof and not administered a VEGF antagonist.

In particular embodiments, the Antagonist A or another pharmaceutically acceptable salt thereof is administered in combination with a VEGF antagonist. In one embodiment, Antagonist A or another pharmaceutically acceptable salt thereof is administered in combination with ranibizumab, bevacizumab, aflibercept, pegaptanib sodium, or ESBA1008.

In particular embodiments, the Antagonist A or another pharmaceutically acceptable salt thereof is administered in combination with a VEGF antagonist and an anti-C5 agent. In one embodiment, Antagonist A or another pharmaceutically acceptable salt thereof is administered in combination with a VEGF antagonist (e.g., ranibizumab, bevacizumab, aflibercept, pegaptanib sodium, or ESBA 1008), and ARC1905.

The invention also provides treatment regimens, including treatment and dosing regimens, related to the coadministration of Antagonist A or another pharmaceutically acceptable salt thereof and a VEGF antagonist, optionally also in combination with an anti-C5 agent.

In further embodiments, another agent (e.g., an agent that is not Antagonist A, VEGF antagonist or an anti-C5 agent) that is useful for treating or preventing an ophthalmological disease or disorder is administered. In some embodiments, the methods comprise administering one or more (e.g., two) VEGF antagonists and/or one or more (e.g., two) anti-C5 agents to the subject in need thereof.

In another aspect, the invention provides methods for treating or preventing an ophthalmological disease or disorder, comprising administering to a subject in need thereof an effective amount of an anti-C5 agent (e.g., ARC1905). In particular embodiments, the subject is not administered Antagonist A or another pharmaceutically acceptable salt thereof. In some embodiments, the subject is not administered a VEGF antagonist.

In addition, the invention provides coformulations that comprise Antagonist A or another pharmaceutically acceptable salt thereof and a VEGF antagonist. In certain embodiments, the coformulations further comprise an anti-C5 agent. In certain embodiments, the coformulations are pharmaceutically compositions comprising an effective amount of Antagonist A or another pharmaceutically acceptable salt thereof and VEGF antagonist, and a pharmaceutically acceptable carrier or vehicle. In certain embodiments, the coformulations are pharmaceutically compositions comprising an effective amount of Antagonist A or another pharmaceutically acceptable salt thereof, VEGF antagonist, and anti-C5 agent, and a pharmaceutically acceptable carrier or vehicle.

In one embodiment, the present invention provides methods for treating or preventing an ophthalmological disease or disorder, comprising administering to a subject in need thereof Antagonist A or another pharmaceutically acceptable salt thereof and optionally a VEGF antagonist, wherein the methods further comprise performing a surgery to treat the ophthalmological disease or disorder and/or administration of an anti-C5 agent.

### Definitions and Abbreviations

As used herein, the following terms and phrases shall have the meanings set forth below. Unless defined otherwise, all technical and scientific terms used herein have the same meaning as commonly understood to one of skill in the art to which this invention belongs.

The term "about" when used in connection with a referenced numeric indication means the referenced numeric indication plus or minus up to 10% of that referenced numeric indication. For example, "about 100" means from 90 to 110 and "about six" means from 5.4 to 6.6.

The term "antagonist" refers to an agent that inhibits, either partially or fully, the activity or production of a target molecule. In particular, the term "antagonist," as applied selectively herein, means an agent capable of decreasing levels of gene expression, mRNA levels, protein levels or protein activity of the target molecule. Illustrative forms of antagonists include, for example, proteins, polypeptides, peptides (such as cyclic peptides), antibodies or antibody fragments, peptide mimetics, nucleic acid molecules, antisense molecules, ribozymes, aptamers, RNAi molecules, and small organic molecules. Illustrative non-limiting mechanisms of antagonist inhibition include repression of ligand synthesis and/or stability (*e.g*., using, antisense, ribozymes or RNAi compositions targeting the ligand gene/nucleic acid), blocking of binding of the ligand to its cognate receptor (*e*.*g*., using anti-ligand aptamers, antibodies or a soluble, decoy cognate receptor), repression of receptor synthesis and/or stability (*e.g*., using, antisense, ribozymes or RNAi compositions targeting the ligand receptor gene/nucleic acid), blocking of the binding of the receptor to its cognate receptor (*e.g*., using receptor antibodies) and blocking of the activation of the receptor by its cognate ligand (*e.g*., using receptor tyrosine kinase inhibitors). In addition, the antagonist may directly or indirectly inhibit the target molecule.

The term "antibody fragment" includes a portion of an antibody that is an antigen binding fragment or single chains thereof. An antibody fragment can be a synthetically or genetically engineered polypeptide. Examples of binding fragments encompassed within the term "antigen-binding portion" of an antibody include (i) a Fab fragment, a monovalent fragment consisting of the V_{L}, V_{H}, C_{L} and C_{H1} domains; (ii) a F(ab')₂ fragment, a bivalent fragment comprising two Fab fragments linked by a disulfide bridge at the hinge region; (iii) a Fd fragment consisting of the V_{H} and C_{H1} domains; (iv) a Fv fragment consisting of the V_{L} and V_{H} domains of a single arm of an antibody, (v) a dAb fragment (Ward et al., (1989) Nature 341:544-546), which consists of a V_{H} domain; and (vi) an isolated complementarity determining region (CDR). Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding fragment" of an antibody. These antibody fragments are obtained using conventional techniques known to those in the art, and the fragments can be screened for utility in the same manner as whole antibodies.

The term "aptamer" refers to a peptide or nucleic acid that has an inhibitory effect on a target. Inhibition of the target by the aptamer can occur by binding of the target, by catalytically altering the target, by reacting with the target in a way which modifies the target or the functional activity of the target, by ionically or covalently attaching to the target as in a suicide inhibitor or by facilitating the reaction between the target and another molecule. Aptamers can be peptides, ribonucleotides, deoxyribonucleotides, other nucleic acids or a mixture of the different types of nucleic acids. Aptamers can comprise one or more modified amino acid, bases, sugars, polyethylene glycol spacers or phosphate backbone units as described in further detail herein.

A nucleotide sequence is "complementary" to another nucleotide sequence if each of the bases of the two sequences matches, *i.e.,* are capable of forming Watson Crick base pairs. The complement of a nucleic acid strand can be the complement of a coding strand or the complement of a non-coding strand.

The phrase "conserved residue" refers to an amino acid of a group of amino acids having particular common properties. A functional way to define common properties among individual amino acids is to analyze the normalized frequencies of amino acid changes among corresponding proteins of homologous organisms. According to such analyses, groups of amino acids may be characterized where amino acids within a group exchange preferentially with each other, and therefore resemble each other most in their impact on the overall protein structure (Schulz, G. E. and R. H. Schinner, Principles of Protein Structure, Springer-Verlag). Examples of amino acid groups defined in this manner include:
(i) a charged group, consisting of Glu and Asp, Lys, Arg and His,
(ii) a positively-charged group, consisting of Lys, Arg and His,
(iii) a negatively-charged group, consisting of Glu and Asp,
(iv) an aromatic group, consisting of Phe, Tyr and Trp,
(v) a nitrogen ring group, consisting of His and Trp,
(vi) a large aliphatic nonpolar group, consisting of Val, Leu and Ile,
(vii) a slightly-polar group, consisting of Met and Cys,
(viii) a small-residue group, consisting of Ser, Thr, Asp, Asn, Gly, Ala, Glu, Gln and Pro,
(ix) an aliphatic group consisting of Val, Leu, Ile, Met and Cys, and
(x) a small hydroxyl group consisting of Ser and Thr.

Members of each of the above groups are conserved residues.

The term "label" includes, but is not limited to, a radioactive isotope, a fluorophore, a chemiluminescent moiety, an enzyme, an enzyme substrate, an enzyme cofactor, an enzyme inhibitor, a dye, a metal ion, a ligand (e.g., biotin or a hapten) and the like. Examples of fluorophore labels include fluorescein, rhodamine, dansyl, umbelliferone, Texas red, luminol, NADPH, alpha-beta-galactosidase and horseradish peroxidase.

The term "nucleic acid" refers to a polynucleotide such as deoxyribonucleic acid (DNA) or ribonucleic acid (RNA). The term also includes analogs of RNA or DNA made from nucleotide analogs, and, as applicable to the embodiment being described, single (sense or antisense) and double-stranded polynucleotides, ESTs, chromosomes, cDNAs, mRNAs, and rRNAs.

The terms "RNA interference," "RNAi," "miRNA," and "siRNA" refer to any method by which expression of a gene or gene product is decreased by introducing into a target cell one or more double-stranded RNAs, which are homologous to a gene of interest (particularly to the messenger RNA of the gene of interest, *e.g.,* PDGF or VEGF).

The term "neovascularization" refers to new blood vessel formation in abnormal tissue or in abnormal positions.

The term "angiogenesis" refers to formation of new blood vessels in normal or in abnormal tissue or positions.

The term "ophthalmological disease" includes diseases of the eye and the ocular adnexa.

The term "ocular neovascular disorder" refers to an ocular disorder characterized by neovascularization. In one embodiment, the ocular neovascular disorder is a disorder other than cancer. Examples of ocular neovascular disorders include diabetic retinopathy and age-related macular degeneration.

The term "mammal" includes a human, monkey, cow, hog, sheep, horse, dog, cat, rabbit, rat and mouse. In certain embodiments, a subject is a mammal.

The term "PDGF" refers to a platelet-derived growth factor that regulates cell growth or division. As used herein, the term "PDGF" includes the various subtypes of PDGF including PDGF-B (see SEQ ID NOS: 2 (nucleic acid) and 3 (polypeptide)), PDGF-A (see SEQ ID NOS: 4 (nucleic acid) and 5 (polypeptide), PDGF-C (see SEQ ID NOS: 6 (nucleic acid) and 7 (polypeptide)), PDGF-D, variants 1(see SEQ ID NOS: 8 (nucleic acid) and 9 (polypeptide )) and 2 (see SEQ ID NOS: 10 (nucleic acid) and 11 (polypeptide)), and dimerized forms thereof, including PDGF-AA, PDGF-AB, PDGF-BB, PDGF-CC, and PDGF-DD. Platelet derived growth factors includes homo- or heterodimers of A-chain (PDGF-A) and B-chain (PDGF-B) that exert their action via binding to and dimerization of two related receptor tyrosine kinase platelet-derived growth factor cell surface receptors (*i*.*e*., PDGFRs), PDGFR-α (see SEQ ID NOS: 12 (nucleic acid) and 13 (polypeptide)) and PDGFR-β (see SEQ ID NOS: 14 (nucleic acid) and 15 (polypeptide)). In addition, PDGF-C and PDGF-D, two additional protease-activated ligands for the PDGFR complexes, have been identified (Li et al., (2000) Nat. Cell. Biol. 2: 302-9; Bergsten et al., (2001) Nat. Cell. Biol. 3: 512-6; and Uutele et al., (2001) Circulation 103: 2242-47). Due to the different ligand binding specificities of the PDGFRs, it is known that PDGFR-α/α binds PDGF-AA, PDGF-BB, PDGF-AB, and PDGF-CC; PDGFR-β/β binds PDGF-BB and PDGF-DD; whereas PDGFR-α/β binds PDGF-AB, PDGF-BB, PDGF-CC, and PDGF-DD (Betsholtz et al., (2001) BioEssays 23: 494-507). As used herein, the term "PDGF" also refers to those members of the class of growth factors that induce DNA synthesis and mitogenesis through the binding and activation of a PDGFR on a responsive cell type. PDGFs can effect, for example: directed cell migration (chemotaxis) and cell activation; phospholipase activation; increased phosphatidylinositol turnover and prostaglandin metabolism; stimulation of both collagen and collagenase synthesis by responsive cells; alteration of cellular metabolic activities, including matrix synthesis, cytokine production, and lipoprotein uptake; induction, indirectly, of a proliferative response in cells lacking PDGF receptors; and potent vasoconstrictor activity. The term "PDGF" can be used to refer to a "PDGF" polypeptide, a "PDGF" encoding gene or nucleic acid, or a dimerized form thereof.

The term "PDGF-A" refers to an A chain polypeptide of PDGF or its corresponding encoding gene or nucleic acid.

The term "PDGF-B" refers to a B chain polypeptide of PDGF or its corresponding encoding gene or nucleic acid.

The term "PDGF-C" refers to a C chain polypeptide of PDGF or its corresponding encoding gene or nucleic acid.

The term "PDGF-D" refers to a D chain polypeptide of PDGF or its corresponding encoding gene or nucleic acid, including variants 1 and 2 of the D chain polypeptide of PDGF.

The term "PDGF-AA" refers to a dimer having two PDGF-A chain polypeptides.

The term "PDGF-AB" refers to a dimer having one PDGF-A chain polypeptide and one PDGF-B chain polypeptide.

The term "PDGF-BB" refers to a dimer having two PDGF-B chain polypeptides.

The term "PDGF-CC" refers to a dimer having two PDGF-C chain polypeptides.

The term "PDGF-DD" refers to a dimer having two PDGF-D chain polypeptides.

The term "VEGF" refers to a vascular endothelial growth factor that induces angiogenesis or an angiogenic process. As used herein, the term "VEGF" includes the various subtypes of VEGF (also known as vascular permeability factor (VPF) and VEGF-A) (see SEQ ID NOS: 16 (nucleic acid) and 17 (polypeptide)) that arise by, *e.g.,* alternative splicing of the VEGF-A/VPF gene including VEGF₁₂₁, VEGF₁₆₅ and VEGF₁₈₉. Further, as used herein, the term "VEGF" includes VEGF-related angiogenic factors such as PIGF (placenta growth factor), VEGF-B, VEGF-C, VEGF-D and VEGF-E, which act through a cognate VEFG receptor (*i.e*., VEGFR) to induce angiogenesis or an angiogenic process. The term "VEGF" includes any member of the class of growth factors that binds to a VEGF receptor such as VEGFR-1 (Flt-1) (see SEQ ID NOS: 18 (nucleic acid) and 19 (polypeptide)), VEGFR-2 (KDR/Flk-1) (see SEQ ID NOS: 20 (nucleic acid) and 21 (polypeptide)), or VEGFR-3 (FLT-4). The term "VEGF" can be used to refer to a "VEGF" polypeptide or a "VEGF" encoding gene or nucleic acid.

The term "PDGF antagonist" refers to an agent that reduces, or inhibits, either partially or fully, the activity or production of a PDGF. In certain embodiments, the PDGF antagonist inhibits one or more of PDGF-A, PDGF-B, PDGF-C and PDGF-D. In certain embodiments, the PDGF antagonist inhibits one or more of PDGF-A, PDGF-B, and PDGF-C. In some embodiments, the PDGF antagonist inhibits a dimerized form of PDGF, such as PDGF-AA, PDGF-AB, PDGF-BB, PDGF-CC, and PDGF-DD. In certain embodiments, the PDGF antagonist inhibits PDGF-BB. In other embodiments, the PDGF antagonist inhibits PDGF-AB. A PDGF antagonist can directly or indirectly reduce or inhibit the activity or production of a specific PDGF such as PDGF-B. Furthermore, "PDGF antagonists" consistent with the above definition of "antagonist," include agents that act on a PDGF ligand or its cognate receptor so as to reduce or inhibit a PDGF-associated receptor signal. Examples of "PDGF antagonists" include antisense molecules, ribozymes or RNAi that target a PDGF nucleic acid; anti-PDGF aptamers, anti-PDGF antibodies to PDGF itself or its receptor, or soluble PDGF receptor decoys that prevent binding of a PDGF to its cognate receptor; antisense molecules, ribozymes or RNAi that target a cognate PDGF receptor (PDGFR) nucleic acid; anti-PDGFR aptamers or anti-PDGFR antibodies that bind to a cognate PDGFR receptor; and PDGFR tyrosine kinase inhibitors.

The term "VEGF antagonist" refers to an agent that reduces, or inhibits, either partially or fully, the activity or production of a VEGF. In certain embodiments, the VEGF antagonist inhibits one or more of VEGF-A, VEGF-B, VEGF-C and VEGF-D. A VEGF antagonist can directly or indirectly reduce or inhibit the activity or production of a specific VEGF such as VEGF₁₆₅. Furthermore, "VEGF antagonists" consistent with the above definition of "antagonist," include agents that act on either a VEGF ligand or its cognate receptor so as to reduce or inhibit a VEGF-associated receptor signal. Examples of "VEGF antagonists" include antisense molecules, ribozymes or RNAi that target a VEGF nucleic acid; anti-VEGF aptamers, anti-VEGF antibodies to VEGF itself or its receptor, or soluble VEGF receptor decoys that prevent binding of a VEGF to its cognate receptor; antisense molecules, ribozymes, or RNAi that target a cognate VEGF receptor (VEGFR) nucleic acid; anti-VEGFR aptamers or anti-VEGFR antibodies that bind to a cognate VEGFR receptor; and VEGFP, tyrosine kinase inhibitors. In certain embodiments, the VEGF antagonist is a peptide, e.g., a peptide comprising three or more amino acid residues. In certain embodiments, the VEGF antagonist is a bicyclic peptide.

The term "effective amount" when used in connection with an active agent, refers to an amount of the active agent, e.g., a PDGF antagonist, a VEGF antagonist or an anti-C5 agent, alone or in combination with another active agent, that is useful to treat or prevent an ophthalmological disease or disorder. The "effective amount" can vary depending upon the mode of administration, specific locus of the ophthalmological disease or disorder, the age, body weight, and general health of the subject. The effective amount of two or more active agents is the combined amount of the active agents that is useful for treating or preventing an ophthalmological disease or disorder, even if the amount of one of the agents, in the absence of one or more of the other agents, is ineffective to treat or prevent the ophthalmological disease or disorder.

A "variant" of polypeptide X refers to a polypeptide having the amino acid sequence of polypeptide X in which is altered in one or more amino acid residues. The variant can have "conservative" changes, wherein a substituted amino acid has similar structural or chemical properties (e.g., replacement of leucine with isoleucine). More rarely, a variant can have "nonconservative" changes (e.g., replacement of glycine with tryptophan). Analogous minor variations may also include amino acid deletions or insertions, or both. Guidance in determining which amino acid residues may be substituted, inserted, or deleted without eliminating biological or immunological activity can be determined using computer programs well known in the art, for example, LASERGENE software (DNASTAR).

The term "variant," when used in the context of a polynucleotide sequence, can encompass a polynucleotide sequence related to that of gene or the coding sequence thereof. This definition also includes, for example, "allelic," "splice," "species," or "polymorphic" variants. A splice variant can have significant identity to a reference molecule, but will generally have a greater or lesser number of polynucleotides due to alternative splicing of exons during mRNA processing. The corresponding polypeptide can possess additional functional domains or an absence of domains. Species variants are polynucleotide sequences that vary from one species to another. The resulting polypeptides generally will have significant amino acid identity relative to each other. A polymorphic variant is a variation in the polynucleotide sequence of a particular gene between individuals of a given species.

The term "anti-C5 agent" refers to an agent that reduces, or inhibits, either partially or fully, the activity or production of a C5 complement protein or a variant thereof. An anti-C5 agent can directly or indirectly reduce or inhibit the activity or production of a C5 complement protein or variant thereof. An anti-C5 agent can reduce or inhibit the conversion of C5 complement protein into its component polypeptides C5a and C5b. Anti-C5 agents can also reduce or inhibit the activity or production of C5a and/or C5b. Examples of "anti-C5 agents" include antisense molecules, ribozymes or RNAi that target a C5 nucleic acid; anti-C5 aptamers including anti-C5a and anti-C5b aptamers, anti-C5 antibodies directed against C5, C5a, C5b, or C5b-9, or soluble C5 receptor decoys that prevent binding of a C5 complement protein or variant or fragment thereof (e.g., C5a or C5b) to a binding partner or receptor.

### Agents Useful for Treatment or Prevention of an Opthalmological Disease or Disorder

### Antagonist A

Antagonist A is a PEGylated, anti-PDGF aptamer having the sequence CAGGCUACGC GTAGAGCAUC ATGATCCUGT (SEQ ID NO: 1) (see Example 3 of US Patent Application Publication No. 20050096257, incorporated herein by reference in its entirety) having 2'-fluoro-2'-deoxyuridine at positions 6, 19 and 28; 2'-fluoro-2'-deoxycytidine at positions 8, 20, 26, and 27; 2'-O-Methyl-2'-deoxyguanosine at positions 9, 14, 16, and 29; 2'-O-Methyl-2'-deoxyadenosine at position 21; an inverted orientation T (i.e., 3'-3'-linked) at position 30; and two heaxethylene-glycol phosphoramidite linkages that join together the 9^{th} and 10^{th} nucleotides and 21^{st} and 22^{nd} nucleotides via phosphodiester linkages between the linker and the respective nucleotides.

The chemical name of Antagonist A is [(monomethoxy 20K polyethylene glycol carbamoyl-N2-) (monomethoxy 20K polyethylene glycol carbamoyl-N6-)]-lysine-amido-6-hexandilyl-(1-5')-2'-deoxycytidylyl-(3'-5')-2'-deoxyadenylyl-(3'-5')-2'-deoxyguanylyl-(3'-5')-2'-deoxyguanylyl-(3'-5')-2'-deoxycytidylyl-(3'-5')-2'-deoxy-2'-fluorouridylyl-(3'-5')-2'-deoxyadenylyl-(3'-5')-2'-deoxy-2'-fluorocytidylyt-(3'-5')-2'-deoxy-2'-methoxyguanylyl-(3'-1)-PO₃-hexa(ethytoxy)-(18-5')-2'-deoxycytidylyl-(3' -5')-2'-deoxyguanylyl-(3'-5')-thymidylyl-(3'-5')-2'-deoxyadenylyl-(3'-5')-2'-deoxy-2'-methoxyguanylyl-(3'-5')-2'-deoxyadenylyl-(3'-5')-2'-deoxy-2'-methoxyguanylyl-(3'-5')-2'-deoxycytidylyl-(3'-5')-2'-deoxyadenylyl-(3'- 5')-2'-deoxy-2'-fluorouridylyl-(3'-5')-2'-deoxy-2'-fluorocytidylyl-(3'-5')-2'-deoxy-2'-methoxyadenylyl-(3'-1)-PO₃-hexa(ethyloxy)-(18-5')-thymidylyl-(3'-5')-2'-deoxyguanylyl-(3'-5')-2'-deoxyadenylyl-(3'-5')-thymidylyl-(3'-5')-2'-deoxy-2'-fluorocytidylyl-(3'-5')-2'-deoxy-2'-fluorocytidylyl-(3'-5')-2'-deoxy-2'-fluorouridylyl-(3'-5')-2'-deoxy-2'-methoxyguanylyl-(3'-3')-thymidine.

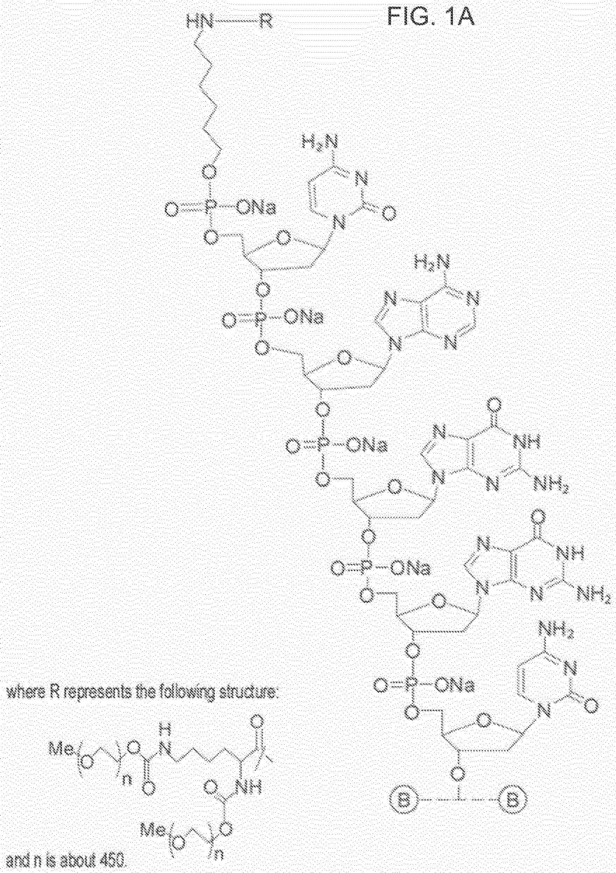

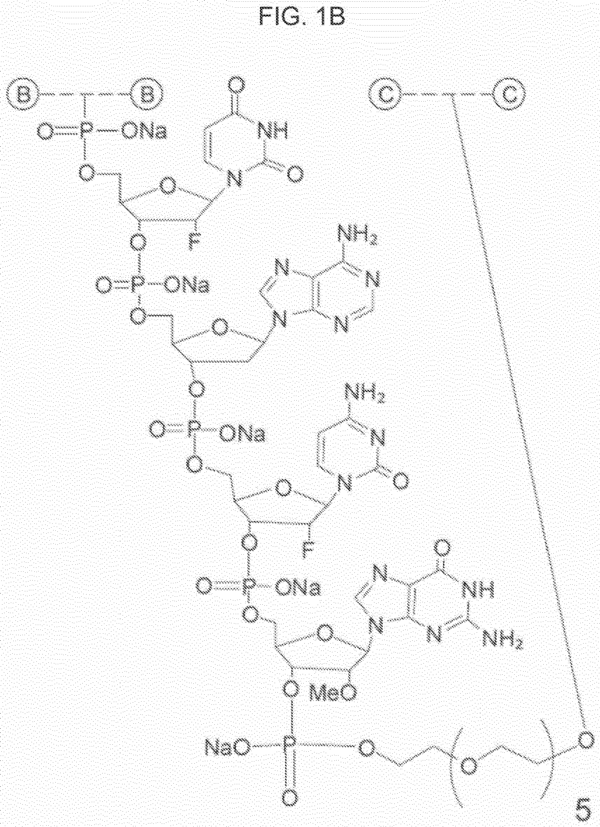

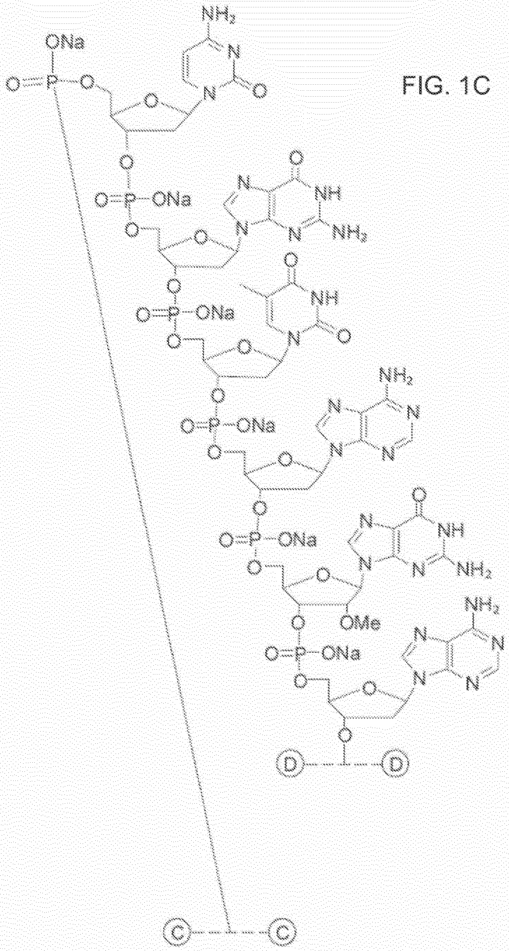

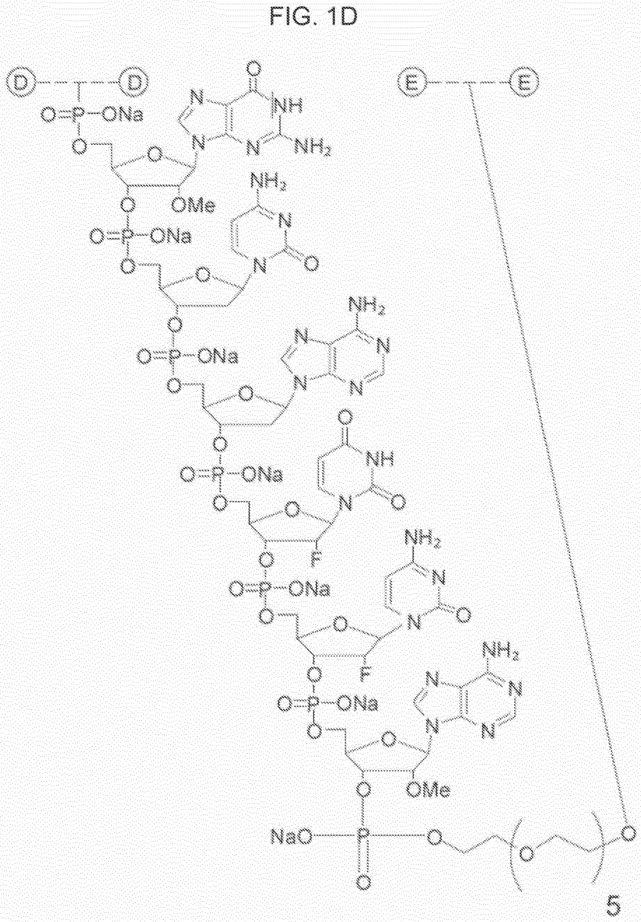

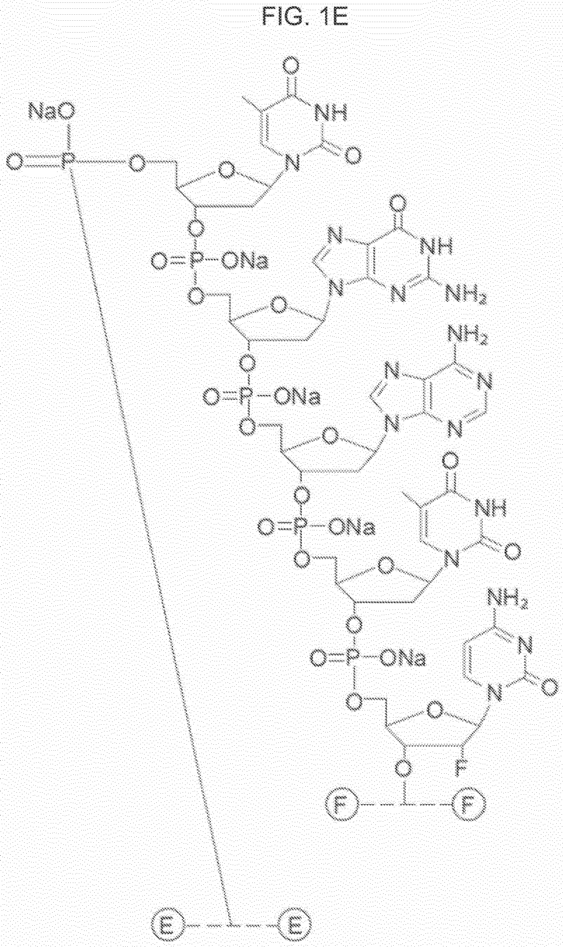

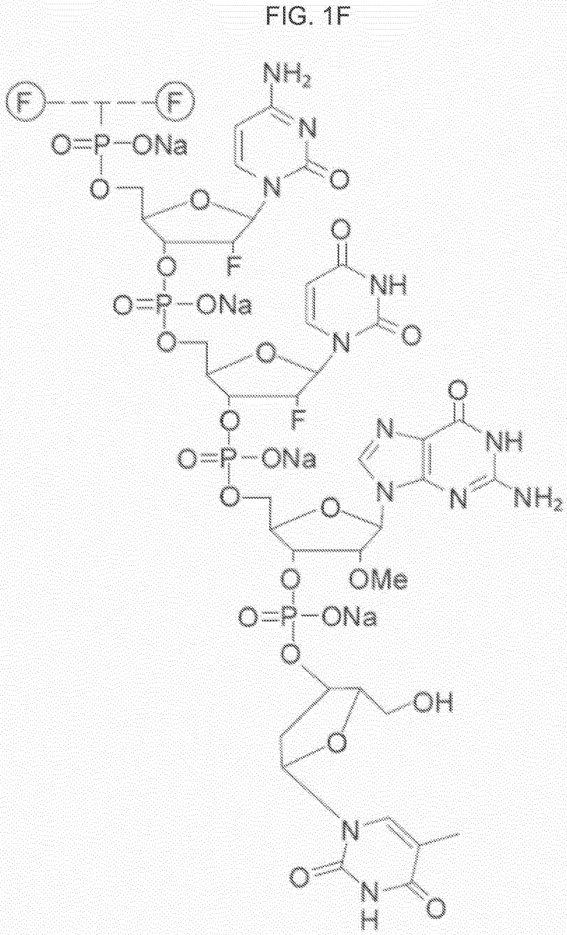

The structure of Antagonist A is shown in **Figure 1****.**

The sequence of Antagonist A is:
5'-[mPEG2 40kD]- [HN-(CH₂)(₆O]CAGGCU_{f}AC_{f}Gₘ[PO₃(CH₂CH₂O)₆] CGTAGₘAGₘCAU_{f}C_{f}Aₘ [PO₃(CH₂CH₂O)₆]TGATC_{f}C_{f}U_{f}Gₘ-[3T]-3', whose aptamer sequence is set forth in (SEQ ID NO: 1),
where [3T] refers to an inverted thymidine nucleotide that is attached to the 3' end of the oligonucleotide at the 3' position on the ribose sugar, and [mPEG2 40 kD] represents two 20 kD polyethylene glycol (PEG) polymer chains, in one embodiment two about 20 kD PEG polymer chains, that are covalently attached to the two amino groups of a lysine residue via carbamate linkages. This moiety is in turn linked with the oligonucleotide via the amino linker described below.

[UN-(CH₂)₆O] represents a bifunctional α-hydroxy-ω-amino linker that is covalently attached to the PEG polymer via an amide bond. The linker is attached to the oligonucleotide at the 5'-end of Antagonist A by a phosphodiester linkage.

[PO₃(CH₂CH₂O)₆] represents the hexaethylene glycol (HEX) moieties that join segments of the oligonucleotide via phosphodiester linkages. Antagonist A has two HEX linkages that join together the 9^{th} and 10^{th} nucleotides and 21^{st} and 22^{nd} nucleotides via phosphodiester linkages between the linker and the respective nucleotides.

C, A, G, and T represent the single letter code for the 2'-deoxy derivatives of cytosine, adenosine, guanosine, and thymidine nucleic acids, respectively. Antagonist A has four 2'-deoxyribocytosine, six 2'-deoxyriboadenosine, four 2'-deoxyriboguanosine, and four 2'-deoxyribothymidine.

Gₘ and Aₘ represent 2'-methoxy substituted forms of guanosine and adenosine, respectively. Antagonist A has four 2'-methoxyguanosines and one 2'-methoxyadenosine. C_{f} and U_{f} represent the 2'-fluoro substituted forms of cytosine and uridine, respectively. Antagonist A has four 2'-fluorocytosines and three 2'-fluorouridines.

The phosphodiester linkages in the oligonucleotide, with the exception of the 3'-terminus, connect the 5'- and 3'-oxygens of the ribose ring with standard nucleoside phosphodiester linkages. The phosphodiester linkage between the 3'-terminal thymidine and the penultimate Gₘ links their respective 3'-oxygens, which is referred to as the 3',3'-cap.

Antagonist A has a molecular weight from 40,000 to 60,000 Daltons, in one embodiment from about 40,000 to about 60,000 Daltons, and can be colorless to slightly yellow in solution. Antagonist A can be present in a solution of monobasic sodium phosphate monohydrate and dibasic sodium phosphate heptahydrate as buffering agents and sodium chloride as a tonicity adjuster. Antagonist A is a hydrophilic polymer. The Antagonist A is soluble in water and in phosphate-buffered saline (PBS), as assessed by visual inspection, to at least 50 mg (based on oligonucleotide weight)/mL solution.

Antagonist A can be synthesized using an iterative chemical synthesis procedure to produce the oligonucleotide portion, which is then covalently bonded to a pegylation reagent, as further described in Example 4 of US Patent Publication NO. 2012/0100136.

Antagonist A is a persodium salt. Other pharmaceutically acceptable salts, however, of Antagonist are useful in the compositions and methods disclosed herein.

### VEGF Antagonists

In some embodiments, the VEGF antagonist is ranibizumab (commercially available under the trademark Lucentis^{®} (Genentech, San Francisco, CA); see Figure 1 of U.S. Pat. No. 7,060,269 for the heavy chain and light chain variable region sequences), bevacizumab (commercially available under the trademark Avastin^{®} (Genentech, San Francisco, CA); see Figure 1 of U.S. Pat. No. 6,054,297 for the heavy chain and light chain variable region sequences), aflibercept (commercially available under the trademark Eylca^{®} (Regeneron, Tarrytown, NY), KH902 VEGF receptor-Fe fusion protein (see Zhang et al. (2008) Mol Vis. 14:37-49), 2C3 antibody (see U.S. Pat. No. 6,342,221, Column 8, lines 48-67, Column 9, lines 1-21), ORA102 (available from Ora Bio, Ltd.), pegaptanib (e.g., pegaptanib sodium; commercially available under the trademark Macugen^{®} (Valeant Pharmaceuticals, Bridgewater, NJ; see Figure 1 of U.S. Pat. No. 6,051,698)), bevasiranib (see Dejneka et al. (2008) Mol Vis. 14:997-1005), SIRNA-027 (Shen et al. (2006) Gene Ther. 13:225-34), decursin (see U.S. Pat. No. 6,525,089 (Column 3, lines 5-16)), decursinol (see Ahn et al. (1997) Planta Med. 63:360-1), picropodophyllin (see Economou (2008) Investigative Ophthalmology & Visual Science. 49:2620-6), guggulsterone (see Kim et al. (2008) Oncol. Rep. 20:1321-7), PLG101 (see Ahmadi and Lim (2008) Expert Opin Pharmacother. 9:3045-52), PLG201 (see Ahmadi and Lim (2008)), eicosanoid LXA4 (see Baker et al (2009) J Immun. 182:3819-26), PTK787 (commercially available under the trademark Vitalanib^{™}; see Barakat and Kaiser (2009) Expert Opin Investig Drugs 18:637-46), pazopanib (see Takahashi et al. (2009) Arch Ophthalmol. 127:494-9), axitinib (see Hu-Lowe et al. (2008) Clin Cancer Res. 14:7272-83), CDDO-Me (see Sogno et al. (2009) Recent Results Cancer Res. 181:209-12), CDDO-Imm (see Sogno et al. (2009)), shikonin (see Hisa et al. (1998) Anticancer Res. 18:783-90), beta-hydroxyisovalerylshikonin (see Hisa et al. (1998)), ganglioside GM3 (Chung et al. (2009) Glycobio. 19:229-39), DC101 antibody (see U.S. Patent No. 6,448,077, Column 2, lines 61-65), Mab25 antibody (see U.S. Patent No. 6,448,077, Column 2, lines 61-65), Mab73 antibody (see U.S. Patent No. 6,448,077, Column 2, lines 61-65), 4A5 antibody (see U.S. Patent No. 6,383,484, Column 12, lines 50-54), 4E10 antibody (see U.S. Patent No. 6,383,484, Column 10, lines 66-67, Column 11, lines 1-2), 5F12 antibody (see U.S. Patent No. 6,383,484, Column 10, lines 62-65), VA01 antibody (see U.S. Patent No. 5,730,977, Column 6, lines 26-30), BL2 antibody (U.S. Patent No. 5,730,977, Column 6, lines 30-32), VEGF-related protein (see U.S. Patent No. 6,451,764, Figure 1), sFLT01 (see Pechan et al. (2009) Gene Ther. 16:10-6), sFLT02 (see Pechan et al. (2009)), Peptide B3 (see Lacal et al. (2008) Eur J Cancer 44:1914-21), TG100801 (see Palanki et al. (2008) J Med Chem. 51:1546-59), sorafenib (commercially available under the trademark Nexavar^{™}; see Kernt et al. (2008) Acta Ophthalmol. 86:456-8), G6-31 antibody (see Crawford et al. (2009) Cancer Cell 15:21-34), ESBA1008 (see U.S. Pat. No. 8,349,322), tivozanib (see U.S. Pat. No. 6,821,987, incorporated by reference in its entirety; Campas et al. (2009) Drugs Fut 2009, 34(10): 793), or a pharmaceutically acceptable salt thereof
In another embodiment, the VEGF antagonist is an antibody or an antibody fragment which binds to an epitope VEGF-A (SEQ ID NO: 22) or VEGF-B (SEQ ID NO: 23), or any portion of the epitopes. In one embodiment, the VEGF antagonist is an antibody or antibody fragment that binds to one or more of an epitope of VEGF (e.g., SEQ ID NOS: 22 and 23). In another embodiment, the VEGF antagonist is an antibody or an antibody fragment which binds to an epitope of VEGF, such as an epitope of VEGF-A, VEGF-B, VEGF-C, VEGF-D, or VEGF-E. In some embodiments, the VEGF antagonist binds to an epitope of VEGF such that binding of VEGF and VEGFR are inhibited. In one embodiment, the epitope encompasses a component of the three dimensional structure of VEGF that is displayed, such that the epitope is exposed on the surface of the folded VEGF molecule. In one embodiment, the epitope is a linear amino acid sequence from VEGF.

In some embodiments, an inhibitory antibody directed against VEGF is known in the art, e.g., those described in U.S. Patent Nos. 6,524,583, 6,451,764 (VRP antibodies), 6,448,077, 6,416,758, 6,403,088 (to VEGF-C), 6,383,484 (to VEGF-D), 6,342,221 (anti-VEGF antibodies), 6,342,219 6,331,301 (VEGF-B antibodies), and 5,730,977, and PCT publications WO96/30046, WO 97/44453, and WO 98/45331, the contents of which are incorporated by reference in their entirety.

Other non-antibody VEGF antagonists include antibody mimetics (e.g., Affibody^{®} molecules, affilins, affitins, anticalins, avimers, Kunitz domain peptides, and monobodies) with VEGF antagonist activity. This includes recombinant binding proteins comprising an ankyrin repeat domain that binds VEGF-A and prevents it from binding to VEGFR-2. One example is MP0112, also known as AGN 150998 (DARPin^{®}). The ankyrin binding domain may have an amino acid sequence of SEQ ID NO: 97.

Recombinant binding proteins comprising an ankyrin repeat domain that binds VEGF-A and prevents it from binding to VEGFR-2 are described in more detail in WO2010/060748 and WO2011/135067.

Further specific antibody mimetics with VEGF antagonist activity are the 40 kD pegylated anticalin PRS-050 and the monobody angiocept (CT-322).

The aforementioned non-antibody VEGF antagonist may be modified to further improve their pharmacokinetic properties or bioavailability. For example, a non-antibody VEGF antagonist may be chemically modified (e.g., pegylated) to extend its in vivo half-life. Alternatively or in addition, it may be modified by glycosylation or the addition of further glycosylation sites not present in the protein sequence of the natural protein from which the VEGF antagonist was derived.

Other non-antibody VEGF antagonist immunoadhesin currently in pre-clinical development is a recombinant human soluble VEGF receptor fusion protein similar to VEGF-trap containing extracellular ligand-binding domains 3 and 4 from VEGFR2/KDR, and domain 2 from VEGFR1/Flt-1; these domains are fused to a human IgG Fc protein fragment (Li et al., 2011 Molecular Vision 17:797-803). This antagonist binds to isoforms VEGF-A. VEGF-B and VEGF-C. The molecule is prepared using two different production processes resulting in different glycosylation patterns on the final proteins. The two glycoforms are referred to as KH902 (conbercept) and KH906. The fusion protein can have the amino acid sequence of SEQ ID NO: 98 and, like VEGF-trap, can be present as a dimer. This fusion protein and related molecules are further characterized in EP1767546.

### Anti-C5 Agents

In certain embodiments, the anti-C5 agent modulates a function of a C5 complement protein or a variant thereof. In some embodiments, the anti-C5 agent inhibits a function of C5 complement protein or a variant thereof. In one embodiment, the function inhibited by the anti-C5 agent is C5 complement protein cleavage.

A C5 complement protein variant as used herein encompasses a variant that performs substantially the same function as a C5 complement protein function. A C5 complement protein variant in some embodiments comprises substantially the same structure and in some embodiments comprises at least 80% sequence identity, in some embodiments at least 90% sequence identity, and in some embodiments at least 95% sequence identity to the amino acid sequence of the C5 complement protein comprising the amino acid sequence SEQ ID NO: 24.

In some embodiments, the anti-C5 agent is selected from a nucleic acid molecule, an aptamer, an antisense molecule, an RNAi molecule, a protein, a peptide, a cyclic peptide, an antibody or antibody fragment, a sugar, a polymer, or a small molecule. In certain embodiments, the anti-C5 agent is an anti-C5 agent described in PCT Patent Application Publication No. WO 2007/103549.

In particular embodiments, the anti-C5 agent is an anti-C5 aptamer. Aptamers are nucleic acid molecules having specific binding affinity to molecules through interactions other than classic Watson-Crick base pairing. Aptamers, like peptides generated by phage display or monoclonal antibodies ("mAbs"), are capable of specifically binding to selected targets and modulating the target's activity, e.g., through binding aptamers may block their target's ability to function. The aptamers may be unpegylated or pegylated. In particular embodiments, the aptamers may contain one or more 2' sugar modifications, such as 2'-O- alkyl (e.g., 2'-O-methyl or 2'-O-methoxyethyl) or 2'-fluoro modifications.

Illustrative C5 specific aptamers include the aptamers disclosed in PCT Publication No. WO 2007/103549, which is incorporated by reference in its entirety. Illustrative C5 specific aptamers include the aptamers ARC185 (SEQ ID NO: 25), ARC186 (SEQ ID NO: 26), ARC188 (SEQ ID NO: 27), ARC 189 (SEQ ID NO: 28), ARC243 (SEQ ID NO: 29), ARC244 (SEQ ID NO: 30), ARC250 (SEQ ID NO: 31), ARC296 (SEQ ID NO: 32), ARC297 (SEQ ID NO: 33), ARC330 (SEQ ID NO: 34), ARC331(SEQ ID NO: 35), ARC332 (SEQ ID NO: 36), ARC333 (SEQ ID NO: 37), ARC334 (SEQ ID NO: 38), ARC411 (SEQ ID NO: 39), ARC412 (SEQ ID NO: 40), ARC413 (SEQ ID NO: 41), ARC414 (SEQ ID NO: 42), ARC415 (SEQ ID NO: 43), ARC416 (SEQ ID NO: 44), ARC417 (SEQ ID NO: 45), ARC418 (SEQ ID NO: 46), ARC419 (SEQ ID NO: 47), ARC420 (SEQ ID NO: 48), ARC421 (SEQ ID NO: 49), ARC422 (SEQ ID NO: 50), ARC423 (SEQ ID NO: 51), ARC424 (SEQ ID NO: 52), ARC425 (SEQ ID NO: 53), ARC426 (SEQ ID NO: 54), ARC427 (SEQ ID NO: 55), ARC428 (SEQ ID NO: 56), ARC429 (SEQ ID NO: 57), ARC430 (SEQ ID NO: 58), ARC431 (SEQ ID NO: 59), ARC432 (SEQ ID NO: 60), ARC433 (SEQ ID NO: 61), ARC434 (SEQ ID NO: 62), ARC435 (SEQ ID NO: 63), ARC436 (SEQ ID NO: 64), ARC437 (SEQ ID NO: 65), ARC438 (SEQ ID NO: 66), ARC439 (SEQ ID NO: 67), ARC440 (SEQ ID NO: 68), ARC457 (SEQ ID NO: 69), ARC458 (SEQ ID NO: 70), ARC459 (SEQ ID NO: 71), ARC473 (SEQ ID NO: 72), ARC522 (SEQ ID NO: 73), ARC523 (SEQ ID NO: 74), ARC524 (SEQ ID NO: 75), ARC525 (SEQ ID NO: 76), ARC532 (SEQ ID NO: 77), ARC543 (SEQ ID NO: 78), ARC544 (SEQ ID NO: 79), ARC550 (SEQ ID NO: 80), ARC551 (SEQ ID NO: 81), ARC552 (SEQ ID NO: 82), ARC553 (SEQ ID NO: 83), ARC554 (SEQ ID NO: 84), ARC657 (SEQ ID NO: 85), ARC658 (SEQ ID NO: 86), ARC672 (SEQ ID NO: 87), ARC706 (SEQ ID NO: 88), ARC913 (SEQ ID NO: 89), ARC874 (SEQ ID NO: 90), ARC954 (SEQ ID NO: 91), ARC1537 (SEQ ID NO: 92), ARC1730 (SEQ ID NO: 93), or a pharmaceutically acceptable salt thereof.

In some embodiments, the anti-C5 agent is an aptamer with SEQ ID NO: 94, 95, or 96.

In a particular embodiment, the anti-C5 agent is a C5 specific aptamer comprising the nucleotide sequence of SEQ ID NO: 26 conjugated to a polyethylene glycol moiety via a linker. In some embodiments, the polyethylene glycol moiety has a molecular weight greater than about 10 kDa, particularly a molecular weight of about 20 kDa, more particularly about 30 kDa and more particulary about 40 kDa. In some embodiments, the polyethylene glycol moiety is conjugated via a linker to the 5' end of the aptamer. In some embodiments, the PEG conjugated to the 5' end of is a PEG of about 40 kDa molecular weight. In particular embodiments the about 40 kDa PEG is a branched PEG. In some embodiments the branched about 40 kDa PEG is 1,3-bis(mPEG-[about 20 kDa])-propyl-2-(4'-butamide). In other embodiments the branched about 40 kDa PEG is 2,3-bis(mPEG-[about 20 kDa])-propyl-1-carbamoyl.

In a particular embodiment, the C5 specific aptamer is a compound, ARC187, having the structure set forth below: 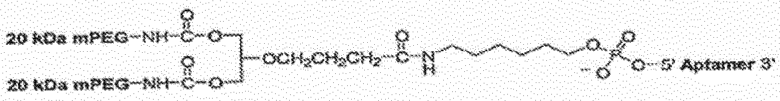
or a pharmaceutically acceptable salt thereof, where Aptamer =
fCmGfCfCGfCmGmGfUfCfUfCmAmGmGfCGfCfUmGmAmGfUfCfUmGmAmGf UfUfUAfCf CfUmGfCmG-3T (SEQ ID NO: 26)
wherein fC and fU= 2'-fluoro nucleotides, and mG and mA = 2'-OMe nucleotides and all other nucleotides are 2'-OH and where 3T indicates an inverted deoxy thymidine. In some embodiments, each 20 kDa mPEG of the above structure has a molecular weight of about 20 kDa.

In another particular embodiment, the C5 specific aptamer is a compound, ARC1905, having the structure set forth below: 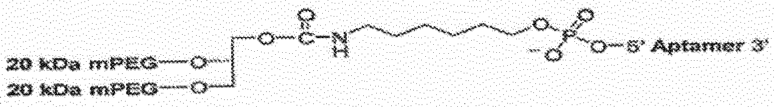
or a pharmaceutically acceptable salt thereof, where Aptamer = fCmGfCfCGfCmGmGfUfCfUfCmAmGmGfCGfCfUmGmAmGfUfCfUmGmAmGfUfUfUAfCf CfUmGfCmG-3T (SEQ ID NO: 26)
wherein fC and fU = 2'-fluoro nucleotides, and mG and mA = 2'-OMe nucleotides and all other nucleotides are 2'-OH and where 3T indicates and inverted deoxy thymidine. In some embodiments, each 20 kDa mPEG of the above structure has a molecular weight of about 20 kDa.

In other embodiments, the anti-C5 agent is an antisense oligonucleotide or ribozyme targeted to C5 that effects C5 inhibition by inhibiting protein translation from the messenger RNA or by targeting degradation of the corresponding C5 mRNA.

In still other embodiments, the anti-C5 agent is an anti-C5 RNA interference (RNAi) construct. Certain double stranded oligonucleotides useful to effect RNAi against C5 complement protein are less than 30 base pairs in length and may comprise about 25, 24, 23, 22, 21, 20, 19, 18 or 17 base pairs of ribonucleic acid and comprise a sequence with substantial sequence identity to the mRNA sequence of complement C5 protein, particularly human complement C5 protein. Optionally, the dsRNA oligonucleotides may include 3' overhang ends. Non-limiting illustrative 2-nucleotide 3' overhangs are composed of ribonucleotide residues of any type and may even be composed of 2'-deoxythymidine resides, which lowers the cost of RNA synthesis and may enhance nuclease resistance of siRNAs in the cell culture medium and within transfected cells (see Elbashi et al., (2001) Nature, 411: 494-8).

### Other Agents for Treatment or Prevention of an Ophthalmological Disease or Disorder

In another embodiment, another agent useful for treating or preventing an ophthalmological disease or disorder is volociximab or a pharmaceutically acceptable salt thereof (Ramakrishnan et al. (2008) J Exp Ther Oncol. 5:273-86, which is hereby incorporated by reference in its entirety).

In some embodiments, a plurality of aptamers can be associated with a single Non-Immunogenic, High Molecular Weight Compound, such as Polyalkylene Glycol or PEG, or a Lipophilic Compound, such as a glycerolipid. The aptamers can all be to one target or to different targets. In embodiments where a compound comprises more than one PDGF aptamer, there can be an increase in avidity due to multiple binding interactions with a target, such as PDGF or VEGF. In yet further embodiments, a plurality of Polyalkylene Glycol, PEG, glycerol lipid molecules can be attached to each other. In these embodiments, one or more aptamers can be associated with each Polyalkylene Glycol, PEG, or glycerol lipid. This can result in an increase in avidity of each aptamer to its target. In addition, in embodiments where there are aptamers to PDGF or aptamers to PDGF and different Targets associated with Polyalkylene Glycol, PEG, or glycerol lipid, a drug can also be associated with, e.g., covalently bonded to, Polyalkylene Glycol, PEG, or glycerol lipid. Thus the compound would provide targeted delivery of the drug, with Polyalkylene Glycol, PEG, or glycerol lipid serving as a Linker, optionally, with one or more additional linkers.

Aptamers can be 5'-capped and/or 3'-capped with a 5'-5' inverted nucleoside cap structure at the 5' end and/or a 3'-3' inverted nucleoside cap structure at the 3' end. In several embodiments, Antagonist A, Antagonist B, Antagonist C, Antagonist D, pegaptanib, bevasiranib and Sirna-027 are 5' or 3' end-capped.

### Methods for Treating or Preventing an Ophthalmological Disease or Disorder

The invention provides methods and compositions useful for treating or preventing ophthalmological diseases and disorders, including but not limited to any of the ophthalmological diseases and disorders described herein.

In some embodiments, the methods for treating or preventing an ophthalmological disease or disorder disclosed herein improve retinal attachment success, improve visual acuity, or stabilize vision. In some embodiments, the methods disclosed herein prevent or retard the rate of further vision loss in a subject.

In some embodiments, administration of Antagonist A or another pharmaceutically acceptable salt thereof in combination with a VEGF antagonist or pharmaceutically acceptable salt thereof and/or an anti-C5 agent improves retinal attachment success, improves visual acuity, or stabilizes vision to a degree that is greater than administration of Antagonist A or another pharmaceutically acceptable salt thereof alone, the VEGF antagonist or pharmaceutically acceptable salt thereof alone, or the anti-C5 agent alone. In some embodiments, the administration of Antagonist A or another pharmaceutically acceptable salt thereof and the VEGF antagonist or pharmaceutically acceptable salt thereof, and optionally, an anti-C5 agent, has a synergistic effect in treating or preventing an ophthalmological disease or disorder. For example, the administration of both Antagonist A or another pharmaceutically acceptable salt thereof and a VEGF antagonist or pharmaceutically acceptable salt thereof can improve retinal attachment success, improve visual acuity, or stabilize vision to a degree that is greater than an additive effect of administering both Antagonist A or another pharmaceutically acceptable salt thereof and the VEGF antagonist or pharmaceutically acceptable salt thereof. In some embodiments, administration of Antagonist A, alone or in combination with a VEGF antagonist and/or an anti-C5 agent, according to the methods described herein, e.g., treatment or dosing regimens, improves retinal attachment success, improves visual acuity, or stabilizes vision to a degree that is greater than administration of Antagonist A, alone or in combination with a VEGF antagonist and/or an anti-C5 agent, according to previously described methods.

In particular embodiments, any of the methods and compositions of the present invention are used to treat or prevent an ophthalmological disease or disorder in particular subjects. For example, in certain embodiments, subjects treated according to a method described herein are defined or identified based on their previous treatments for the disease or disorder, specific manifestations of their disease or disorder being treated, and/or other characteristics. In one embodiment, the subject has a defined phenotype or medical history.

Accordingly, any of the methods described herein may further comprise identifying the subject to be treated, such as by determining whether the subject was previously administered a VEGF antagonist for treating or preventing the disease or disorder or whether the subject had previously failed monotherapy with a VEGF antagonist, e.g., by inquiring of the subject or his health care provider, or by reviewing the subject's medical records.

In one embodiment, the subject was previously treated with a VEGF antagonist or anti-VEGF monotherapy for any ocular disease or disorder for which a VEGF antagonist is used, or for any of the ocular diseases or disorders described herein (e.g., wet-type AMD).

In particular embodiments, the methods and compositions described herein are useful for treating or preventing an ophthalmological disease or disorder in a subject who is anti-VEGF resistant, was previously administered or treated with anti-VEGF monotherapy, does not respond or had not responded favorably or adequately to anti-VEGF monotherapy, and/or failed monotherapy with a VEGF antagonist. In some embodiments, a subject who failed monotherapy is anti-VEGF resistant, has complement-mediated inflammation, and/or did not respond adequately to anti-VEGF monotherapy. In one embodiment, the subject who failed monotherapy with a VEGF antagonist is a subject who experienced a poor visual or anatomic outcome after treatment or administration with a VEGF antagonist. In one embodiment, the subject did not exhibit improved vision or exhibited reduced vision following anti-VEGF monotherapy.

In certain embodiments, the subject does not respond or had not responded favorably or adequately to anti-VEGF monotherapy, as determined by the subject's vision loss or by the subject's lack of significant vision gain following anti-VEGF monotherapy. In one embodiment, the subject's lack of significant vision gain following anti-VEGF monotherapy is determined by the subject's loss of ability to read one or more, in some embodiments three or more, and in some embodiments fifteen or more, letters of a standardized chart of vision testing, e.g., the Early Treatment for Diabetic Retinopathy Study Chart ("ETDRS chart"). In some embodiments, the vision testing is as described in Early Treatment Diabetic Retinopathy Study Research Group (ETDRS), Manual of Operations, Baltimore: ETDRS Coordinating Center, University of Maryland. Available from: National Technical Information Service, 5285 Port Royal Road, Springfield, VA 22161; Accession No. PB85 223006/AS; Ferris et al., Am J Ophthalmol 94:91-96, 1982; or Example 4, as described herein. In some embodiments, the vision testing uses one or more charts available from http://www.nei.nih.gov/photo/keyword.asp?conditions=Eye+Charts&match=all, e.g., ETDRS visual acuity Chart 1, 2 and/or R.

In another embodiment, the subject's vision loss following anti-VEGF monotherapy is determined by the subject's loss of ability to read one or more, in some embodiments three or more, letters or lines of a standardized chart of vision testing, e.g., the ETDRS chart, from baseline. In one embodiment, the subject's lack of significant vision gain following anti-VEGF monotherapy is determined by the subject's inability to read an additional one or more, in some embodiment three or more, and in some embodiments fifteen or more, letters of a standardized chart of vision testing, e.g., the ETDRS chart, from baseline. In another embodiment, the subject's lack of significant vision gain following anti-VEGF monotherapy is determined by the subject's inability to read an additional one or more, in some embodiments three or more, lines of a standardized chart of visual testing, e.g., the ETDRS chart, from baseline. In some embodiments, a subject's vision loss or lack of significant vision gain is determined by the subject's visual loss or anatomic signs of poor treatment response, for example, persistent leakage, increased hemorrhage, persistent or increased retinal pigment epithelium (RPE) detachment, signs of neovascular activity, or growth of neovascularization or increased deposition of abnormal matrix or fibrosis. In particular embodiments, a subject's vision loss or lack of significant vision gain is determined at 12 weeks or at 24 weeks following the initiation of treatment.

In certain embodiments, the subject is anti-VEGF-resistant to a VEGF antagonist, e.g., anti-VEGF monotherapy. In one embodiment, a subject is anti-VEGF resistant if the subject was previously administered with a VEGF antagonist, e.g., anti-VEGF monotherapy, that did not result in the treatment or prevention of the ophthalmological disease or disorder; resulted in only a temporary treatment or prevention of the ophthalmological disease or disorder and rendered the subject in further need of treatment or prevention of the ophthalmological disease or disorder; or that resulted in the subject's visual decline and rendered the subject in further need of treatment or prevention of the ophthalmological disease or disorder.

In another embodiment, a subject is anti-VEGF resistant if the subject was previously treated or administered with an anti-VEGF treatment, e.g., anti-VEGF monotherapy, and failed to achieve any visual gain or experienced visual decline. In some embodiments, the subject did not respond adequately to anti-VEGF treatment. In one embodiment, the subject was administered the anti-VEGF treatment for one year or longer. In some such embodiments, the subject is in need of treatment for wet AMD.

Accordingly, the present invention provides methods for treating, preventing, or stabilizing wet AMD in a subject, such as a subject who has failed monotherapy with a VEGF antagonist (e.g., is anti-VEGF resistant, has complement-mediated inflammation, and/or did not respond adequately to anti-VEGF monotherapy). In particular embodiments, the methods comprise determining whether the subject was previously administered or treated with anti-VEGF monotherapy. In certain embodiments, anti-VEGF monotherapy means administration of only one or more VEGF antagonists. In certain embodiments, anti-VEGF monotherapy includes the optional administration of other drugs that are not agents specifically adapted for treating an ophthalmological disease or disorder, e.g, wet AMD.

In certain embodiments, the methods and compositions described herein are useful for treating or preventing an ophthalmological disease or disorder in a subject that is treatment-naïve. In some embodiments, the subject is treatment-naïve if the subject was not previously treated for the ophthalmological disease or disorder. In some embodiments, the subject is treatment-naive if the subject was not previously administered or treated with a VEGF antagonist or anti-VEGF monotherapy ("anti-VEGF-treatment-naïve"). In particular embodiments, the methods further comprise determining whether the subject was previously treated for the ophthalmological disease or disorder or administered a VEGF antagonist or anti-VEGF monotherapy, e.g., by inquiring of the subject or his or her health care provider, or by reviewing the subject's medical records. In certain embodiments, anti-VEGF monotherapy means administration of only one or more VEGF antagonists. In certain embodiments, anti-VEGF monotherapy includes the optional administration of other drugs that are not agents specifically adapted for treating an ophthalmological disease or disorder, e.g, wet AMD. In some embodiments, the subject is treatment-naïve if the subject was not previously treated for AMD (e.g., wet AMD). In some embodiments, the subject is treatment-naïve if the subject was not previously treated, or has underwent no previous treatment for AMD (e.g., wet AMD) in either eye. In yet other embodiments, the subject is treatment-naive if the subject was not previously treated, or has underwent no previous treatment, for AMD (e.g., wet AMD; e.g., in either eye) except for one or more oral supplements of vitamins and minerals. In some embodiments, the subject is treatment-naive if the subject was not previously administered a therapeutic agent used for the treatment of AMD (e.g., wet AMD).

In certain embodiments, the subject has complement-mediated inflammation. In certain embodiments, the subject is anti-VEGF resistant and has complement-mediated inflammation. In certain embodiments, the complement-mediated inflammation is present in an eye of the subject. In certain embodiments, the complement-mediated inflammation results from previous administration with anti-VEGF monotherapy. In other embodiments, the subject has or has been diagnosed with complement-mediated inflammation. In still other embodiments, the subject did not respond adequately to anti-VEGF monotherapy and has or has been diagnosed with complement-mediated inflammation. In certain embodiments, complement-mediated inflammation is diagnosed in the subject using a genetic screening method. Such genetic screening methods are known to those of skill in the art and include, but are not limited to, screening for mutations in complement genes, such as complement factor H (CFH), CFI, CFHR5, and MCP, BF, and C2 genes.

In certain embodiments, the methods and compositions described herein are useful for treating or preventing an ophthalmological disease or disorder in a subject who is newly diagnosed with the ophthalmological disease or disorder. In some embodiments, the subject is newly diagnosed if the subject was not previously diagnosed for the ophthalmological disease or disorder. In some embodiments, the subject is newly diagnosed with age-related macular degeneration. In some embodiments, the subject is newly diagnosed with dry age-related macular degeneration. In some embodiments, the subject is newly diagnosed with wet-type AMD. In particular embodiments, the methods further comprise determining whether the subject was previously diagnosed for the ophthalmological disease or disorder, e.g., by inquiring of the subject or his or her health care provider, or by reviewing the subject's medical records.

In some embodiments of the invention, the methods and compositions described herein are useful for treating or preventing an ophthalmological disease or disorder that is a neovascular disorder. In other embodiments of the invention, the ophthalmological disease or disorder results in retinal edema. Illustrative ophthalmological diseases or disorders that can be treated or prevented are described herein.

### Treatment or Prevention of Age-Related Macular Degeneration

In one embodiment, the ophthalmological disease or disorder treated or prevented by any of the methods or compositions described herein is age-related macular degeneration. Vision changes that can be associated with macular degeneration include distortions and/or blind spots (scotoma) detected using an Amsler grid, changes in dark adaptation (diagnostic of rod cell health), changes in color interpretation (diagnostic of cone cell health), or a decrease in visual acuity. Examples of age-related macular degeneration are nonneovascular (also known as "dry") and neovascular (also known as "wet" or "exudative") macular degeneration.

In one embodiment, the dry age-related macular degeneration is associated with the formation of drusen. In one embodiment, treating or preventing dry macular degeneration encompasses treating or preventing an abnormality of the retinal pigment epithelium and/or underlying vasculature, known as choriocapilaries. Examples of abnormalities of the retinal pigment epithelium include geographic atrophy, non-geographic atrophy, focal hypopigmentation, and focal hyperpigmentation. In another embodiment, treating or preventing wet age-related macular degeneration encompasses treating or preventing choroidal neovascularization or pigment epithelial detachment.

In one embodiment, the invention provides methods for treating or preventing wet age-related macular degeneration. Another aspect of the present invention is methods for treating, preventing, or inhibiting a choroidal neovascular complex in a subject, e.g., inhibiting the formation or growth of a choroidal neovascular complex.

In another aspect of the invention, the invention provides methods for treating or preventing choroidal neovascularization in a subject. In some embodiments, the choroidal neovascularization is subfoveal choroidal neovascularization. In some embodiments, the subfoveal choroidal neovascularization is due to age-related macular degeneration. In one embodiment, the subfoveal choroidal neovascularization is secondary to exudative type AMD. In other embodiments, the subfoveal choroidal neovascularization is present in subjects who have exudative type AMD, and in other embodiments, subfoveal choroidal neovascularization is present in subjects who do not have exudative type AMD. In some embodiments, the subfoveal choroidal neovascularization is secondary to inflammatory, traumatic, myopic, idiopathic or neoplastic afflictions of the macula.

In some embodiments, wet age-related macular degeneration is classified according to the appearance of its choroidal neovascularization (CNV), into classic, occult or mixed (classic and occult) CNV types, as determined by an angiography, known as fluorescence angiography. Classic, occult or mixed (classic and occult) CNV classification can be based on the time, intensity and level of definition of dye appearance, and leakage from the CNV, as assessed by the fluorescein angiography. In some embodiments, the subject has classic CNV (e.g., pure classic) or mixed CNV (predominantly or minimally classic CNV). In some embodiments, the subject has occult CNV (e.g., pure occult CNV).

The administration of Antagonist A or another pharmaceutically acceptable salt thereof and the VEGF antagonist and/or anti-C5 agent can have a synergistic effect in treating or preventing classic CNV or occult CNV. For example, administration of both Antagonist A or another pharmaceutically acceptable salt thereof and the VEGF antagonist can improve visual acuity or stabilize vision to a degree that is greater than an additive effect of both Antagonist A or another pharmaceutically acceptable salt thereof and the VEGF antagonist. In another example, administration of both Antagonist A or another pharmaceutically acceptable salt thereof and the VEGF antagonist can reduce CNV or inhibit the growth of CNV to a greater degree than administration of Antagonist A or another pharmaceutically acceptable salt thereof or the VEGF antagonist. In some embodiments, administration of both Antagonist A or another pharmaceutically acceptable salt thereof and the VEGF antagonist can reduce CNV in a shorter timeframe or with a lower dosage amount or frequency, as compared to the timeframe or dosage amount with administration of Antagonist A or another pharmaceutically acceptable salt thereof or the VEGF antagonist. In some embodiments, administration of both Antagonist A or another pharmaceutically acceptable salt thereof and the VEGF antagonist can reduce CNV or inhibit the growth of CNV to a greater degree than an additive effect of both Antagonist A or another pharmaceutically acceptable salt thereof and the VEGF antagonist. In some embodiments, administration of both Antagonist A or another pharmaceutically acceptable salt thereof and the VEGF antagonist can reduce CNV in a shorter timeframe or with a lower dosage amount or frequency, as compared to an additive timeframe, dosage amount or frequency with administration of both Antagonist A or another pharmaceutically acceptable salt thereof and the VEGF antagonist.

In one embodiment, the present invention provides methods for treating, preventing, or stabilizing non-exudative type ("dry type") AMD. In one embodiment, Antagonist A or another pharmaceutically acceptable salt thereof, an anti-C5 agent, the combination of Antagonist A or another pharmaceutically acceptable salt thereof and an anti-C5 agent, or the combination of an anti-C5 agent and a VEGF antagonist is administered in an amount effective to maintain about the same level of drusen or reduce the level of drusen (e.g., amount, size, number, area and/or morphology) (e.g., size, number, area and/or morphology) as compared to the subject's drusen level prior to administration of Antagonist A or another pharmaceutically acceptable salt thereof, the anti-C5 agent, the combination of Antagonist A or another pharmaceutically acceptable salt thereof and the anti-C5 agent, or or the combination of an anti-C5 agent and a VEGF antagonist, respectively. In a particular embodiment, the level of drusen is reduced by at least or about 5%, at least or about 10%, at least or about 20%, at least or about 30%, at least or about 40%, or at least or about 50%.

In some embodiments, Antagonist A or another pharmaceutically acceptable salt thereof, an anti-C5 agent, the combination of Antagonist A or another pharmaceutically acceptable salt thereof and the anti-C5 agent, or the combination of the anti-C5 agent and a VEGF antagonist is administered in an amount effective to inhibit, slow, or prevent the progression of non-exudative type AMD to geographic atrophy (GA). GA is an advanced form of non-exudative type AMD. In other embodiments, the Antagonist A or another pharmaceutically acceptable salt thereof and/or the anti-C5 agent or a pharmaceutically acceptable salt thereof is administered in an amount effective to reduce the growth or area of a GA lesion over time as compared to that in a subject not receiving Antagonist A or another pharmaceutically acceptable salt thereof and/or the anti-C5 agent. In other embodiments, the anti-C5 agent or a pharmaceutically acceptable salt thereof and a VEGF antagonist is administered in an amount effective to reduce the growth or area of a GA lesion over time as compared to that in a subject not receiving the anti-C5 agent and/or the VEGF antagonist. In a particular embodiment, the change in area or growth of the geographic atrophy lesion over time is reduced by at least or about 5%, at least or about 1 0%, at least or about 20%, at least or about 30%, at least or about 40%, or at least or about 50%. Methods of identifying and assessing the size of geographic lesions are known to those of skill in the art and include autofluorescence imaging and optical coherence tomography.

In particular embodiments, a subject in whom non-exudative AMD converts to exudative AMD, e.g., when new blood vessels invade the overlying retina, is treated. The present invention further provides methods for treating, preventing, or stabilizing drusen retinopathy secondary to complement-mediated immune disorders, including drusen retinopathy secondary to membranoproliferative glomerulonephritis type II disease. In some embodiments, Antagonist A or another pharmaceutically acceptable salt thereof and/or an anti-C5 agent and/or a VEGF antagonist is administered in an amount effective to reduce retinal drusen in subjects having or having been diagnosed with membranoproliferative glomerulonephritis type II disease or exudative-type AMD as compared to the level of retinal drusen prior to administration of Antagonist A or another pharmaceutically acceptable salt thereof and/or an anti-C5 agent and/or a VEGF antagonist. In certain embodiments, the level of drusen is reduced by at least or about 5%, at least or about 10%, at least or about 20%, at least or about 30%, at least or about 40%, or at least or about 50%.

In one embodiment, the ophthalmological disease or disorder is polypoidal choroidal vasculopathy (PCV), a variant of wet AMD.

### Treatment or Prevention of a Condition Associated with Choroidal Neovascularization

In one embodiment, the ophthalmological disease or disorder is a condition associated with choroidal neovascularization. Examples of conditions associated with choroidal neovascularization include a degenerative, inflammatory, traumatic or idiopathic condition. Treating or preventing a degenerative disorder associated with choroidal neovascularization also encompasses treating or preventing a heredodegerative disorder. Examples of heredodegenerative disorders include vitelliform macular dystrophy, fundus flavimaculatus and optic nerve head drusen. Examples of degenerative conditions associated with choroidal neovascularization include myopic degeneration or angioid streaks. In some embodiments, treating or preventing an inflammatory disorder associated with choroidal neovascularization encompasses treating or preventing ocular histoplasmosis syndrome, multifocal choroiditis, serpininous choroiditis, toxoplasmosis, toxocariasis, rubella, Vogt-Koyanagi-Harada syndrome, Behcet syndrome or sympathetic ophthalmia. In some embodiments, treating or preventing a traumatic disorder associated with choroidal neovascularization encompasses treating or preventing choroidal rupture or a traumatic condition caused by intense photocoagulation.

### Treatment or Prevention of Proliferative Retinopathy

One particular aspect of the invention provides methods and compositions for treating or preventing proliferative vitreoretinopathy (PVR). In some embodiments, the PVR is a moderate form. In other embodiments, the PVR is a severe form. In some embodiments, the PVR is a recurrent form. In one embodiment, the subject with PVR also has or had retinal detachment, or the subject has PVR associated with retinal detachment, or PVR related scarring (e.g., scarring resulting from PVR, e.g., retinal scarring). In some embodiments, the PVR is characterized based on the configuration of the retina and the location of the scar tissue, such as in shown in Table 2 *(See* Lean J, et al. Classification of proliferative vitreoretinopathy used in the silicone study. The Silicone study group. Ophthalmology 1989;96:765-771*).* Any of these categories or types of PVR can be treated or prevented according to the present invention.

**Table 2. Classification of PVR**

| **Type no.** | **Type of contraction** | **Location of PVR** | **Summary of Clinical Signs** |
|---|---|---|---|
| 1 | Focal | Posterior | Starfold |
| 2 | Diffuse | Posterior | Confluent irregular retinal folds in posterior retina; remainder of retina drawn posteriorly; optic disc may not be visible |
| 3 | Sub-retinal | Posterior | "Napkin ring" around disc or "clothesline" elevation of retina |
| 4 | Circumferential | Anterior | Irregular retinal folds in the anterior retina; series of radial folds more posteriorly; peripheral retina within vitreous base stretched inward |
| 5 | Perpendicular | Anterior | Smooth circumferential fold of retina at insertion of posterior hyaloid |
| 6 | Anterior | Anterior | Circumferential fold of retina at insertion of posterior hyaloid pulled forward; trough of peripheral retina anteriorly; ciliary processes stretched with possible hypotony; iris retracted |

The present methods for treating PVR can further comprise administering another agent useful for treating PVR, such as a corticosteriod; antineoplastic drug, such as 5-fluorouracil; colchicine; retinoid; heparin; epidermal growth factor receptor (EGFR) inhibitor, such as gefitinib or erlotinib.

Another aspect of the invention is methods for treating or preventing a proliferative retinopathy, such as one related to PVR (e.g., treating or preventing an ocular manifestation of a proliferative retinopathy), such as proliferative diabetic retinopathy, sickle cell retinopathy, post traumatic retinopathy, hyperviscosity syndromes, Aortic arch syndromes, ocular ischemic syndromes, carotid-cavernous fistula, multiple sclerosis, retinal vasculitis, systemic lupus erythematosus, arteriolitis with SS-A autoantibody, acute multifocal hemorrhagic vasculitis, vasculitis resulting from infection, vasculitis resulting from Behçet's disease, sarcoidosis, coagulopathies, sickling hemoglobinopathies, AC and G-β thalassemia, small vessel hyalinosis, incontinentia pigimenti, Eales' disease, branch retinal artery or vein occlusion, frosted branch angiitis, idiopathic retinal vasculitis, aneurysms, neuroretinitis, retinal embolization, retinopathy of prematurity, Uveitis, pars planitis, acute retinal necrosis, birdshot retinochoroidopathy, longstanding retinal detachment, choroidal melanoma, radiation retinopathy, familial exudative vitreoretinopathy, inherited retinal venous beading, retinoschisis, retinitis pigmentosa, or autosomal dominant vitreoretinochoroidopathy.

Another aspect of the invention is methods for treating or preventing a disease or condition that is a cause that results in proliferative retinopathy or PVR. In one embodiment, post-retinal detachment (e.g., that causes or results in PVR) is treated or prevented. In another embodiment, proliferative diabetic retinopathy (e.g., that causes or results in PVR) or sickle-cell retinopathy (e.g., that causes or results in PVR), as well as scarring caused by one or more of these disorders is treated or prevented.

### Treatment or Prevention of Glaucoma

In one embodiment, the opthalmological disease or disorder is glaucoma. In one embodiment the glaucoma is open angle glaucoma, primary open angle glaucoma, secondary open angle glaucoma, closed angle glaucoma, glaucoma that is associated with diabetes, glaucoma that is associated with diabetic retinopathy, angle closure glaucoma, narrow angle glaucoma or acute glaucoma.

### Treatment or Prevention of a Neoplasm

In one embodiment, the ophthalmological disease or disorder is a neoplasm. Examples of neoplams include an eyelid tumor, a conjunctival tumor, a choroidal tumor, an iris tumor, an optic nerve tumor, a retinal tumor, an infiltrative intraocular tumor or an orbital tumor. Examples of an eyelid tumor include basal cell carcinoma, squamous carcinoma, sebaceous carcinoma, malignant melanoma, capillary hemangioma, hydrocystoma, nevus or seborrheic keratosis. Examples of a conjunctival tumor include conjunctival Kaposi's sarcoma, squamous carcinoma, intraepithelial neoplasia of the conjunctiva, epibular dermoid, lymphoma of the conjunctiva, melanoma, pingueculum, or pterygium. Examples of a choroidal tumor include choroidal nevus, choroidal hemangioma, metastatic choroidal tumor, choroidal osteoma, choroidal melanoma, ciliary body melanoma or nevus of Ota. Examples of an iris tumor include anterior uveal metastasis, iris cyst, iris melanocytoma, iris melanoma, or pearl cyst of the iris. Examples of an optic nerve tumor include optic nerve melanocytoma, optic nerve sheath meningioma, choroidal melanoma affecting the optic nerve, or circumpapillary metastasis with optic neuropathy. Examples of a retinal tumor include retinal pigment epithelial (RPE) hypertrophy, RPE adenoma, RPE carcinoma, retinoblastoma, or hamartoma of the RPE. In some embodiments, the present invention provides methods for inhibiting retinal pigment epithelium (RPE) or glial cells, such as inhibiting the migration of RPE or glial cells. Examples of an infiltrative intraocular tumor include chronic lymphocytic leukemia, infiltrative choroidopathy, or intraocular lymphoma. Examples of an orbital tumor include adenoid cystic carcinoma of the lacrimal gland, cavernous hemangioma of the orbit, lymphangioma of the orbit, orbital mucocele, orbital pseudotumor, orbital rhabdomyosarcoma, periocular hemangioma of childhood, or sclerosing orbital psuedotumor.

Another aspect of the invention is methods for treating or preventing von Hippel-Lindau (VHL) disease (e.g., treating or preventing visual loss associated VHL disease). In some embodiments, VHL disease is characterized by tumors. The tumors may be malignant or benign. In another embodiment, a benign or malignant tumor in the eye (e.g., ocular tumor) or a cyst (e.g., an ocular cyst), associated with VHL is treated or prevented. In some embodiments, the tumors are hemangioblastomas. In some embodiments, the tumors are von Hippel angioma or retinal capillary hemangiomas (e.g., juxtapapillary hemangioma).

In some embodiments, the subject with VHL disease has a deficiency of the protein "pVHL."

In some embodiments, the VHL disease is severe (e.g., a subject with severe VHL disease has a lesion that cannot be effectively treated with a non-pharmacologic modality (e.g., laser or or cryotherapy), for example, as the lesion resides over or adjacent to a significant neural structure (e.g., optic nerve, macula, papillomacular bundle) that can be damaged with laser or cryotherapy).

In some embodiments, the methods for treating or preventing VHL disease comprise treating an ocular or non-ocular manifestation (e.g., benign or malignant neoplasm or cyst of the kidney, adrenal gland, pancreas, brain, spinal cord, inner ear, epididymis, or broad ligament) of VHL.

In some embodiments, the subjected being treated has a family history of VHL disease or one or more of retinal capillary hemangioma (RCH), spinal or cerebellar hemangioblastoma, pheochromocytoma, multiple pancreatic cysts, epididymal or broad ligament cystadenoma, multiple renal cysts, and renal cell carcinoma. In some embodiments, the subject has one or more RCH, spinal and cerebellar hemangioblastoma, pheochromocytoma, multiple pancreatic cysts, epididymal or broad ligament cystadenomas, multiple renal cysts, or renal cell carcinoma before the age of 60 years. In some embodiments, the subject has two or more hemangioblastomas of the retina or brain or a single hemangioblastoma in association with a visceral manifestation, such as kidney or pancreatic cysts; renal cell carcinoma; adrenal or extra-adrenal pheochromocytomas; endolymphatic sac tumors; papillary cystadenomas of the epididymis or broad ligament; or neuroendocrine tumors of the pancreas. In some embodiments, the subject has a disease-causing germline mutation in the VHL gene.

In some embodiments, the subject has RCH that exhibit activity, such as associated intra- or sub-retinal exudation or lipid deposition (which may reflect ongoing vascular incompetence and is not reflective of residual changes following previous treatment or secondary to coexistent retinal traction); increased size of the tumor compared to a previous time point as assessed by fundus photography or fluorescein angiography (FA); associated intra-, sub-, or pre-retinal hemorrhage not secondary to previous treatment, as assessed by fundus photography or FA; appearance of new feeder vessels or greater dilation or tortuosity of existing feeder vessels compared to a previous time point; and/or vitreous cell or haze indicative of vitreous exudation, in the absence of other ocular features potentially responsible for such findings. In some embodiments, the subject has RCH that is not readily treatable using cryotherapy or thermal laser because of its size, posterior location, poor previous response to conventional therapy, or other factors.

In some embodiments, methods or compositions of the invention are used to treat or prevent a complication of VHL, visual dysfunction (e.g., from VHL), or a fibrous complication of VHL (e.g., fibrous meningioma). In certain embodiments, the methods or compositions of the present invention are used to treat a manifestation of VHL as vascular proliferation that comprises fine, superficial, juxtapapillary vessels that are often associated with fibrovascular proliferation and epiretinal membrane formation.

### Treatment or Prevention of Scarring or Fibrosis

Another aspect the invention provides methods for treating, inhibiting or preventing scarring or fibrosis (e.g., scarring or fibrosis is under the macular region of the retina). In some embodiments, the scarring is a fibrovascular scar (e.g., in the retina). In some embodiments, the fibrosis is hepatic, pulmonary or renal fibrosis. In some embodiments, the fibrosis is ocular fibrosis. In some embodiments, the fibrosis is sub-retinal fibrosis (e.g., associated with neovascular AMD). In some embodiments, the sub-retinal fibrosis is not associated with neovascular AMD. In some embodiments, the fibrosis is subfoveal fibrosis. In some embodiments, the subfoveal fibrosis is with retinal atrophy. In some embodiments, subfoveal fibrosis or sub-retinal fibrosis develops after administration of a VEGF antagonist, e.g., anti-VEGF monotherapy.

In some embodiments, the scarring results from glaucoma surgery, or follows glaucoma surgery, such as trabeculectomy, filtering surgery (such as partial thickness filtering surgery), glaucoma filtering procedures, minimally invasive glaucoma surgery, glaucoma valve implant surgery, glaucoma seton surgery, glaucoma tube shunt placement, glaucoma stent placement, or combined cataract and glaucoma surgery. In some embodiments, the methods of the present invention are useful to treat or prevent scarring relating to or resulting from glaucoma surgery (e.g., that can result in scar related proliferation). In some embodiments, the scarring is sub-retinal scarring. In some embodiments, the scarring is sub-retinal scarring that occurs following choroidal neovascular regression.

In particular embodiments, methods for treating, inhibiting or preventing sub-retinal fibrosis (e.g., reducing the formation of sub-retinal fibrosis) comprise administering to a subject in need thereof an effective amount of Antagonist A or another pharmaceutically acceptable salt thereof and a VEGF antagonist. In some embodiments, the subject has or is diagnosed with AMD (e.g., wet AMD). In some embodiments, the subject has or is diagnosed with advanced wet AMD.

### Treatment or Prevention of Other Ophthalmological Diseases and Disorders

In certain embodiments , the ophthalmological disease or disorder is a cataract (e.g., age-related cataract), diabetic macula edema, macular telangiectasia (e.g., type 1 or 2 macular telangiectasia), atrophic macular degeneration, cliorioretinopathy (e.g., central serous chorioretinopathy), retinal inflammatory vasculopathy, pathological retinal angiogenesis, age-related maculopathy, retinoblastoma, Pseudoxanthoma elasticum, a vitreoretinal disease, choroidal sub-retinal neovascularization, central serous chorioretinopathy, ischemic retinopathy, hypertensive retinopathy or diabetic retinopathy (e.g., nonproliferative or proliferative diabetic retinopathy, such as macular edema or macular ischemia), retinopathy of prematurity (e.g., associated with abnormal growth of blood vessels in the vascular bed supporting the developing retina), venous occlusive disease (e.g., a retinal vein occlusion, branch retinal vein occlusion or central retinal vein occlusion), arterial occlusive disease (e.g., branch retinal artery occlusion (BRAO), central retinal artery occlusion or ocular ischemic syndrome), central serous chorioretinopathy (CSC), cystoid macular edema (CME) (e.g., affecting the central retina or macula, or after cataract surgery), retinal telangiectasia (e.g., characterized by dilation and tortuosity of retinal vessels and formation of multiple aneurysms, idiopathic JXT, Leber's miliary aneurysms, or Coats' disease), arterial macroaneurysm, retinal angiomatosis, radiation-induced retinopathy (RIRP), or rubeosis iridis (e.g., associated with the formation of neovascular glaucoma, diabetic retinopathy, central retinal vein occlusion, ocular ischemic syndrome, or chronic retinal detachment).

In other embodiments, the ophthalmological disease or disorder is sickle cell disease (SCD), anemia, or sickle cell retinopathy (e.g., non-neovascular or non-proliferative ocular manifestations). In some embodiments, vaso-occlusive phenomena or hemolysis associated with SCD is treated or prevented. In some embodiments, ocular manifestations of SCD include vascular occlusions in the conjunctiva, iris, retina, or choroid. Non-neovascular or non-proliferative ocular manifestations can include conjunctival vascular occlusions which transform smooth vessels into comma-shaped fragments, iris atrophy, retinal "salmon patch" hemorrhages, retinal pigmentary changes and other abnormalities of the retinal vasculature, macula, choroid, and optic disc. In some embodiments, neovascularization or the proliferative ocular manifestation involves the growth of abnormal vascular fronds which can lead to vitreous hemorrhage, retinal detachment, epiretinal membranes, resulting in vision loss. In some embodiments, the methods further comprise performing another treatment, such as diathermy, cryotherapy, laser photocoagulation or surgery (e.g., vitrectomy).

In one embodiment, the ophthalmological disease or disorder is a condition associated with peripheral retinal neovascularization. Examples of conditions associated with peripheral retinal neovascularization include ischemic vascular disease, inflammatory disease with possible ischemia, incontinentia pigmenti, retinitis pigmentosa, retinoschisis or chronic retinal detachment.

Examples of ischemic vascular disease include proliferative diabetic retinopathy, branch retinal vein occlusion, branch retinal arteriolar occlusion, carotid cavernous fistula, sickling hemoglobinopathy, non-sickling hemoglobinopathy, IRVAN syndrome (retinal vasculitic disorder characterized by idiopathic retinal vasculitis, an aneurysm, and neuroretinitis), retinal embolization, retinopathy of prematurity, familial exudative vitreoretinopathy, hyperviscosity syndrome, aortic arch syndrome or Eales disease. Examples of sickling hemoglobinopathy include SS hemoglobinopathy and SC hemoglobinopathy. Examples of non-sickling hemoglobinopathy include AC hemoglobinopathy and AS hemoglobinopathy. Examples of hyperviscosity syndrome include leukemia, Waldenstrom macroglobulinemia, multiple myeloma, polycythemia or myeloproliferative disorder.

In some embodiments, treating or preventing an inflammatory disease with possible ischemia encompasses treating or preventing retinal vasculitis associated with systemic disease, retinal vasculitis associated with an infectious agent, uveitis or birdshot retinopathy. Examples of systemic diseases include systemic lupus erythematosis, Behcet's disease, inflammatory bowel disease, sarcoidosis, multiple sclerosis, Wegener's granulomatosis and polyarteritis nodosa. Examples of infectious agents include a bacterial agent that is the causative agent for syphilis, tuberculosis, Lyme disease or cat-scratch disease, a virus such as herpesvirus, or a parasite such as Toxocara canis or Toxoplasma gondii. Examples of uveitis include pars planitis or Fuchs uveitis syndrome.

### Compositions for Therapeutic or Prophylactic Administration

Antagonist A or another pharmaceutically acceptable salt thereof, VEGF antagonists, or anti-C5 agents can be administered as a component of a composition that further comprises a pharmaceutically acceptable carrier or vehicle, e.g., a pharmaceutical composition. In certain embodiments, each therapeutic agent is administered to the subject in a separate composition. However, in other embodiments, two or more therapeutic agents may be administered to the subject in the same composition. In one embodiment, a composition of the invention comprises an effective amount of Antagonist A or another pharmaceutically acceptable salt thereof, a VEGF antagonist, and/or an anti-C5 agent and a pharmaceutically acceptable carrier or vehicle. In another embodiment, a composition comprising Antagonist A or another pharmaceutically acceptable salt thereof and another composition comprising a VEGF antagonist are administered. In some embodiments, another composition comprising an anti-C5 agent is administered. In some embodiments, a composition comprising Antagonist A or another pharmaceutically acceptable salt thereof and a VEGF antagonist is administered. In some embodiments, another composition comprising an anti-C5 agent is also administered.

Administration of each antagonist may be by any suitable means that results in an amount of Antagonist A or another pharmaceutically acceptable salt thereof, VEGF antagonist, and/or anti-C5 agent that is effective for the treatment or prevention of an ophthalmological disease or disorder. Each antagonist, for example, can be admixed with a suitable carrier substance, and is generally present in an amount of 1-95% by weight of the total weight of the composition. The composition may be provided in a dosage form that is suitable for ophthalmic, oral, parenteral (e.g., intravenous, intramuscular, subcutaneous), rectal, transdermal, nasal, or inhalant administration. In one embodiment, the composition is in a form that is suitable for injection directly in the eye. The composition may be in form of, e.g., tablets, capsules, pills, powders, granulates, suspensions, emulsions, solutions, gels including hydrogels, pastes, ointments, creams, plasters, delivery devices, suppositories, enemas, injectables, implants, sprays, drops or aerosols. The compositions comprising one or more antagonists can be formulated according to conventional pharmaceutical practice (see, e.g., Remington: The Science and Practice of Pharmacy, (20th ed.) ed. A. R. Gennaro, 2000, Lippincott Williams & Wilkins, Philadelphia, Pa. and Encyclopedia of Pharmaceutical Technology, eds., J. Swarbrick and J. C. Boylan, 1988-2002, Marcel Dekker, New York).

The compositions are, in one useful aspect, administered parenterally (e.g., by intramuscular, intraperitoneal, intravenous, intraocular, intravitreal, retro-bulbar, subconjunctival, subtenon or subcutaneous injection or implant) or systemically. Formulations for parenteral or systemic administration include sterile aqueous or non-aqueous solutions, suspensions, or emulsions. A variety of aqueous carriers can be used, e.g., water, buffered water, saline, and the like. Examples of other suitable vehicles include polypropylene glycol, polyethylene glycol, vegetable oils, gelatin, hydrogels, hydrogenated naphalenes, and injectable organic esters, such as ethyl oleate. Such formulations may also contain auxiliary substances, such as preserving, wetting, buffering, emulsifying, and/or dispersing agents. Biocompatible, biodegradable lactide polymer, lactide/glycolide copolymer, or polyoxyethylene-polyoxypropylene copolymers may be used to control the release of the active ingredients.

Alternatively, the compositions can be administered by oral ingestion. Compositions intended for oral use can be prepared in solid or liquid forms, according to any method known to the art for the manufacture of pharmaceutical compositions.

Solid dosage forms for oral administration include capsules, tablets, pills, powders, and granules. Generally, these pharmaceutical preparations contain active ingredients admixed with non-toxic pharmaceutically acceptable excipients. These include, for example, inert diluents, such as calcium carbonate, sodium carbonate, lactose, sucrose, glucose, mannitol, cellulose, starch, calcium phosphate, sodium phosphate, kaolin and the like. Binding agents, buffering agents, and/or lubricating agents (e.g., magnesium stearate) may also be used. Tablets and pills can additionally be prepared with enteric coatings. The compositions may optionally contain sweetening, flavoring, coloring, perfuming, and preserving agents in order to provide a more palatable preparation.

Compositions useful for ophthalmic use include tablets comprising one or more antagonists in admixture with a pharmaceutically acceptable excipient. These excipients may be, for example, inert diluents or fillers (e.g., sucrose and sorbitol), lubricating agents, glidants, and anti adhesives (e.g., magnesium stearate, zinc stearate, stearic acid, silicas, hydrogenated vegetable oils, or talc).

The antagonists of the present invention may be admixed in a tablet or other vehicle, or may be partitioned. In one example, one antagonist is contained on the inside of the tablet, and the other antagonist is on the outside, such that a substantial portion of the other antagonist is released prior to the release of the contained antagonist. If desired, antagonists in a tablet form may be administered using a drug delivery device (see below).

For example, compositions of the present invention may be administered intraocularly by intravitreal injection into the eye as well as by subconjunctival and subtenon injections. Other routes of administration include transcleral, retrobulbar, intraperitoneal, intramuscular, and intravenous. Alternatively, compositions can be administered using a drug delivery device or an intraocular implant (see below).

In one embodiment, Antagonist A or another pharmaceutically acceptable salt thereof or VEGF antagonist (e.g., ranibizumab, bevacizumab, aflibercept, pegaptanib sodium, or ESBA 1008) is administered intravitreally with a 30-gauge or 27-gauge needle. In some embodiments, a 0.5 inch needle is used. In one embodiment, Antagonist A or another pharmaceutically acceptable salt thereof is administered intravitreally with a 30-gauge 0.5 inch needle and a VEGF antagonist (e.g., ranibizumab, bevacizumab, aflibercept, pegaptanib sodium, or ESBA1008) is administered intravitreally with a 27-gauge needle. In some embodiments, 50µL (1.5 mg in 0.05 mL) of Antagonist A or another pharmaceutically acceptable salt thereof is administered intravitreally with a 30-gauge 0.5 inch needle and 50µL of a VEGF antagonist (e.g., 0.5 mg of ranibizumab, 1.25 mg of bevacizuamb, or 2.0 mg of aflibercept) is administered intravitreally with a 27-gauge needle.

Liquid dosage forms for oral administration can include pharmaceutically acceptable emulsions, solutions, suspensions, syrups, and soft gelatin capsules. These forms can contain inert diluents commonly used in the art, such as water or an oil medium, and can also include adjuvants, such as wetting agents, emulsifying agents, and suspending agents.

In some instances, the compositions can also be administered topically, for example, by patch or by direct application to a region, such as the epidermis or the eye, susceptible to or affected by a neovascular disorder, or by iontophoresis.

In one embodiment, the compositions can comprise one or more pharmaceutically acceptable excipients. In one embodiment, excipients for compositions that comprise an antagonist include, but are not limited to, buffering agents, nonionic surfactants, preservatives, tonicity agents, sugars, amino acids, and pH-adjusting agents. Suitable buffering agents include, but are not limited to, monobasic sodium phosphate, dibasic sodium phosphate, and sodium acetate. Suitable nonionic surfactants include, but are not limited to, polyoxyethylene sorbitan fatty acid esters such as polysorbate 20 and polysorbate 80. Suitable preservatives include, but are not limited to, benzyl alcohol. Suitable tonicity agents include, but are not limited to sodium chloride, mannitol, and sorbitol. Suitable sugars include, but are not limited to, α,α-trehalose. Suitable amino acids include, but are not limited to glycine and histidine. Suitable pH-adjusting agents include, but are not limited to, hydrochloric acid, acetic acid, and sodium hydroxide. In one embodiment, the pH-adjusting agent or agents are present in an amount effective to provide a pH of about 3 to about 8, about 4 to about 7, about 5 to about 6, about 6 to about 7, or about 7 to about 7.5. In one embodiment, the compositions do not comprise a preservative. In another embodiment, the composition does not comprise an antimicrobial agent. In another embodiment, the composition does not comprise a bacteriostat. Suitable excipients for a VEGF antagonist also include those described in U.S. Pat. No. 7,365,166, the contents of which are herein incorporated by reference in their entirety.

In one embodiment, the composition is in the form of an aqueous solution that is suitable for injection. In one embodiment, a composition is in the form of an aqueous solution that is suitable for injection. In one embodiment, a composition comprises Antagonist A or another pharmaceutically acceptable salt thereof, a buffering agent, a pH-adjusting agent, and water for injection. In another embodiment, a composition comprises Antagonist A or another pharmaceutically acceptable salt thereof, monobasic sodium phosphate, dibasic sodium phosphate, sodium chloride, hydrochloride acid, and sodium hydroxide..

In one embodiment, the composition comprises a VEGF antagonist, a buffering agent, a sugar, a nonionic surfactant, and water for injection. In another embodiment, the composition comprises a VEGF antagonist, monobasic sodium phosphate, dibasic sodium phosphate, α,α-trehalose dehydrate, and polysorbate 20. In one embodiment, the composition comprises a VEGF antagonist, a buffering agent, a pH-adjusting agent, a tonicity agent, and water that is suitable for injection. In another embodiment, the composition comprises a VEGF antagonist, monobasic sodium phosphate, dibasic sodium phosphate, sodium chloride, hydrochloric acid, and sodium hydroxide. In one embodiment, the VEGF antagonist is a pegylated anti-VEGF aptamer, e.g., pegaptanib sodium

In another embodiment, the VEGF antagonist is ranibizumab, bevacizumab, aflibercept or ESBA1008. This invention provides the pharmaceutically acceptable salts of the antagonists. An antagonist of the present invention can possess a sufficiently basic functional group, which can react with any of a number of inorganic and organic acids, to form a pharmaceutically acceptable salt. A pharmaceutically-acceptable acid addition salt is formed from a pharmaceutically-acceptable acid, as is well known in the art. Such salts include the pharmaceutically acceptable salts listed in Journal of Pharmaceutical Science, 66, 2-19 (1977) and The Handbook of Pharmaceutical Salts; Properties, Selection, and Use. P. H. Stahl and C. G. Wermuth (ED.s), Verlag, Zurich (Switzerland) 2002, which are hereby incorporated by reference in their entirety.

Examples of a pharmaceutically acceptable salts include sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, camphorsulfonate, pamoate, phenylacetate, trifluoroacetate, acrylate, chlorobenzoate, dinitrobenzoate, hydroxybenzoate, methoxybenzoate, methylbenzoate, o-acetoxybenzoate, naphthalene-2-benzoate, isobutyrate, phenylbutyrate, a-hydroxybutyrate, butyne-1,4-dicarboxylate, hexyne-1,4-dicarboxylate, caprate, caprylate, cinnamate, glycollate, heptanoate, hippurate, malate, hydroxymaleate, malonate, mandelate, mesylate, nicotinate, phthalate, teraphthalate, propiolate, propionate, phenylpropionate, sebacate, suberate, p-bromobenzenesulfonate, chlorobenzenesulfonate, ethylsulfonate, 2-hydroxyethylsulfonate, methylsulfonate, naphthalene-1-sulfonate, naphthalene-2-sulfonate, naphthalene-1,5-sulfonate, xylenesulfonate, and tartarate salts. The term "pharmaceutically acceptable salt" includes a hydrate of a compound of the invention and also refers to a salt of an antagonist of the present invention having an acidic functional group, such as a carboxylic acid functional group or a hydrogen phosphate functional group, and a base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines, dicyclohexylamine; tributyl amine; pyridine; N-methyl, N-ethylamine; diethylamine; triethylamine; mono-, bis-, or tris-(2-OH-lower alkylamines), such as mono-; bis-, or tris-(2-hydroxyethyl)amine, 2-hydroxy-tert-butylamine, or tris-(hydroxymethyl)methylamine, N,N-di-lower alkyl-N-(hydroxyl-lower alkyl)-amines, such as N,N-dimethyl-N-(2-hydroxyethyl)amine or tri-(2-hydroxyethyl)amine; N-methyl-D-glucamine; and amino acids such as arginine, lysine, and the like. In one embodiment, the pharmaceutically acceptable salt is a sodium salt. In another embodiment, the pharmaceutically acceptable salt is a persodium salt.

The present invention further provides comprising Antagonist A or another pharmaceutically acceptable salt thereof. In one embodiment, the present compositions comprise about 30.0 mg of Antagonist A or another pharmaceutically acceptable salt thereof, about 0.3 mg of monobasic sodium phosphate monohydrate, about 2.1 mg of dibasic sodium phosphate heptahydrate and about 9.0 mg of sodium chloride per about 1 mL. In some embodiments, hydrochloric acid and/or sodium hydroxide are present as needed to adjust the pH of the composition. In some embodiments, the pH is about pH 5.5 to about pH 7.5 or about pH 6.0.

In some embodiments, the compositions comprise about 3% (w/v) of Antagonist A or another pharmaceutically acceptable salt thereof, about 0.03% (w/v) of monobasic sodium phosphate monohydrate, about 0.2% (w/v) of dibasic sodium phosphate heptahydrate, about 0.9% (w/v) of sodium chloride and about 95.9% (w/v) of water. In some embodiments, hydrochloric acid and/or sodium hydroxide are present as needed to adjust the pH of the composition. In some embodiments, the pH is about pH 5.5 to about pH 7.5 or about pH 6.0.

In certain embodiments, the concentration of Antagonist A or another pharmaceutically acceptable salt thereof, a VEGF antagonist (e.g., ranibizumab, bevacizumab, aflibercept, ESBA1008 or pegaptanib sodium), and/or an anti-C5 agent (e.g., ARC1905 or a pharmaceutically acceptable salt thereof) in a composition is about 0.002 mg/mL to about 50 mg/mL. In some embodiments, the concentration of Antagonist A or another pharmaceutically acceptable salt thereof, a VEGF antagonist (e.g., ranibizumab, bevacizumab, aflibercept, ESBA1008 or pegaptanib sodium), and/or an anti-C5 agent (e.g., ARC1905 or a pharmaceutically acceptable salt thereof) in a composition is less than or about 100 mg/mL, less than about 50 mg/mL, less than about 40 mg/mL, less than about 30 mg/mL, less than about 25 mg/mL, less than about 20 mg/mL, less than about 15 mg/mL, less than about 10 mg/mL, or less than about 5 mg/mL. In certain embodiments, the concentration of Antagonist A or another pharmaceutically acceptable salt thereof, a VEGF antagonist (e.g., ranibizumab, bevacizumab, aflibercept, ESBA1008 or pegaptanib sodium), and/or an anti-C5 agent (e.g., ARC1905 or a pharmaceutically acceptable salt thereof) in a composition is about 0.3 mg/mL to about 100 mg/mL, about 0.3 mg/mL to about 50 mg/mL, about 0.3 mg/mL to about 40 mg/mL, about 0.3 mg/mL to about 30 mg/mL, about 0.3 to about 25 mg/mL, about 0.3 mg/mL to about 20 mg/mL, about 0.3 mg/mL to about 15 mg/mL, about 0.3 mg/mL to about 10 mg/mL, about 1 mg/mL to about 100 mg/mL, about 1 mg/mL to about 50 mg/mL, about 1 mg/mL to about 40 mg/mL, about 1 mg/mL to about 30 mg/mL, about 1 mg/mL to about 25 mg/mL, about 1 mg/mL to about 20 mg/mL, about 1 mg/mL to about 15 mg/mL, about 1 mg/mL to about 10 mg/mL, about 1 mg/ mL to about 5 mg/mL, about 5 mg/mL to about 100 mg/mL, or about 5 mg/mL to about 50 mg/mL.

In certain embodiments, methods of the invention comprise administering Antagonist A and optionally one or both of a VEGF antagonist and an anti-C5 agent as a component of a pharmaceutical composition. In one embodiment, the present invention provides compositions comprising an effective amount of: (a) Antagonist A or another pharmaceutically acceptable salt thereof; and (b) a VEGF antagonist or a pharmaceutically acceptable salt thereof. In certain embodiments, the compositions further comprise an effective amount of an anti-C5 agent or a pharmaceutically acceptable salt thereof. In some embodiments, the compositions stabilize one or more of the Antagonist A or another pharmaceutically acceptable salt thereof, the VEGF antagonist, and the anti-C5 agent. In certain embodiments, the Antagonist A or another pharmaceutically acceptable salt thereof, the VEGF antagonist and/or the anti-C5 agent does not adversely affect the activity of the other active agent(s) present in the composition. In particular embodiments, at least about 90% of one or more of the active agents in the composition, e.g., Antagonist A or another pharmaceutically acceptable salt thereof, VEGF antagonist, or anti-C5 agent, is chemically stable when the composition is stored at a temperature of from about 2.0° C to about 8.0° C for at least about twelve weeks.

In particular embodiments, Antagonist A or another pharmaceutically acceptable salt thereof, the VEGF antagonist or the anti-C5 agent is chemically stable when it shows no sign of decomposition or modification resulting in formation of a new chemical entity. In particular embodiments, Antagonist A or another pharmaceutically acceptable salt thereof, the VEGF antagonist or the anti-C5 agent is chemically stable when at least about 50%, at least about 60%, at least about 70%, at least about 80%, at least about 90%, a least about 95%, or at least about 99% of Antagonist A or another pharmaceutically acceptable salt thereof, the VEGF antagonist or the anti-C5 agent shows no sign of decomposition or modification resulting in formation of a new chemical entity, e.g., when stored at a temperature of from about 2.0° C to about 8.0° C for at least about twelve weeks.

In certain embodiments, the Antagonist A or another pharmaceutically acceptable salt thereof does not adversely affect the activity of the VEGF antagonist (e.g., ranibizumab, bevacizumab, aflibercept, pegaptanib sodium, or ESBA1008) or the ARC1905 or a pharmaceutically acceptable salt thereof. In certain embodiments, the VEGF antagonist (e.g., ranibizumab, bevacizumab, aflibercept, pegaptanib sodium, or ESBA1008) does not adversely affect the activity of the Antagonist A or another pharmaceutically acceptable salt thereof, or ARC1905 or a pharmaceutically acceptable salt thereof. In certain embodiments, ARC1905 or a pharmaceutically acceptable salt thereof does not adversely affect the activity of the Antagonist A or another pharmaceutically acceptable salt thereof, or the VEGF antagonist (e.g., ranibizumab, bevacizumab, aflibercept, pegaptanib sodium, or ESBA1008).

In particular embodiments, the compositions comprise Antagonist A or another pharmaceutically acceptable salt thereof; and ranibizumab, bevacizumab, aflibercept, pegaptanib sodium or ESBA 1008, or a pharmaceutically acceptable salt thereof, and the compositions are physically or chemically stable with respect to both active agents at a particular pH or suitable for parenteral administration. In particular embodiments, the compositions comprise Antagonist A or another pharmaceutically acceptable salt thereof; ranibizumab, bevacizumab, aflibercept, pegaptanib sodium or ESBA1008 or a pharmaceutically acceptable salt thereof; and ARC1905 or a pharmaceutically acceptable salt thereof, and the compositions are physically or chemically stable with respect to all active agents at a particular pH or suitable for parenteral administration. In particular embodiments, a composition is physically stable if at least about 50%, at least about 60%, at least about 70%, at least about 80%, at least about 90%, at least about 95%, or at least about 99% of all active agents, i.e., the Antagonist A or another pharmaceutically acceptable salt thereof, the VEGF antagonist, and the anti-C5 agent (when present) present in the composition show no sign of aggregation, precipitation or denaturation upon visual examination of color or clarity, or as measured by UV light scattering or by size exclusion chromatography (SEC) or differential scanning calorimetry (DSC).

In particular embodiments, the compositions of the invention are considered physically stable if after storage the average number of particles detected does not exceed about 50 particles/mL, where the particles have a diameter > about 10 µm and does not exceed 5 particles/mL, where the particles have a diameter > 25 µm, as measured by the Light Obscuration Particle Count Test described in *(788)* Particulate Matter in Injections, Revised Bulletin, Official October 1, 2011, The United States Pharmacopeial Convention.

In particular embodiments, the compositions are considered physically stable if after storage the average number of particles detected does not exceed 50 particles/mL, where the particles have a diameter > 10 µm; does not exceed 5 particles/mL, where the particles have a diameter > 25 µm; and does not exceed 2 particles/mL, where the particles have a diameter > 50 µm, as measured by the microscopic method particle count test described in *(788)* Particulate Matter in Infections, Revised Bulletin, Official October 1, 2011, The United States Pharmacopeial Convention.

In particular embodiments, the compositions comprise Antagonist A or another pharmaceutically acceptable salt thereof, a VEGF antagonist (e.g., ranibizumab, bevacizumab, aflibercept, ESBA1008 or pegaptanib sodium) and, optionally, an anti-C5 agent (e.g., ARC1905 or a pharmaceutically acceptable salt thereof) and are chemically stable for at least eight weeks or at least twelve weeks at 25°C or for at least twelve weeks or at least sixteen weeks or at least 24 weeks at 4°C. In particular embodiments, at least 80% of each of Antagonist A or another pharmaceutically acceptable salt thereof, VEGF antagonist, and anti-C5 agent (if present) show no sign of decomposition or modification resulting in formation of a new chemical entity under at least one of these conditions.

In particular embodiments, compositions comprise the following: (1) Antagonist A or another pharmaceutically acceptable salt thereof; (2) a VEGF antagonist; optionally, (3) an anti-C5 agent; (4) a buffer; optionally, (5) a tonicity modifier; and, optionally, (6) a surfactant. In specific embodiments of such compositions, the buffer is an acetate, phosphate, Tris or histidine buffer, or a mixture thereof; the tonicity modifier is sodium chloride, mannitol, sorbitol, or trehalose, or a mixture thereof; and the surfactant is polysorbate 20. In various embodiments, Antagonist A or another pharmaceutically acceptable salt thereof is present in compositions of the invention at a concentration of about 0.1 mg/mL to about 200 mg/mL; and the VEGF antagonist is present at a concentration of about 0.1 mg/mL to about 200 mg/mL. When present, the anti-C5 agent is present at a concentration of about 0.1 mg/mL to about 200 mg/mL. The buffer is present at a concentration of about 1 mM to about 200 mM; the tonicity modifier is present at a concentration of about 10 mM to about 200 mM (sodium chloride), about 1 % to about 10% (w/v) (sorbitol), or about 1% to about 20% (w/v) (trehalose); and the surfactant, when present, is present at a concentration of about 0.005% to about 0.05% or a concentration of about 0.001% to about 0.05%).

In particular embodiments, the ratio of the concentration (mass of Antagonist A or another pharmaceutically acceptable salt thereof less that of its -R group/volume of composition) of Antagonist A or another pharmaceutically acceptable salt thereof to the concentration (mass/volume of composition) of the VEGF antagonist (e.g., ranibizumab, bevacizumab, aflibercept, pegaptanib sodium, or ESBA1008), ARC1905, or a pharmaceutically acceptable salt thereof, present in the composition is less than, or less than or equal to, 25.0, less than, or less than or equal to, 10.0, less than, or less than or equal to, 9.0, less than, or less than or equal to, 8.0, less than, or less than or equal to, 7.0, less than, or less than or equal to, 6.0, less than, or less than or equal to, 5.0, less than, or less than or equal to, 4.0, less than, or less than or equal to, 3.0, less than, or less than or equal to, 2.0 or less than, or less than or equal to, 1.0. Antagonist A's - R group is depicted in FIG. 1. In particular embodiments, the ratio of the concentration (mass of Antagonist A or another pharmaceutically acceptable salt thereof less that of its -R group/volume of composition) of Antagonist A or another pharmaceutically acceptable salt thereof to the concentration (mass/volume of composition) of the VEGF antagonist (e.g., ranibizumab, bevacizumab, aflibercept, pegaptanib sodium, or ESBA1008), ARC1905, or a pharmaceutically acceptable salt thereof, present in the composition is in the range of about 1 to about 10, about 2 to about 5, about 3 about 4, or about 5. In certain embodiments, the compositions comprise Antagonist A or another pharmaceutically acceptable salt thereof, a VEGF antagonist (e.g., ranibizumab, bevacizumab, aflibercept, pegaptanib sodium, or ESBA1008), and ARC1905 or a pharmaceutically acceptable salt thereof.

In one particular embodiment, the compositions comprise Antagonist A or another pharmaceutically acceptable salt thereof, a VEGF antagonist (e.g., ranibizumab, bevacizumab, aflibercept, ESBA1008, or pegaptanib sodium), and, optionally, an anti-C5 agent (e.g., ARC1905 or a pharmaceutically acceptable salt thereof), wherein the ratio of the concentration of PDGF antagonist to the concentration of VEGF antagonist (and/or anti-C5 agent) is less than 2; and the compositions further comprise sodium chloride at a concentration of about 10 mM to about 200 mM, histidine at a concentration of about 1 mM to about 100 mM, and polysorbate (e.g., polysorbate 20) at a concentration of about 0.005% to about 0.05%, where the pH of the composition is about 5.5 to about 7.0.

In certain embodiments, the compositions comprise one or more of a tonicity modifier, a surfactant, and a buffer suitable to achieve or maintain the particular pH or be suitable for parenteral administration. Appropriate buffers include those described herein as well as others known in the art, such as, e.g., Good's buffers, e.g., MES.

In certain embodiments, the compositions comprise Antagonist A or another pharmaceutically acceptable salt thereof, a VEGF antagonist (e.g., ranibizumab, bevacizumab, aflibercept, ESBA1008 or pegaptanib sodium), and a tonicity modifier that is sorbitol or sodium chloride, or mixtures thereof. In certain embodiments, the compositions further comprise an anti-C5 agent (e.g., ARC1905 or a pharmaceutically acceptable salt thereof). In particular embodiments, the tonicity modifier is sorbitol, and the pH of the composition is about 5.0 to about 8.0, about 5.0 to about 7.0, about 6.0 or about 7.0. In particular embodiments, the tonicity modifier is sodium chloride, and the pH of the composition is about 5.0 to about 8.0, about 5.0 to about 7.0, about 5.5 to about 7.5, about 6.0 to about 8.0, about 8.0, about 7.0, or about 6.0. In certain embodiments, the tonicity modifier is sorbitol at about 1% to about 10 % (w/v), or about 1% (w/v), about 2% (w/v), about 3% (w/v), about 4% (w/v), about 5% (w/v), about 6% (w/v), about 7% (w/v), about 8% (w/v), about 9% (w/v), or about 10% (w/v). In particular embodiments, the tonicity modifier is sodium chloride at a concentration of about 10 mM to about 200 mM, about 50 mM to 200 mM, about 75 mM to about 200 mM, about 50 mM to about 150 mM, about 100 mM, about 110 mM, about 120 mM, about 130 mM about 140 mM or about 150 mM. In one embodiment, the tonicity modifier is sodium chloride at a concentration of about 130 mM. In other embodiments, the tonicity modifier is sodium chloride at a concentration of about 75 mM or about 120 mM. With respect to tonicity modifier concentration, "mM" refers to milimoles of the tonicity modifier per liter of composition.

In certain embodiments, the compositions comprise Antagonist A or another pharmaceutically acceptable salt thereof, a VEGF antagonist (e.g., ranibizumab, bevacizumab, aflibercept, ESBA1008 or pegaptanib sodium), and a buffer capable of achieving or maintaining the pH of the composition within a desired range. In certain embodiments, the compositions further comprise an anti-C5 agent (e.g., ARC1905 or a pharmaceutically acceptable salt thereof). In certain embodiments, the compositions comprise histidine (e.g., L-histidine or a pharmaceutically acceptable salt thereof) or phosphate as a buffer, e.g., sodium phosphate, potassium phosphate, or both. In certain embodiments, the buffer is present at a concentration of about 1 mM to about 200 mM, about 1 mM to about 150 mM, about 1 mM to about 20 mM, about 1 mM to about 10 mM, about 2 mM to about 100 mM, about 2 mM to about 20 mM, about 5 mM to about 20 mM, or about 10 mM. In particular embodiments, the pH of the buffered composition is about 5.0 to about 8.0, about 5.0 to about 7.0, about 5.5 to about 7.5, about 5.5 to about 7.0, or about 6.0. In one embodiment, the buffered composition has a pH of about 5.5 to about 7.0. In certain embodiments, the buffer comprises histidine at a concentration of about 1 mM to about 200 mM, about 1 mM to about 150 mM, about 2 mM to about 100 mM, about 5 mM to about 20 mM, or about 10 mM, and the buffered composition has a pH of about 5.5 to about 7.0, or about 6.0. In one particular embodiment, the buffer comprises histidine at a concentration of about 10 mM and the pH of the histidine-buffered composition is about 6.0. With respect to buffer concentration, "mM" refers to millimoles of buffer (e.g., histidine) per liter of composition.

In certain embodiments, the compositions comprise Antagonist A or another pharmaceutically acceptable salt thereof, a VEGF antagonist (e.g., ranibizumab, bevacizumab, aflibercept, ESBA1008 or pegaptanib sodium), and a buffer that comprises phosphate, alone or in combination with histidine. In certain embodiments, the compositions further comprise an anti-C5 agent (e.g., ARC1905 or a pharmaceutically acceptable salt thereof). The phosphate buffer may be, e.g., a sodium phosphate or potassium phosphate buffer. In certain embodiments, the buffer comprises phosphate at a concentration of about 1 mM to about 200 mM, about 1 mM to about 50 mM, about 2 mM to about 200 mM, about 2 mM to about 50 mM, about 5 mM to about 200 mM, about 5 mM to about 100 mM, about 5 mM to about 50 mM, about 10 mM to about 150 mM, about 10 mM to about 100 mM, about 5 mM, about 10 mM, about 25 mM, or about 50 mM. In particular embodiments, the pH of the buffered composition is about 5.0 to about 8.0, about 6.0 to about 8.0, about 5.5 to about 7.5, about 5.5 to about 7.0, about 6.0, about 7.0, or about 8.0. In one embodiment, the buffer comprises phosphate, and the buffered composition has a pH of about 6.0 to about 8.0. In certain embodiments, the buffer comprises phosphate at a concentration of about 5 mM to about 200 mM, about 5 mM to about 150 mM, about 5 mM to about 100 mM, about 5 mM, about 8 mM, about 10 mM, about 25 mM, or about 50 mM, and the buffered composition has a pH of about 5.5 to about 7.5, about 5.5 to about 7.0, or about 6.0. In one particular embodiment, the buffer comprises phosphate at a concentration of about 10 mM, and the buffered composition has a pH of about 6.2.

In certain embodiments, the compositions comprise Antagonist A or another pharmaceutically acceptable salt thereof), a VEGF antagonist (e.g., ranibizumab, bevacizumab, aflibercept, ESBA1008 or pegaptanib sodium), and a surfactant. In certain embodiments, the compositions further comprise an anti-C5 agent (e.g., ARC1905 or a pharmaceutically acceptable salt thereof). In particular embodiments, the surfactant is polysorbate 20 at a concentration of about 0.001 % (w/v) to about 0.05% (w/v), about 0.002% (w/v) to about 0.05% (w/v), about 0.005% (w/v) to about 0.05% (w/v)*,* about 0.01% (w/v) to about 0.05% (w/v), or about 0.02% (w/v).

In one embodiment, the compositions comprise Antagonist A or another pharmaceutically acceptable salt thereof, a VEGF antagonist (e.g., ranibizumab, bevacizumab, aflibercept, ESBA1008 or pegaptanib sodium), histidine, and NaCl. In certain embodiments, the compositions further comprise an anti-C5 agent (e.g., ARC 1905 or a pharmaceutically acceptable salt thereof). The composition may further comprise polysorbate.

In certain embodiments, the compositions comprise an effective amount of: (a) about 0.3 mg/mL to about 30 mg/mL of Antagonist A or another pharmaceutically acceptable salt thereof; (b) about 0.5 mg/mL to about 20 mg/mL of a VEGF antagonist (e.g., ranibizumab, bevacizumab, aflibercept, ESBA1008 or pegaptanib sodium); and one or both of: (c) a buffer capable of achieving or maintaining the pH of the compositions at about pH 5.0 to about pH 8.0; and (d) a tonicity modifier. In certain embodiments, the compositions further comprise (e) about 0.3 mg/mL to about 30 mg/mL of an anti-C5 agent (e.g., ARC1905 or a pharmaceutically acceptable salt thereof). In certain embodiments, the buffer is about 1 mM to about 20 mM L-histidine or about 1 mM to about 20 mM sodium phosphate, and the tonicity modifier is about 10 mM to about 200 mM NaCl, about 1% to about 20% (w/v) sorbitol, or about 1% to about 20% (w/v) trehalose. In particular embodiments, the compositions further comprise: (f) about 0.001% (w/v) to about 0.05% (w/v) surfactant.

In certain embodiments, the compositions comprise: (a) about 0.3 mg/mL to about 30 mg/mL of Antagonist A or another pharmaceutically acceptable salt thereof; and (b) about 0.5 mg/mL to about 20 mg/mL of a VEGF antagonist (e.g., ranibizumab, bevacizumab, aflibercept, ESBA1008 or pegaptanib sodium). In certain embodiments, the compositions further comprise (c) about 0.3 mg/mL to about 30 mg/mL of an anti-C5 agent (e.g., ARC1905 or a pharmaceutically acceptable salt thereof). In certain embodiments, any of these the compositions further comprise one or both of: (d) about 1 mM to about 20 mM L-histidine; and (e) about 10 mM to about 200 mM NaCl. In further embodiments, the compositions further comprise: (f) about 0.001% (w/v) to about 0.05% (w/v) surfactant, which is optionally polysorbate. In a particular embodiment, the compositions comprise: (a) about 0.3 mg/mL to about 30 mg/mL of Antagonist A or another pharmaceutically acceptable salt thereof; (b) about 0.5 mg/mL to about 20 mg/mL of a VEGF antagonist (e.g., ranibizumab, bevacizumab, aflibercept, ESBA1008 or pegaptanib sodium); (c) about 1 mM to about 20 mM L-histidine; and (d) about 10 mM to about 200 mM NaCl, wherein the pH of the compositions is about pH 5.0 to about pH 7.0. In certain embodiments, the compositions further comprise (e) about 0.3 mg/mL to about 30 mg/mL of an anti-C5 agent (e.g., ARC1905 or a pharmaceutically acceptable salt thereof). In certain embodiments, the compositions further comprise: (f) about 0.01% (w/v) polysorbate 20.

In certain embodiments, compositions comprise: (a) about 1.0 mg/mL to about 100 mg/mL, or about 5.0 mg/mL to about 50 mg/mL of Antagonist A or another pharmaceutically acceptable salt thereof); and (b) about 1.0 mg/mL to about 50 mg/mL of a VEGF antagonist (e.g., ranibizumab, bevacizumab, aflibercept, ESBA 1008 or pegaptanib sodium). In certain embodiments, the compositions further comprise (c) about 1.0 mg/mL to about 100 mg/mL of an anti-C5 agent (e.g., ARC1905 or a pharmaceutically acceptable salt thereof). In other embodiments, any of the compositions further comprise one or both of (d) about 1 mM to about 20 mM L-histidine; and (e) about 10 mM to about 200 mM NaCl. In further embodiments, any of the compositions further comprise: (f) about 0.001% (w/v) to about 0.05% (w/v) surfactant, which is optionally polysorbate.

In certain embodiments, compositions comprise: (a) about 0.3 mg/mL to about 30 mg/mL of Antagonist A or another pharmaceutically acceptable salt thereof); (b) about 0.5 mg/mL to about 20 mg/mL of a VEGF antagonist (e.g., ranibizumab, bevacizumab, aflibercept, ESBA1008 or pegaptanib sodium); and one or both of (c) a buffer capable of achieving or maintaining the pH of the composition to about pH 5.0 to about pH 8.0; and (d) a tonicity modifier. In certain embodiments, the compositions further comprise about 0.3 mg/mL to about 30 mg/mL of an anti-C5 agent (e.g., ARC1905 or a pharmaceutically acceptable salt thereof). In particular embodiments, the buffer, where present, is about 1 mM to about 20 mM L-histidine or about 1 mM to about 20 mM sodium phosphate; and the tonicity modifier, where present, is about 10 mM to about 200 mM NaCl, about 1% to about 20% (w/v) sorbitol, or about 1% to about 20% (w/v) trehalose. In certain embodiments, the buffer is about 1 mM to about 20 mM L-histidine; and the tonicity modifier is about 10 mM to about 200 mM NaCl, wherein the pH of the compositions is about pH 5.0 to about pH 7.0.

Any of the compositions can also comprise a surfactant, e.g., about 0.001% (w/v) to about 0.05% (w/v) surfactant.

In certain embodiments the compositions comprise: (a) about 3 mg/mL to about 90 mg/mLAntagonist A or another pharmaceutically acceptable salt thereof; (b) about 1.0 mg/mL to about 30 mg/mL of a VEGF antagonist (e.g., ranibizumab, bevacizumab, aflibercept, ESBA1008 or pegaptanib sodium); and one or both of (c) a buffer capable of achieving or maintaining the pH of the compositions to about pH 5.0 to about pH 8.0; and (d) a tonicity modifier. In certain embodiments, any of the compositions further comprises (e) about 3 mg/mL to about 90 mg/mL of an anti-C5 agent (e.g., ARC 1905 or a pharmaceutically acceptable salt thereof). In particular embodiments, the buffer, where present, comprises about 1 mM to about 100 mM sodium phosphate or about 1.0 mM to about 10 mM histidine.HCl; and the tonicity modifier, where present, is about 0.5% (w/v) to about 10% (w/v) trehalose.

In certain embodiments, a composition of the invention comprises: (a) about 0.3 mg/mL to about 30 mg/mL Antagonist A or another pharmaceutically acceptable salt thereof; (b) about 0.5 mg/mL to about 20 mg/mL ranibizumab or a pharmaceutically acceptable salt thereof; and one or both of: (c) a buffer capable of achieving or maintaining the pH of the composition at about pH 5.0 to about pH 8.0; and (d) a tonicity modifier. In certain embodiments, the buffer is about 1 mM to about 20 mM L-histidine or about 1 mM to about 20 mM sodium phosphate, and the tonicity modifier is about 10 mM to about 200 mM NaCl, about 1% to about 20% (w/v) sorbitol, or about 1% to about 20% (w/v) trehalose. In particular embodiments, the composition of the invention further comprises: (e) about 0.001% (w/v) to about 0.05% (w/v) surfactant. In particular embodiments, the composition further comprises: (f) an anti-C5 agent, another PDGF antagonist, or another VEGF antagonist. In particular embodiments, the anti-C5 agent is ARC 186, ARC 187, or ARC1905, and the other VEGF antagonist is bevacizumab or aflibercept.

In certain embodiments, a composition of the invention comprises: (a) about 0.3 mg/mL to about 30 mg/mL Antagonist A or another pharmaceutically acceptable salt thereof; and (b) about 0.5 mg/mL to about 25 mg/mL bevacizumab or a pharmaceutically acceptable salt thereof, and one or both of: (c) a buffer capable of achieving or maintaining the pH of the composition at about pH 5.0 to about pH 8.0; and (d) a tonicity modifier. In certain embodiments, the buffer is about 5 mM to about 200 mM sodium phosphate or about 5 mM to about 200 mM Tris.HCl, and the tonicity modifier is about 10 mM to about 200 mM NaCl, about 1% to about 20% (w/v) sorbitol, or about 1% to about 20% (w/v) trehalose. In particular embodiments, the composition of the invention further comprises: (e) about 0.001% (w/v) to about 0.05% (w/v) surfactant. In particular embodiments, the composition further comprises: (f) an anti-C5 agent, another PDGF antagonist, and/or another VEGF antagonist. In particular embodiments, the anti-C5 agent is ARC186, ARC187, or ARC1905, and the other VEGF antagonist is ranibizumab or aflibercept.

In certain embodiments, a composition of the invention comprises: (a) about 0.3 mg/mL to about 30 mg/mL Antagonist A or another pharmaceutically acceptable salt thereof; (b) about 5 mg/mL to about 40 mg/mL aflibercept or a pharmaceutically acceptable salt thereof; and one or more of: (c) a buffer capable of achieving or maintaining the pH of the composition at about pH 5.0 to about pH 8.0; (d) a tonicity modifier; and (e) 0 to about 10% (w/v) sucrose. In certain embodiments, the buffer is about 5 mM to about 50 mM phosphate, and the tonicity modifier is about 10 mM to about 200 mM NaCl. In particular embodiments, the composition of the invention further comprises: (f) about 0.001% (w/v) to about 0.05% (w/v) surfactant. In particular embodiments, the composition further comprises: (g) an anti-C5 agent, another PDGF antagonist, and/or another VEGF antagonist. In particular embodiments, the anti-C5 agent is ARC 186, ARC 187, or ARC1905, and the other VEGF antagonist is ranibizumab or bevacizumab.

In certain embodiments, a composition of the invention comprises: (a) about 3 mg/mL to about 90 mg/mL Antagonist A or another pharmaceutically acceptable salt thereof; (b) about 1.0 mg/mL to about 30 mg/mL ranibizumab or a pharmaceutically acceptable salt thereof; and one or both of: (c) a buffer capable of achieving or maintaining the pH of the composition at about pH 5.0 to about pH 8.0; and (d) a tonicity modifier. In certain embodiments, the buffer comprises about 1 mM to about 100 mM sodium phosphate or about 1.0 mM to about 10 mM histidine.HCl, and the tonicity modifier is about 0.5% (w/v) to about 10% (w/v) trehalose. In particular embodiments, the composition further comprises: (e) an anti-C5 agent, another PDGF antagonist, and/or another VEGF antagonist. In particular embodiments, the anti-C5 agent is ARC186, ARC187, or ARC1905, and the other VEGF antagonist is bevacizumab or aflibercept.

Illustrative compositions include F1-F31, as described in Tables 3 and 4. Illustrative compositions are also described in PCT Application Publication No. WO2013/181495. Any of these compositions may further comprise an anti-C5 agent, such as ARC 1905 or a pharmaceutically acceptable salt thereof.

**Table 3. Composition Matrix for Illustrative Antagonist A:Ranibizumab Compositions**

| **Comp.** | **Buffer** | **pH** | **Tonicity Modifier** | **[Ant. A] (mg/mL)** | **[ran.] (mg/mL)** | **Polysorbate 20 (% w/v)** |
|---|---|---|---|---|---|---|
| F1 | 10 mM Sodium Phosphate | 7.3 | 150 mM NaCl | 3 | 0 | 0% |
| F2 | 10 mM Sodium Acetate | 5.0 | 5% (w/v) Sorbitol | 3 | 5 | 0.01% |
| F3 | 10 mM Sodium Acetate | 5.0 | 130 mM NaCl | 3 | 5 | 0.01% |
| F4 | 10 mM Histidine.HCl | 5.5 | 10% (w/v) Trehalose | 0 | 5 | 0.01% |
| F5 | 10 mM Histidine.HCl | 6.0 | 5% (w/v) Sorbitol | 3 | 5 | 0.01% |
| F6 | 10 mM Histidine.HCl | 6.0 | 130 mM NaCl | 3 | 5 | 0.01% |
| F7 | 10 mM Sodium Phosphate | 7.0 | 5% (w/v) Sorbitol | 3 | 5 | 0.01% |
| F8 | 10 mM Sodium Phosphate | 7.0 | 130 mM NaCl | 3 | 5 | 0.01% |
| F9 | 10 mM Tris.HCl | 8.0 | 5% (w/v) Sorbitol | 3 | 5 | 0.01% |
| F10 | 10 mM Tris.HCl | 8.0 | 130 mM NaCl | 3 | 5 | 0.01% |
| F11 | 5 mM Sodium Phosphate + 5 mM Histidine | 6.5 | 75 mM NaCl + 5% (w/v) Trehalose | 3 | 5 | 0.005% |
| F27 | 10 mM Sodium Phosphate | 7.3 | 150 mM NaCl | 30 | 0 | 0% |
| F28 | 10 mM Histidine.HCl | 5.5 | 10% (w/v) Trehalose | 0 | 10 | 0.01% |
| F29 | 10 mM Histidine.HCl | 5.5 | 10% (w/v) Trehalose | 0 | 40 | 0.01% |
| F30 | 5 mM Sodium Phosphate + 5 mM Histidine.HCl | | 75 mM NaCl + 5% (w/v) Trehalose | 15 | 5 | 0.005% |
| F31 | 8 mM Sodium Phosphate + 2 mM Histidine.HCl | | 120 mM NaCl + 2% (w/v) Trehalose | 24 | 8 | 0.002% |

| | | | | | | |
|---|---|---|---|---|---|---|
| "Ant. A" is Antagonist A; "ran." is ranibizumab | | | | | | |

**Table 4. Composition Matrix for Illustrative Antagonist A:Bevacizumab Compositions**

| **Comp.** | **Buffer** | **pH** | **Tonicity Modifier** | **Antagonist A Concentration (mg/mL, oligo wt.)** | **Bevacizumab Concentration (mg/mL)** | **Surfactant** |
|---|---|---|---|---|---|---|
| F12 | 10 mM Phosphate | 7.3 | 150 mM Sodium Chloride | 30 | 0.0 | 0% |
| F13 | 50 mM Acetate | 4 | 5% (w/v) Sorbitol | 3 | 12.5 | 0.02% Polysorbate 20 |
| F14 | 50 mM Acetate | 4 | 130 mM Sodium Chloride | 3 | 12.5 | 0.02% Polysorbate 20 |
| F15 | 50 mM Acetate | 5 | 5% (w/v) Sorbitol | 3 | 12.5 | 0.02% Polysorbate 20 |
| F16 | 50 mM Acetate | 5 | 130 mM Sodium Chloride | 3 | 12.5 | 0.02% Polysorbate 20 |
| F17 | 50 mM Phosphate | 6 | 5% (w/v) Sorbitol | 3 | 12.5 | 0.02% Polysorbate 20 |
| F18 | 50 mM Phosphate | 6.2 | 6% (w/v) Trehalose | 0 | 12.5 | 0.02% Polysorbate 20 |
| F19 | 50 mM Phosphate | 6 | 130 mM Sodium Chloride | 3 | 12.5 | 0.02% Polysorbate 20 |
| F20 | 50 mM Phosphate | 7 | 5% (w/v) Sorbitol | 3 | 12.5 | 0.02% Polysorbate 20 |
| F21 | 50 mM Phosphate | 7 | 130 mM Sodium Chloride | 3 | 12.5 | 0.02% Polysorbate 20 |
| F22 | 50 mM Tris | 8 | 5% (w/v) Sorbitol | 3 | 12.5 | 0.02% Polysorbate 20 |
| F23 | 50 mM Tris | 8 | 130 mM Sodium Chloride | 3 | 12.5 | 0.02% Polysorbate 20 |
| F24 | 30 mM Phosphate | 6.3 | 75 mM sodium Chloride + 3% (w/v) Trehalose | 15 | 12.5 | 0.02% Polysorbate 20 |
| F25 | 10 mM Phosphate | 7.3 | 150 mM Sodium Chloride | 3 | 0.0 | 0% |
| F26 | 30 mM Phosphate | 6.3 | 75 mM sodium Chloride + 3% (w/v) Trehalose | 3 | 12.5 | 0.02% Polysorbate 20 |

### Administration and Dosage

The methods or compositions according to the invention can be administered alone or in conjunction with another therapy and can be provided at home, a doctor's office, a clinic, a hospital's outpatient department, or a hospital. Treatment can begin at a hospital so that the doctor can observe the therapy's effects closely and make any adjustments that are needed. The duration of the administration can depend on the type of ophthalmological disease or disorder being treated or prevented, the age and condition of the subject, the stage and type of the subject's disease or disorder, and how the subject responds to the treatment. Additionally, a subject having a greater risk of developing an ophthalmological disease or disorder (e.g., a diabetic patient) can receive treatment to inhibit or delay the onset of symptoms. In one embodiment, the present methods or compositions allow for the administration of a relatively lower dose of each antagonist.

The dosage and frequency of administration of each antagonist can be controlled independently. For example, one antagonist can be administered three times per day, while the other antagonist can be administered once per day. Administration can be performed in on-and-off cycles that include rest periods so that the subject's body has a chance to recover from a side effect, if any. The antagonists can also be present in the same composition.

In other embodiments, Antagonist A or another pharmaceutically acceptable salt thereof and optionally, a VEGF antagonist and/or anti-C5 agent are administered prior to, during, and/or after another treatment. In one embodiment, Antagonist A or another pharmaceutically acceptable salt thereof and the VEGF antagonist and/or anti-C5 agent are administered concurrently, such as in a co-formulation, prior to, during, and/or after the other treatment. In other embodiments, Antagonist A or another pharmaceutically acceptable salt thereof and the VEGF antagonist are administered sequentially, prior to, during, and/or after the other treatment. In some embodiments, Antagonist A or another pharmaceutically acceptable salt thereof is administered prior to the administration of the VEGF antagonist. In other embodiments, Antagonist A or another pharmaceutically acceptable salt thereof is administered subsequent to the administration of the VEGF antagonist. In some embodiments, the other treatment is performing surgery. Examples of other treatment include pneumatic retinopexy, laser retinopexy, scleral buckling, and pars plana vitrectomy (PPV), laser photocoagulation, or cryotherapy.

Administration of a composition disclosed herein with performing another treatment can improve retinal attachment success, improve visual acuity, reduce choroidal neovascularization or stabilize vision to a degree that is greater than performing the other treatment alone. For example, in some embodiments, the administration of both Antagonist A or another pharmaceutically acceptable salt thereof with performing another treatment can improve retinal attachment success, improve visual acuity, or stabilize vision to a degree that is greater than an additive effect of both Antagonist A or another pharmaceutically acceptable salt thereof with performing the other treatment. In some embodiments, the synergistic effect is in reducing the size or growth of a tumor (e.g., in treating or preventing VHL disease, retinal capillary hemangioma, or von Hippel angioma). In some embodiments, the synergistic effect is reducing or inhibiting scarring or fibrosis (e.g., ocular scarring of fibrosis, such as subretinal fibrosis).

Administration of both Antagonist A or another pharmaceutically acceptable salt thereof and the VEGF antagonist can improve retinal attachment success, improve visual acuity, or stabilize vision to a degree that is greater than administration of Antagonist A or another pharmaceutically acceptable salt thereof or the VEGF antagonist. In some embodiments, the administration of Antagonist A or another pharmaceutically acceptable salt thereof and the VEGF antagonist can have a synergistic effect in treating or preventing an ophthalmological disease or disorder. For example, the administration of both Antagonist A or another pharmaceutically acceptable salt thereof and the VEGF antagonist can improve retinal attachment success, improve visual acuity, or stabilize vision to a degree that is greater than an additive effect of administering both Antagonist A or another pharmaceutically acceptable salt thereof and the VEGF antagonist. In some embodiments, the synergistic effect is in reducing the size or growth of a tumor (e.g., in treating or preventing VHL disease, retinal capillary hemangioma, or von Hippel angioma). In some embodiments, the synergistic effect is reducing or inhibiting scarring or fibrosis (e.g., ocular scarring of fibrosis, such as subretinal fibrosis).

In some embodiments, the methods comprise administering Antagonist A or another pharmaceutically acceptable salt thereof, VEGF antagonist and anti-C5 agent, in which two or more of Antagonist A or another pharmaceutically acceptable salt thereof, the VEGF antagonist and the anti-C5 agent are present in the same composition. In certain embodiments, the PDGF antagonist and the VEGF antagonist are present in the same composition; in certain embodiments, Antagonist A or another pharmaceutically acceptable salt thereof and the anti-C5 agent are present in the same composition; and in certain embodiments, the VEGF antagonist and the anti-C5 agent are present in the same composition. In some embodiments, all three of Antagonist A or another pharmaceutically acceptable salt thereof, the VEGF antagonist and the anti-C5 agent are present in the same composition.

In some embodiments, Antagonist A or another pharmaceutically acceptable salt thereof, the VEGF antagonist and the anti-C5 agent are administered sequentially. In one embodiment, Antagonist A or another pharmaceutically acceptable salt thereof is administered prior to the VEGF antagonist or the anti-C5 agent. In one embodiment, the VEGF antagonist is administered prior to Antagonist A or another pharmaceutically acceptable salt thereof or the anti-C5 agent. In one embodiment, the anti-C5 agent is administered prior to the VEGF antagonist or Antagonist A or another pharmaceutically acceptable salt thereof. In one embodiment, Antagonist A or another pharmaceutically acceptable salt thereof is administered prior to the VEGF antagonist and anti-C5 agent. In one embodiment, the VEGF antagonist is administered prior to Antagonist A or another pharmaceutically acceptable salt thereof and he - C5 agent. In one embodiment, the anti-C5 agent is administered prior to the VEGF antagonist and PDGF antagonist.

In certain embodiments, the subject is administered two or more active agents (e.g., Antagonist A or another pharmaceutically acceptable salt thereof and a VEGF antagonist) in a staggered dosing regimen, wherein one or more of the two or more active agents is administered before another one or more of the two or more active agents is administered to the subject.

In certain embodiments, the one or more active agent(s) is administered at least one day before the other one or more active agent(s). Accordingly, in some embodiments the present methods comprise administering on one or more days Antagonist A or another pharmaceutically acceptable salt thereof, one or more VEGF antagonists or one or more anti-C5 agents.

In one embodiment, the order of administration is: Antagonist A or another pharmaceutically acceptable salt thereof, followed by VEGF antagonist, followed by anti-C5 agent. In another embodiment, the order of administration is: Antagonist A or another pharmaceutically acceptable salt thereof, followed by anti-C5 agent, followed by VEGF antagonist. In another embodiment, the order of administration is: VEGF antagonist, followed by anti-C5 agent, followed by Antagonist A or another pharmaceutically acceptable salt thereof. In another embodiment, the order of administration is: VEGF antagonist, followed by Antagonist A or another pharmaceutically acceptable salt thereof, followed by anti-C5 agent. In yet another embodiment the order of administration is: anti-C5 agent, followed by Antagonist A or another pharmaceutically acceptable salt thereof, followed by VEGF antagonist. In another embodiment the order of administration is: anti-C5 agent, followed by VEGF antagonist, followed by PDGF antagonist.

In some embodiments, the Antagonist A or another pharmaceutically acceptable salt thereof and the VEGF antagonist are administered concurrently, and the anti-C5 agent is administered prior to or subsequent to administration of the PDGF antagonist and VEGF antagonist. In some embodiments, Antagonist A or another pharmaceutically acceptable salt thereof and the anti-C5 agent are administered concurrently, and the VEGF antagonist is administered prior to or subsequent to administration of Antagonist A or another pharmaceutically acceptable salt thereof and the VEGF antagonist. In some embodiments, the VEGF antagonist and anti-C5 agent are administered concurrently, and Antagonist A or another pharmaceutically acceptable salt thereof is administered prior to or subsequent to administration of the anti-C5 agent and VEGF antagonist.

In other embodiments, the order of administration is: Antagonist A or another pharmaceutically acceptable salt thereof, followed by VEGF antagonist and anti-C5 agent, wherein the VEGF antagonist and anti-C5 agent are present in the same composition. In another embodiment, the order of administration is: VEGF antagonist, followed by anti-C5 agent and Antagonist A or another pharmaceutically acceptable salt thereof, wherein the anti-C5 agent and PDGF antagonist are present in the same composition. In yet another embodiment the order of administration is: anti-C5 agent, followed by Antagonist A or another pharmaceutically acceptable salt thereof and VEGF antagonist, wherein the PDGF antagonist and VEGF antagonist are present in the same composition.

In still other embodiments, the order of administration is: Antagonist A or another pharmaceutically acceptable salt thereof and VEGF antagonist, wherein Antagonist A or another pharmaceutically acceptable salt thereof and the VEGF antagonist are present in the same composition, followed by anti-C5 agent. In another embodiment, the order of administration is: Antagonist A or another pharmaceutically acceptable salt thereof and anti-C5 agent, wherein Antagonist A or another pharmaceutically acceptable salt thereof and the anti-C5 agent are present in the same composition, followed by VEGF antagonist. In another embodiment, the order of administration is: VEGF antagonist and anti-C5 agent, wherein the VEGF antagonist and anti-C5 agent are present in the same composition, followed by Antagonist A or another pharmaceutically acceptable salt thereof.

For example, Antagonist A or another pharmaceutically acceptable salt thereof can be administered prior to or subsequent to administration of a VEGF antagonist and/or an anti-C5 agent; a VEGF antagonist can be administered prior to or subsequent to administration of Antagonist A or another pharmaceutically acceptable salt thereof and/or anti-C5 agent; or an anti-C5 agent can be administered prior to or subsequent to administration of Antagonist A or another pharmaceutically acceptable salt thereof and/or a VEGF antagonist.

In some embodiments, the present methods comprise administering a first agent prior to administering a second agent. In some embodiments, the present methods comprise administering a first agent prior to administering a second agent and administering the second agent prior to administering a third agent.

In some embodiments, the present methods comprise concurrently administering a first agent and a second agent. In some embodiments, the present methods comprise concurrently administering a first agent and a second agent prior to administering a third agent.

In some embodiments, the present methods comprise administering a first agent prior to concurrently administering a second agent and third agent.

In some embodiments, the present methods comprise concurrently administering a first agent, a second agent and a third agent.

Illustrative groups of first agent, second agent and third agent are set forth below in Tables 5 and 6.

**Table 5**

| **Group** | **First Agent** | **Second Agent** | **Third Agent** |
|---|---|---|---|
| A | Antagonist A or another pharmaceutically acceptable salt thereof | VEGF antagonist | Anti-C5 Agent |
| B | Antagonist A or another pharmaceutically acceptable salt thereof | Anti-C5 Agent | VEGF antagonist |
| C | VEGF antagonist | Antagonist A or another pharmaceutically acceptable salt thereof | Anti-C5 Agent |
| D | VEGF antagonist | Anti-C5 Agent | Antagonist A or another pharmaceutically acceptable salt thereof |
| E | Anti-C5 Agent | Antagonist A or another pharmaceutically acceptable salt thereof | VEGF antagonist |
| F | Anti-C5 Agent | VEGF antagonist | Antagonist A or another pharmaceutically acceptable salt thereof |

**Table 6**

| **Group** | **First Agent** | **Second Agent** | **Third Agent** |
|---|---|---|---|
| A | Antagonist A | ranibizumab | ARC1905 |
| B | Antagonist A | bevacizumab | ARC1905 |
| C | Antagonist A | aflibercept | ARC1905 |
| D | Antagonist A | pegaptanib sodium | ARC1905 |
| E | Antagonist A | ESBA1008 | ARC1905 |
| F | Antagonist A | ARC1905 | ranibizumab |
| G | Antagonist A | ARC1905 | bevacizumab |
| H | Antagonist A | ARC1905 | aflibercept |
| I | Antagonist A | ARC1905 | pegaptanib sodium |
| J | Antagonist A | ARC1905 | ESBA1008 |
| K | ranibizumab | Antagonist A | ARC1905 |
| L | bevacizumab | Antagonist A | ARC1905 |
| M | aflibercept | Antagonist A | ARC1905 |
| N | pegaptanib sodium | Antagonist A | ARC1905 |
| O | ESBA1008 | Antagonist A | ARC1905 |
| P | ranibizumab | ARC1905 | Antagonist A |
| Q | bevacizumab | ARC1905 | Antagonist A |
| R | aflibercept | ARC 1905 | Antagonist A |
| S | pegaptanib sodium | ARC1905 | Antagonist A |
| T | ESBA1008 | ARC1905 | Antagonist A |
| U | ARC1905 | Antagonist A | ranibizumab |
| V | ARC1905 | Antagonist A | bevacizumab |
| W | ARC1905 | Antagonist A | aflibercept |
| X | ARC1905 | Antagonist A | pegaptanib sodium |
| Y | ARC 1905 | Antagonist A | ESBA1008 |
| Z | ARC1905 | ranibizumab | Antagonist A |
| AA | ARC1905 | bevacizumab | Antagonist A |
| AB | ARC1905 | aflibercept | Antagonist A |
| AC | ARC1905 | pegaptanib sodium | Antagonist A |
| AD | ARC1905 | ESBA1008 | Antagonist A |

In some embodiments, the present methods comprise administering Antagonist A or another pharmaceutically acceptable salt thereof and two or more VEGF antagonists. In some embodiments, the present methods comprise administering Antagonist A or another pharmaceutically acceptable salt thereof and two or more anti-C5 agents. In some embodiments, the present methods comprise administering a VEGF antagonist and two or more anti-C5 agents.

In some embodiments, the present methods comprise administering Antagonist A or another pharmaceutically acceptable salt thereof prior to administering two or more VEGF antagonists. In some embodiments, the present methods comprise administering Antagonist A or another pharmaceutically acceptable salt thereof prior to administering a first VEGF antagonist and administering the first VEGF antagonist prior to administering a second VEGF antagonist.

In some embodiments, the present methods comprise concurrently administering Antagonist A or another pharmaceutically acceptable salt thereof and a VEGF antagonist. In some embodiments, the present methods comprise concurrently administering Antagonist A or another pharmaceutically acceptable salt thereof and a first VEGF antagonist prior to administering a second VEGF antagonist.

In some embodiments, the present methods comprise administering Antagonist A or another pharmaceutically acceptable salt thereof prior to concurrently administering a first VEGF antagonist and a second VEGF antagonist.

In some embodiments, the present methods comprise concurrently administering Antagonist A or another pharmaceutically acceptable salt thereof, a first VEGF antagonist and a second VEGF antagonist.

In some embodiments, the present methods comprise administering a VEGF antagonist prior to administering two PDGF antagonists (e.g., Antagonist A or another pharmaceutically acceptable salt thereof and another PDGF antagonist). In some embodiments, the present methods comprise administering a VEGF antagonist prior to administering a first PDGF antagonist and administering the first PDGF antagonist prior to administering a second PDGF antagonist.

In some embodiments, the present methods comprise concurrently administering a VEGF antagonist and Antagonist A or another pharmaceutically acceptable salt thereof. In some embodiments, the present methods comprise concurrently administering a VEGF antagonist and a first PDGF antagonist prior to administering a second PDGF antagonist.

In some embodiments, the present methods comprise administering a VEGF antagonist prior to concurrently administering a first PDGF antagonist and a second PDGF antagonist.

In some embodiments, the present methods comprise concurrently administering a VEGF antagonist, a first PDGF antagonist and a second PDGF antagonist.

In some embodiments, the present methods comprise administering Antagonist A or another pharmaceutically acceptable salt thereof prior to administering two or more anti-C5 agents. In some embodiments, the present methods comprise administering Antagonist A or another pharmaceutically acceptable salt thereof prior to administering a first anti-C5 agent and administering the first anti-C5 agent prior to administering a second anti-C5 agent.

In some embodiments, the present methods comprise concurrently administering Antagonist A or another pharmaceutically acceptable salt thereof and an anti-C5 agent. In some embodiments, the present methods comprise concurrently administering Antagonist A or another pharmaceutically acceptable salt thereof and a first anti-C5 agent prior to administering a second anti-C5 agent.

In some embodiments, the present methods comprise administering Antagonist A or another pharmaceutically acceptable salt thereof prior to concurrently administering a first anti-C5 agent and a second anti-C5 agent.

In some embodiments, the present methods comprise concurrently administering Antagonist A or another pharmaceutically acceptable salt thereof, a first anti-C5 agent and a second anti-C5 agent.

In some embodiments, the present methods comprise administering an anti-C5 agent prior to administering two or more PDGF antagonists. In some embodiments, the present methods comprise administering an anti-C5 agent prior to administering a first PDGF antagonist and administering the first PDGF antagonist prior to administering a second PDGF antagonist.

In some embodiments, the present methods comprise concurrently administering an anti-C5 agent and Antagonist A or another pharmaceutically acceptable salt thereof. In some embodiments, the present methods comprise concurrently administering an anti-C5 agent and a first PDGF antagonist prior to administering a second PDGF antagonist.

In some embodiments, the present methods comprise administering an anti-C5 agent prior to concurrently administering a first PDGF antagonist and a second PDGF antagonist.

In some embodiments, the present methods comprise concurrently administering an anti-C5 agent, a first PDGF antagonist and a second PDGF antagonist.

In some embodiments, the present methods comprise administering a VEGF antagonist prior to administering two or more anti-C5 agents. In some embodiments, the present methods comprise administering a VEGF antagonist prior to administering a first anti-C5 agent and administering the first anti-C5 agent prior to administering a second anti-C5 agent.

In some embodiments, the present methods comprise concurrently administering a VEGF antagonist and an anti-C5 agent. In some embodiments, the present methods comprise concurrently administering a VEGF antagonist and a first anti-C5 agent prior to administering a second anti-C5 agent.

In some embodiments, the present methods comprise administering a VEGF antagonist prior to concurrently administering a first anti-C5 agent and a second anti-C5 agent.

In some embodiments, the present methods comprise concurrently administering a VEGF antagonist, a first anti-C5 agent and a second anti-C5 agent.

In some embodiments, the present methods comprise administering an anti-C5 agent prior to administering two or more VEGF antagonists. In some embodiments, the present methods comprise administering an anti-C5 agent prior to administering a first VEGF antagonist and administering the first VEGF antagonist prior to administering a second VEGF antagonist.

In some embodiments, the present methods comprise concurrently administering an anti-C5 agent and a VEGF antagonist. In some embodiments, the present methods comprise concurrently administering an anti-C5 agent and a first VEGF antagonist prior to administering a second VEGF antagonist.

In some embodiments, the present methods comprise administering an anti-C5 agent prior to concurrently administering a first VEGF antagonist and a second VEGF antagonist.

In some embodiments, the present methods comprise concurrently administering an anti-C5 agent, a first VEGF antagonist and a second VEGF antagonist.

In some embodiments, the first agent and second agent are PDGF antagonists, which can be the same or different. In some embodiment, the first agent and second agent are VEGF antagonists, which can be the same or different. In some embodiments, the first agent and second agent are anti-C5 agents, which can be the same or different.

In some embodiments, the first agent and third agent are PDGF antagonists, which can be the same or different. In some embodiment, the first agent and third agent are VEGF antagonists, which can be the same or different. In some embodiments, the first agent and third agent are anti-C5 agents, which can be the same or different.

In some embodiments, the second agent and third agent are PDGF antagonists, which can be the same or different. In some embodiment, the second agent and third agent are VEGF antagonists, which can be the same or different. In some embodiments, the second agent and third agent are anti-C5 agents, which can be the same or different.

Illustrative groups of first agent, second agent and third agent are set forth below in Tables 7, 8, 9 and 10.

**Table 7**

| **Group** | **First Agent** | **Second Agent** | **Third Agent** |
|---|---|---|---|
| A | PDGF Antagonist | VEGF antagonist | VEGF antagonist |
| B | VEGF antagonist | PDGF Antagonist | VEGF antagonist |
| C | VEGF antagonist | VEGF antagonist | PDGF Antagonist |
| D | PDGF Antagonist | Anti-C5 Agent | Anti-C5 Agent |
| E | Anti-C5 Agent | PDGF Antagonist | Anti-C5 Agent |
| F | Anti-C5 Agent | Anti-C5 Agent | PDGF Antagonist |
| G | PDGF Antagonist | PDGF Antagonist | VEGF antagonist |
| H | PDGF Antagonist | VEGF antagonist | PDGF Antagonist |
| I | VEGF antagonist | PDGF Antagonist | PDGF Antagonist |
| J | PDGF Antagonist | PDGF Antagonist | Anti-C5 Agent |
| K | PDGF Antagonist | Anti-C5 Agent | PDGF Antagonist |
| L | Anti-C5 Agent | PDGF Antagonist | PDGF Antagonist |

**Table 8**

| **Group** | **First Agent** | **Second Agent** | **Third Agent** |
|---|---|---|---|
| A | PDGF Antagonist | First VEGF antagonist | Second VEGF antagonist |
| B | First VEGF antagonist | PDGF Antagonist | Second VEGF antagonist |
| C | First VEGF antagonist | Second VEGF antagonist | PDGF Antagonist |
| D | PDGF Antagonist | First Anti-C5 Agent | Second Anti-C5 Agent |
| E | First Anti-C5 Agent | PDGF Antagonist | Second Anti-C5 Agent |
| F | First Anti-C5 Agent | Second Anti-C5 Agent | PDGF Antagonist |
| G | First PDGF Antagonist | Second PDGF Antagonist | VEGF antagonist |
| H | First PDGF Antagonist | VEGF antagonist | Second PDGF Antagonist |
| I | VEGF antagonist | First PDGF Antagonist | Second PDGF Antagonist |
| J | First PDGF Antagonist | Second PDGF Antagonist | Anti-C5 Agent |
| K | First PDGF Antagonist | Anti-C5 Agent | Second PDGF Antagonist |
| L | Anti-C5 Agent | First PDGF Antagonist | Second PDGF Antagonist |

**Table 9**

| **Group** | **First Agent** | **Second Agent** | **Third Agent** |
|---|---|---|---|
| A | Antagonist A | ranibizumab | Antagonist A |
| B | Antagonist A | ranibizumab | ranibizumab |
| C | Antagonist A | bevacizumab | Antagonist A |
| D | Antagonist A | bevacizumab | bevacizumab |
| E | Antagonist A | aflibercept | Antagonist A |
| F | Antagonist A | aflibercept | aflibercept |
| G | Antagonist A | pegaptanib sodium | Antagonist A |
| H | Antagonist A | pegaptanib sodium | pegaptanib sodium |
| I | Antagonist A | ESBA1008 | Antagonist A |
| J | Antagonist A | ESBA1008 | ESBA1008 |
| K | Antagonist A | ARC1905 | Antagonist A |
| L | Antagonist A | ARC1905 | ARC1905 |
| M | ranibizumab | Antagonist A | ranibizumab |
| N | ranibizumab | Antagonist A | Antagonist A |
| O | bevacizumab | Antagonist A | bevacizumab |
| P | bevacizumab | Antagonist A | Antagonist A |
| Q | aflibercept | Antagonist A | aflibercept |
| R | aflibercept | Antagonist A | Antagonist A |
| S | pegaptanib sodium | Antagonist A | pegaptanib sodium |
| T | pegaptanib sodium | Antagonist A | Antagonist A |
| U | ESBA1008 | Antagonist A | ESBA1008 |
| V | ESBA1008 | Antagonist A | Antagonist A |
| W | ARC1905 | Antagonist A | ARC 1905 |
| X | ARC1905 | Antagonist A | Antagonist A |
| Y | ranibizumab | ranibizumab | Antagonist A |
| Z | bevacizumab | bevacizumab | Antagonist A |
| AA | aflibercept | aflibercept | Antagonist A |
| AB | pegaptanib sodium | pegaptanib sodium | Antagonist A |
| AC | ESBA1008 | ESBA 1008 | Antagonist A |
| AD | ARC1905 | ARC1905 | Antagonist A |
| AE | ranibizumab | ranibizumab | bevacizumab |
| AF | ranibizumab | bevacizumab | ranibizumab |
| AG | ranibizumab | ranibizumab | aflibercept |
| AH | ranibizumab | aflibercept | ranibizumab |
| AI | ranibizumab | ranibizumab | pegaptanib sodium |
| AJ | ranibizumab | pegaptanib sodium | ranibizumab |
| AK | ranibizumab | ranibizumab | ESBA1008 |
| AL | ranibizumab | ESBA1008 | ranibizumab |
| AM | ranibizumab | ranibizumab | ARC1905 |
| AN | ranibizumab | ARC1905 | ranibizumab |
| AO | bevacizumab | bevacizumab | ranibizumab |
| AP | bevacizumab | ranibizumab | bevacizumab |
| AQ | bevacizumab | bevacizumab | aflibercept |
| AR | bevacizumab | aflibercept | bevacizumab |
| AS | bevacizumab | bevacizumab | pegaptanib sodium |
| AT | bevacizumab | pegaptanib sodium | bevacizumab |
| AU | bevacizumab | bevacizumab | ESBA1008 |
| AV | bevacizumab | ESBA1008 | bevacizumab |
| AW | bevacizumab | bevacizumab | ARC1905 |
| AX | bevacizumab | ARC1905 | bevacizumab |
| AY | aflibercept | aflibercept | ranibizumab |
| AZ | aflibercept | ranibizumab | aflibercept |
| BA | aflibercept | aflibercept | bevacizumab |
| BB | aflibercept | bevacizumab | aflibercept |
| BC | aflibercept | aflibercept | pegaptanib sodium |
| BD | aflibercept | pegaptanib sodium | aflibercept |
| BE | aflibercept | aflibercept | ESBA1008 |
| BF | aflibercept | ESBA 1008 | aflibercept |
| BG | aflibercept | aflibercept | ARC 1905 |
| BH | aflibercept | ARC1905 | aflibercept |
| BI | pegaptanib sodium | pegaptanib sodium | ranibizumab |
| BJ | pegaptanib sodium | ranibizumab | pegaptanib sodium |
| BK | pegaptanib sodium | pegaptanib sodium | bevacizumab |
| BL | pegaptanib sodium | bevacizumab | pegaptanib sodium |
| BM | pegaptanib sodium | pegaptanib sodium | aflibercept |
| BN | pegaptanib sodium | aflibercept | pegaptanib sodium |
| BO | pegaptanib sodium | pegaptanib sodium | ESBA1008 |
| BP | pegaptanib sodium | ESBA1008 | pegaptanib sodium |
| BQ | pegaptanib sodium | pegaptanib sodium | ARC1905 |
| BR | pegaptanib sodium | ARC 1905 | pegaptanib sodium |
| BS | ESBA1008 | ESBA1008 | ranibizumab |
| BT | ESBA1008 | ranibizumab | ESBA1008 |
| BU | ESBA1008 | ESBA1008 | bevacizumab |
| BV | ESBA1008 | bevacizumab | ESBA1008 |
| BW | ESBA 1008 | ESBA1008 | aflibercept |
| BX | ESBA 1008 | aflibercept | ESBA1008 |
| BY | ESBA1008 | ESBA1008 | pegaptanib sodium |
| BZ | ESBA1008 | pegaptanib sodium | ESBA1008 |
| CA | ESBA1008 | ESBA 1008 | ARC 1905 |
| CB | ESBA1008 | ARC1905 | ESBA1008 |
| CC | ARC1905 | ARC1905 | ranibizumab |
| CD | ARC1905 | ranibizumab | ARC1905 |
| CE | ARC1905 | ARC1905 | bevacizumab |
| CF | ARC 1905 | bevacizumab | ARC 1905 |
| CO | ARC 1905 | ARC 1905 | aflibercept |
| CH | ARC1905 | aflibercept | ARC1905 |
| CI | ARC1905 | ARC1905 | pegaptanib sodium |
| CJ | ARC1905 | pegaptanib sodium | ARC1905 |
| CK | ARC1905 | ARC1905 | ESBA 1008 |
| CL | ARC1905 | ESBA1008 | ESBA1008 |

**Table 10**

| **Group** | **First Agent** | **Second Agent** | **Third Agent** |
|---|---|---|---|
| A | Antagonist A | ranibizumab | bevacizumab |
| B | Antagonist A | ranibizumab | aflibercept |
| C | Antagonist A | ranibizumab | pegaptanib sodium |
| D | Antagonist A | bevacizumab | aflibercept |
| E | Antagonist A | bevacizumab | pegaptanib sodium |
| F | Antagonist A | aflibercept | pegaptanib sodium |
| G | ranibizumab | bevacizumab | Antagonist A |
| H | ranibizumab | aflibercept | Antagonist A |
| I | ranibizumab | pegaptanib sodium | Antagonist A |
| J | bevacizumab | aflibercept | Antagonist A |
| K | bevacizumab | pegaptanib sodium | Antagonist A |
| L | aflibercept | pegaptanib sodium | Antagonist A |
| M | ranibizumab | Antagonist A | bevacizumab |
| N | ranibizumab | Antagonist A | aflibercept |
| O | ranibizumab | Antagonist A | pegaptanib sodium |
| P | bevacizumab | Antagonist A | aflibercept |
| Q | bevacizumab | Antagonist A | pegaptanib sodium |
| R | aflibercept | Antagonist A | pegaptanib sodium |
| S | bevacizumab | ranibizumab | Antagonist A |
| T | aflibercept | ranibizumab | Antagonist A |
| U | pegaptanib sodium | ranibizumab | Antagonist A |
| V | aflibercept | bevacizumab | Antagonist A |
| W | pegaptanib sodium | bevacizumab | Antagonist A |
| X | pegaptanib sodium | aflibercept | Antagonist A |
| Y | bevacizumab | Antagonist A | ranibizumab |
| Z | aflibercept | Antagonist A | ranibizumab |
| AA | pegaptanib sodium | Antagonist A | ranibizumab |
| AB | aflibercept | Antagonist A | bevacizumab |
| AC | pegaptanib sodium | Antagonist A | bevacizumab |
| AD | pegaptanib sodium | Antagonist A | aflibercept |
| AE | Antagonist A | ARC187 | ARC1905 |
| AF | Antagonist A | ARC 1905 | ARC187 |
| AG | ARC187 | ARC1905 | Antagonist A |
| AH | ARC1905 | ARC187 | Antagonist A |
| AI | ARC187 | Antagonist A | ARC 1905 |
| AJ | ARC1905 | Antagonist A | ARC187 |

In one embodiment, two or more agents are administered concurrently. In one embodiment, the two or more agents administered concurrently are present in the same composition. In another embodiment, the two or more agents administered concurrently are each present in a separate composition.

In certain embodiments, the time period from administration of a first agent to administration of a second agent is at least 1 min, at least 5 min, at least 10 min, at least 15 min, at least 30 min, or at least one hour. In certain embodiments, the time period from administration of a first agent to administration of a second agent is between 1 min and 2 hours, between 5 min and 2 hours, between 10 min and 2 hours, between 15 min and 2 hours, between 30 min and 2 hours, between 45 min and 2 hours, between 1 hour and 2 hours, or between 30 min and 1 hour. In certain embodiments, the time period from administration of a first agent to administration of a second agent is about 1 min, about 2 min, about 3 min, about 5 min, about 10 min, about 15 min, about 20 min, about 25 min, about 30 min, about 35 min, about 40 min, about 45 min, about 50 min, about 55 min, about 60 min, about 90 min, or about 120 min. In certain embodiments, a second agent is administered within 90 days, 30 days, 10 days, 5 days, 2 days, 1 day, 24 hours, 1 hour, 30 minutes, 10 minutes, 5 minutes or one minute after administration of a second agent.

In certain embodiments, the time period from administration of a second agent to administration of a third agent is at least 1 min, at least 5 min, at least 10 min, at least 15 min, at least 30 min, or at least one hour. In certain embodiments, the time period between administration of a second agent and administration of a third agent is between 1 min and 2 hours, between 5 min and 2 hours, between 10 min and 2 hours, between 15 min and 2 hours, between 30 min and 2 hours, between 45 min and 2 hours, between 1 hour and 2 hours, or between 30 min and 1 hour. In certain embodiments, the time period between administration of a second agent and administration of a third agent is about 1 min, about 2 min, about 3 min, about 5 min, about 10 min, about 15 min, about 20 min, about 25 min, about 30 min, about 35 min, about 40 min, about 45 min, about 50 min, about 55 min, about 60 min, about 90 min, or about 120 min. In certain embodiments, a third agent is administered within 90 days, 30 days, 10 days, 5 days, 2 days, 1 day, 24 hours, 1 hour, 30 minutes, 10 minutes, 5 minutes or one minute after administration of a second agent.

In certain embodiments, the time period between concurrent administration of a first agent and a second agent and administration of a third agent is at least 1 min, at least 5 min, at least 10 min, at least 15 min, at least 30 min, or at least one hour. In certain embodiments, the time period between concurrent administration of a first agent and a second agent and administration of a third agent is between 1 min and 2 hours, between 5 min and 2 hours, between 10 min and 2 hours, between 15 min and 2 hours, between 30 min and 2 hours, between 45 min and 2 hours, between 1 hour and 2 hours, or between 30 min and 1 hour. In certain embodiments, the time period from concurrent administration of a first agent and a second agent to administration of a third agent is about 1 min, about 2 min, about 3 min, about 5 min, about 10 min, about 15 min, about 20 min, about 25 min, about 30 min, about 35 min, about 40 min, about 45 min, about 50 min, about 55 min, about 60 min, about 90 min, or about 120 min. In certain embodiments, adminstration of a third agent is within 90 days, 30 days, 10 days, 5 days, 2 days, 1 day, 24 hours, 1 hour, 30 minutes, 10 minutes, 5 minutes or one minute of concurrent administration of a first agent and a second agent.

In certain embodiments, the time period from administration of a first agent to concurrent administration a second agent and a third agent is at least 1 min, at least 5 min, at least 10 min, at least 15 min, at least 30 min, or at least one hour. In certain embodiments, the time period from administration of a first agent to concurrent administration of a second agent and a third agent is between 1 min and 2 hours, between 5 min and 2 hours, between 10 min and 2 hours, between 15 min and 2 hours, between 30 min and 2 hours, between 45 min and 2 hours, between 1 hour and 2 hours, or between 30 min and 1 hour. In certain embodiments, the time period from administration of a first agent to concurrent administration of a second agent and a third agent is about 1 min, about 2 min, about 3 min, about 5 min, about 10 min, about 15 min, about 20 min, about 25 min, about 30 min, about 35 min, about 40 min, about 45 min, about 50 min, about 55 min, about 60 min, about 90 min, or about 120 min. In certain embodiments, concurrent administration of a second agent and a third agent is within 90 days, 30 days, 10 days, 5 days, 2 days, 1 day, 24 hours, 1 hour, 30 minutes, 10 minutes, 5 minutes or one minute of administration of a first agent.

The administration of two or more, such as three or more, active agents (e.g., Antagonist A or another pharmaceutically acceptable salt thereof, a VEGF antagonist and an anti-C5 agent) can have a synergistic effect in treating or preventing a disease or disorder, e.g., an ophthalmological disease or disorder. For example, administration of Antagonist A or another pharmaceutically acceptable salt thereof, VEGF antagonist and anti-C5 agent (or any two of these active agents) can improve retinal attachment success, improve visual acuity, reduce choroidal neovascularization or stabilize vision to a degree that is greater than an additive effect of the active agents.

In certain embodiments, the invention provides methods for treating or preventing an ophthalmological disease or disorder, comprising administering to a subject in need thereof one or more, in some embodiments two or more or three or more, active agents via an apparatus. In other embodiments, the methods further comprise performing surgery on the subject. In other embodiments, the methods further comprise administering another active agent, such as an antineoplastic drug, including but not limited to any of those described herein. In particular embodiments, the methods further comprise administering another active agent and performing surgery on the subject.

In some embodiments, administration of Antagonist A or another pharmaceutically acceptable salt thereof, and optionally a VEGF antagonist and/or an anti-C5 agent to a subject results in improved vision, such as increased visual acuity. In some embodiments, the subject experienced moderate vision loss, defined as losing 15 letters or more from baseline on ETDRS visual acuity testing, measured at week 24, prior to treatment with Antagonist A or another pharmaceutically acceptable salt thereof.

In some embodiments, visual acuity testing is as described in Early Treatment Diabetic Retinopathy Study Research Group (ETDRS), Manual of Operations, Baltimore: ETDRS Coordinating Center, University of Maryland. Available from: National Technical Information Service, 5285 Port Royal Road, Springfield, VA 22161; Accession No. PB85 223006/AS; Ferris et al., Am J Ophthalmol 94:91-96, 1982; or or Example 4, as described herein. In some embodiments, the visual acuity testing uses one or more charts available from http://www.nei.nih.gov/photo/keyword.asp?conditions=Eye+Charts&match=all, e.g., ETDRS visual acuity Chart 1, 2 and/or R.

In other embodiments, administration of Antagonist A or another pharmaceutically acceptable salt thereof and a VEGF antagonist results in fewer ocular adverse events, a decrease in size of RCH (e.g., measured by fundus photography and FA), a decrease in exudation (measured by fundus photography, OCT, and FA), or a decrease in epiretinal proliferation or retinal traction (assessed by fundus photography), compared to those experienced by a subject who was not administered with Antagonist A or another pharmaceutically acceptable salt thereof. In some embodiments, the subject does not require, and the methods do not comprise, ablative treatment of RCH or ocular surgery.

In some embodiments, administration of Antagonist A or another pharmaceutically acceptable salt thereof, and optionally a VEGF antagonist and/or an anti-C5 agent, to a subject results in improved vision independent of baseline lesion size or baseline vision, compared to vision of a subject who was not administered with Antagonist A or another pharmaceutically acceptable salt thereof, or compared to a subject administered anti-VEGF monotherapy. In some embodiments, administration of Antagonist A or another pharmaceutically acceptable salt thereof, and optionally a VEGF antagonist and/or an anti-C5 agent, to a subject results in the subject having a visual acuity of 20/40 or better, or 20/25 or better vision. In some embodiments, administration of Antagonist A or another pharmaceutically acceptable salt thereof, and optionally a VEGF antagonist and/or an anti-C5 agent to a subject results in an increased reduction in CNV size in the subject, compared to CNV size in a patient who was not administered with Antagonist A or another pharmaceutically acceptable salt thereof, or compared to a subject administered anti-VEGF monotherapy. In some embodiments, administration of Antagonist A or another pharmaceutically acceptable salt thereof, and optionally a VEGF antagonist and/or an anti-C5 agent, to a subject results in a reduction in CNV size (e.g., reduction in disc area (DA) size). In some embodiments, administration of Antagonist A or another pharmaceutically acceptable salt thereof, and optionally a VEGF antagonist and/or an anti-C5 agent to a subject result in an increased reduction in DA in the subject, compared to DA in a patient who was not administered with Antagonist A or another pharmaceutically acceptable salt thereof , or compared to a subject administered anti-VEGF monotherapy. In some embodiments, the increased reduction in CNV size is in subjects with small baseline CNV, e.g., less than or equal to 1.62 DA (disc area). In some embodiments, the increased reduction in CNV size (e.g., in disc area) is in subjects with large baseline CNV, e.g., greater than 1.62 DA. In some embodiments, administration of Antagonist A or another pharmaceutically acceptable salt thereof, and optionally a VEGF antagonist and/or an anti-C5 agent, to a subject results in neovascular regression. In some embodiments, administration of Antagonist A or another pharmaceutically acceptable salt thereof, and optionally a VEGF antagonist and/or an anti-C5 agent, to a subject results in reduced neovascular growth, compared to that occurring in a subject who was not administered with Antagonist A or another pharmaceutically acceptable salt thereof, or compared to a subject administered anti-VEGF monotherapy. In some embodiments, the reduced neovascular growth is anti-fibrosis. In some embodiments, administration of Antagonist A or another pharmaceutically acceptable salt thereof, and optionally a VEGF antagonist and/or an anti-C5 agent, to a subject results in a decrease in or absence of hyper-reflective material, e.g., sub-retinal hyper-reflective material, such as a decrease in the size of sub-retinal hyper-reflective material (SHRM) as evidenced by spectral domain optical coherence tomography (SD-OCT). In some embodiments, administration of Antagonist A or another pharmaceutically acceptable salt thereof, and optionally a VEGF antagonist and/or an anti-C5 agent, to a subject results in an increase in resolution of hyper-reflective material, e.g., sub-retinal hyper-reflective material, such as compared to a subject who was not administered with Antagonist A or another pharmaceutically acceptable salt thereof, or compared to a subject administered a VEGF antagonist, anti-VEGF monotherapy, and/or an anti-C5 agent.

In some embodiments, a subject with improved vision has a greater than 3-line, 4-line or 5-line gain in visual acuity. In one embodiment, a subject's visual acuity is determined using a protocol such as the Early Treatment for Diabetic Retinopathy Study ("ETDRS") or the Age-Related Eye Disease Study ("AREDS") protocol. In some embodiments, visual acuity is measured using a modified ETDRS and/or AREDS protocol, such as the measurement of visual acuity described in Ferris et al., Am J Ophthalmol 94:91-96, 1982. In some embodiments, visual acuity is measured as described in Early Treatment Diabetic Retinopathy Study Research Group (ETDRS), Manual of Operations, Baltimore: ETDRS Coordinating Center, University of Maryland. Available from: National Technical Information Service, 5285 Port Royal Road, Springfield, VA 22161; Accession No. PB85 223006/AS. In other embodiments, visual acuity testing is measured as described in Example 4 below. In some embodiments, the visual acuity testing uses one or more charts available from http://www.nei.nih.gov/photo/keyword.asp?conditions=Eye+Charts&match=all, e.g., ETDRS visual acuity Chart 1, 2 and/or R.

In one embodiment, a subject's visual acuity is determined by one or more of the following procedures: (1) measurement of best-corrected visual acuity (BCVA) with required manifest refraction; (2) measurement of corrected visual acuity with conditional manifest refraction; or (3) measurement of corrected visual acuity without manifest refraction.

In one embodiment, each of the PDGF and VEGF antagonists is administered in an amount effective to treat or prevent an ophthalmological disease or disorder. The amount of antagonist that is admixed with the carrier materials to produce a single dosage can vary depending upon the subject being treated and the particular mode of administration.

The dosage of each antagonist can depend on several factors including the severity of the condition, whether the condition is to be treated or prevented, and the age, weight, and health of the person to be treated. Additionally, pharmacogenomic (the effect of genotype on the pharmacokinetic, pharmacodynamic or efficacy profile of a therapeutic) information about a particular patient may affect dosage used. Furthermore, the exact individual dosages can be adjusted somewhat depending on a variety of factors, including the specific combination of antagonists being administered, the time of administration, the route of administration, the nature of the formulation, the rate of excretion, the particular ophthalmological disease or disorder being treated, the severity of the disorder, and the anatomical location of the neovascular disorder. Some variations in the dosage can be expected.

Generally, when orally administered to a subject, the dosage of an antagonist of the present invention is normally 0.001 mg/kg/day to 100 mg/kg/day, 0.01 mg/kg/day to 50 mg/kg/day, or 0.1 mg/kg/day to 10 mg/kg/day. Generally, when orally administered to a human, the dosage of an antagonist of the present invention is normally 0.001 mg to 300 mg per day, 1 mg to 200 mg per day, or 5 mg to 50 mg per day. Dosages up to 200 mg per day may be necessary. For administration of an antagonist of the present invention by parenteral injection, the dosage is normally 0.1 mg to 250 mg per day, 1 mg to 20 mg per day, or 3 mg to 5 mg per day. Injections may be given up to four times daily. In some embodiments, the dosage of a PDGF or VEGF antagonist for use in the present invention is normally 0.1 mg to 1500 mg per day, or 0.5 mg to 10 mg per day, or 0.5 mg to 5 mg per day. A dosage of up to 3000 mg per day can be administered.

In some embodiments, for administration by parenteral injection of a three active agents (e.g., Antagonist A or another pharmaceutically acceptable salt thereof, VEGF antagonist and an anti-C5 agent or other combination disclosed herein), the dosage of each of the PDGF antagonist, VEGF antagonist and anti-C5 agent, is typically 0.1 mg to 250 mg per day, 1 mg to 20 mg per day, or 3 mg to 5 mg per day. Injections may be given up to four times daily. Generally, when parenterally administered, the dosage of Antagonist A or another pharmaceutically acceptable salt thereof, VEGF antagonist, or anti-C5 agent is typically 0.1 mg to 1500 mg per day, or 0.5 mg to 10 mg per day, or 0.5 mg to 5 mg per day. A dosage of at least up to 3000 mg per day can be administered.

In some embodiments, in which Antagonist A or another pharmaceutically acceptable salt thereof, VEGF antagonist and/or anti-C5 agent are ophthalmologically administered to a human, for example intravitreally, the dosage of each of Antagonist A or another pharmaceutically acceptable salt thereof, VEGF antagonist and anti-C5 agent is typically 0.003 mg to 5.0 mg per eye per administration, or 0.03 mg to 3.0 mg per eye per administration, or 0.1 mg to 1.0 mg per eye per administration. In one embodiment, the dosage of each of Antagonist A or another pharmaceutically acceptable salt thereof, VEGF antagonist and anti-C5 agent is about 0.03 mg, about 0.3 mg, about 0.5 mg, about 1.0 mg, about 1.25 mg, about 1.5 mg, about 2.0 mg or about 3.0 mg per eye. In one embodiment, the dosage Antagonist A or another pharmaceutically acceptable salt thereof is about 0.03 mg, about 0.3 mg, about 0.5 mg, about 1.0 mg, about 1.25 mg, about 1.5 mg, about 2.0 mg, about 3.0 mg, or about 4.0 mg per eye. In another embodiment, the dosage of a VEGF antagonist (e.g., ranibizumab, bevacizumab, aflibercept, ESBA1008 or pegaptanib sodium) is about 0.03 mg, about 0.3 mg, about 0.5 mg, about 1.0 mg, about 1.25 mg, about 1.5 mg, about 1.65 mg, about 2.0 mg, about 3.0 mg, or about 4.0 mg per eye. In another embodiment, the dosage of the anti-C5 agent (e.g., ARC1905or a pharmaceutically acceptable salt thereof) is about 0.03 mg, about 0.3 mg, about 0.5 mg, about 1.0 mg, about 1.25 mg, about 1.5 mg, about 1.65 mg, about 2.0 mg, about 3.0 mg, or about 4.0 per eye.

In certain embodiments where a subject is administered both Antagonist A or another pharmaceutically acceptable salt thereof and a VEGF antagonist, and optionally an anti-C5 agent, the dosage of Antagonist A or another pharmaceutically acceptable salt thereof) is about 1.5 mg, and the dosage of the VEGF antagonist (e.g., ranibizumab) is about 0.5 mg. In certain embodiments where a subject is administered both Antagonist A or another pharmaceutically acceptable salt thereof and a VEGF antagonist, the dosage of Antagonist A or another pharmaceutically acceptable salt thereofis about 3.0 mg, and the dosage of the VEGF antagonist (e.g., ranibizumab) is about 0.5 mg. In certain embodiments, a subject is administered both Antagonist A or another pharmaceutically acceptable salt thereof and a VEGF antagonist, wherein the dosage of Antagonist A or another pharmaceutically acceptable salt thereof) is about 1.5 mg, and the dosage of the VEGF antagonist (e.g., bevacizumab) is about 1.25 mg. In certain embodiments, a subject is administered both Antagonist A or another pharmaceutically acceptable salt thereof and a VEGF antagonist, wherein the dosage of Antagonist A or another pharmaceutically acceptable salt thereof is about 3.0 mg, and the dosage of the VEGF antagonist (e.g., bevacizumab) is about 1.25 mg. In certain embodiments, a subject is administered both Antagonist A or another pharmaceutically acceptable salt thereof and a VEGF antagonist, wherein the dosage of Antagonist A or another pharmaceutically acceptable salt thereof is about 1.5 mg, and the dosage of the VEGF antagonist (e.g., aflibercept) is about 2.0 mg. In certain embodiments, a subject is administered both Antagonist A or another pharmaceutically acceptable salt thereof and a VEGF antagonist, wherein the dosage of Antagonist A or another pharmaceutically acceptable salt thereof is about 3.0 mg, and the dosage of the VEGF antagonist (e.g., aflibercept) is about 2.0 mg. In certain embodiments, a subject is administered both Antagonist A or another pharmaceutically acceptable salt thereof and a VEGF antagonist, wherein the dosage of Antagonist A or another pharmaceutically acceptable salt thereof is about 1.5 mg, and the dosage of the VEGF antagonist, e.g., pegaptanib sodium, is about 1.65 mg. In certain embodiments, a subject is administered both Antagonist A or another pharmaceutically acceptable salt thereof and a VEGF antagonist, wherein the dosage of Antagonist A or another pharmaceutically acceptable salt thereof is about 3.0 mg, and the dosage of the VEGF antagonist, e.g., pegaptanib sodium, is about 1.65 mg.

The dosage can range from about 0.01 mL to about 0.2 mL administered per eye, or about 0.03 mL to about 0.15 mL administered per eye, or about 0.05 mL to about 0.10 mL administered per eye.

Antagonist A or a pharmaceutically acceptable salt thereof can be delivered intravitreally at up to about 30 mg/ml with injection volumes up to 100 µL.

Illustrative Antagonist A/VEGF antagonist combination pairs and their dosages are set forth in Table 11:

**Table 11**

| **Combination No.** | **PDGF Antagonist** | **VEGF Antagonist** |
|---|---|---|
| 1 | Antagonist A (about 1.5 mg) | ranibizumab (about 0.5 mg) |
| 2 | Antagonist A (about 3.0 mg) | ranibizumab (about 0.5 mg) |
| 3 | Antagonist A (about 1.5 mg) | bevacizumab (about 1.25 mg) |
| 4 | Antagonist A (about 3.0 mg) | bevacizumab (about 1.25 mg) |
| 5 | Antagonist A (about 1.5 mg) | aflibercept (about 2.0 mg) |
| 6 | Antagonist A (about 3.0 mg) | aflibercept (about 2.0 mg) |
| 7 | Antagonist A (about 3.0 mg) | pegaptanib sodium (about 1.65 mg) |
| 8 | Antagonist A (about 3.0 mg) | pegaptanib sodium (about 1.65 mg) |

In particular embodiments wherein the subject is administered an anti-C5 agent in combination with Antagonist A or another pharmaceutically acceptable salt thereof and the VEGF antagonist, the anti-C5 agent may be administered at a dosage of about 0.03 mg, about 0.3 mg, about 0.5 mg, about 1.0 mg, about 1.25 mg, about 1.5 mg, about 2.0 mg or about 3.0 mg per eye.

In certain embodiments, ocular dosages of compositions comprising anti-C5 aptamers, such as ARC1905 and ARC187, or a pharmaceutically acceptable salt thereof, can range from about 0.01 mg to about 5 mg/eye or from about 0.1 mg to about 3 mg/eye. For instance, ocular dosages of compositions comprising ARC1905, ARC 187, or a pharmaceutically acceptable salt thereof may be about 0.01 mg, about 0.03 mg, about 0.05 mg, about 0.1 mg, about 0.3 mg, about 0.5 mg, about 1 mg, about 1.5 mg, about 2 mg, about 2.5 mg, about 3 mg, about 3.5 mg, about 4 mg, about 4.5 mg, or about 5 mg. Such dosages may be administered ocularly, for example by intravitreal injection, weekly, biweekly, monthly, or quarterly, optionally by a sustained release device or formulation. In some embodiments, the anti-C5 aptamers (e.g., ARC1905, ARC187, or a pharmaceutically acceptable salt thereof) can be administered in multiple injections (e.g., intravitreal injections) over a period of months separated by varying time intervals. In certain such embodiments, initial injections received early in the treatment regimen are separated by a shorter interval than injections received later in the treatment regimen. For instance, one dosage regimen, particularly useful in methods for treating, preventing, or stabilizing AMD (e.g., non-exudative type AMD or geographic atrophy), comprises administering initial injections at the start of treatment (e.g., first two, three, four, or five injections) of anti-C5 aptamer (e.g., ARC1905, ARC187, or a pharmaceutically acceptable salt thereof) on a monthly basis and administering subsequent injections at longer intervals (e.g., every three, four, five, or six months). By way of example, the first three injections of anti-C5 aptamer are administered to a subject every month, whereas the fourth and fifth injections are administered three or four months after the previous injection. Intervals between injections of anti-C5 aptamer may be adjusted based on the subject's response to treatment as measured, for example, by change in geographic atrophy lesion size or improvement or stabilization of visual acuity.

In some embodiments, an anti-C5 aptamer is administered to a subject with a VEGF antagonist, wherein the dosage of the anti-C5 aptamer is about 0.03 mg, and the dosage of the VEGF antagonist, e.g., ranibizumab, is about 0.5 mg. In certain embodiments, a subject is administered both an anti-C5 aptamer and a VEGF antagonist, wherein the dosage of the anti-C5 aptamer is about 1.0 mg, and the dosage of the VEGF antagonist, e.g., ranibizumab, is about 0.5 mg. In certain embodiments, a subject is administered both an anti-C5 aptamer and a VEGF antagonist, wherein the dosage of the anti-C5 aptamer is about 2.0 mg, and the dosage of the VEGF antagonist, e.g., ranibizumab, is about 0.5 mg.

In some embodiments, an anti-C5 aptamer is administered to a subject with a VEGF antagonist, wherein the dosage of the anti-C5 aptamer is about 0.03 mg, and the dosage of the VEGF antagonist, e.g., bevacizumab, is about 1.25 mg. In certain embodiments, a subject is administered both an anti-C5 aptamer and a VEGF antagonist, wherein the dosage of the anti-C5 aptamer is about 1.0 mg, and the dosage of the VEGF antagonist, e.g., bevacizumab, is about 1.25 mg. In certain embodiments, a subject is administered both an anti-C5 aptamer and a VEGF antagonist, wherein the dosage of the anti-C5 aptamer is about 2.0 mg, and the dosage of the VEGF antagonist, e.g., bevacizumab, is about 1.25 mg.

In some embodiments, an anti-C5 aptamer is administered to a subject with a VEGF antagonist, wherein the dosage of the anti-C5 aptamer is about 0.03 mg, and the dosage of the VEGF antagonist, e.g., aflibercept, is about 2.0 mg. In certain embodiments, a subject is administered both an anti-C5 aptamer and a VEGF antagonist, wherein the dosage of the anti-C5 aptamer is about 1.0 mg, and the dosage of the VEGF antagonist, e.g., aflibercept, is about 2.0 mg. In certain embodiments, a subject is administered both an anti-C5 aptamer and a VEGF antagonist, wherein the dosage of the anti-C5 aptamer is about 2.0 mg, and the dosage of the VEGF antagonist, e.g., aflibercept, is about 2.0 mg.

Administration of each antagonist can, independently, be one to four times daily or one to four times per month or one to six times per year or once every two, three, four or five years, Administration can be for the duration of one day or one month, two months, three months, six months, one year, two years, three years, and may even be for the life of the patient. In one embodiment, the administration is performed once a month for three months. Chronic, long-term administration will be indicated in many cases. The dosage may be administered as a single dose or divided into multiple doses. In general, the desired dosage should be administered at set intervals for a prolonged period, usually at least over several weeks or months, although longer periods of administration of several months or years or more may be needed.

In addition to treating pre-existing ophthalmological diseases and disorders, the compositions can be administered prophylactically in order to prevent or slow the onset of these disease and disorders. The term "prevent" encompasses inhibiting or delaying the onset or progression of a disease or disorder. In prophylactic applications, the composition can be administered to a patient susceptible to or otherwise at risk of a particular ophthalmological disease or disorder.

In one embodiment, Antagonist A or another pharmaceutically acceptable salt thereof and the VEGF antagonist are administered to a subject in need of treatment therewith, typically in the form of an injectable pharmaceutical composition. Antagonist A or another pharmaceutically acceptable salt thereof and VEGF antagonist can be administered either in separate compositions or in a pharmaceutical composition comprising both the PDGF antagonist and VEGF antagonist. The administration can be by injection, for example by intraocular injection, or by using a drug delivery device. Parenteral, systemic, or transdermal administration is also within the scope of the invention. The administration of Antagonist A or another pharmaceutically acceptable salt thereof and the VEGF antagonist can be sequential in time or concurrent. When administered sequentially, the administration of each can be by the same or different route. In one embodiment, Antagonist A or another pharmaceutically acceptable salt thereof is administered within 90 days, 30 days, 10 days, 5 days, 24 hours, 1 hour, 30 minutes, 10 minutes, 5 minutes or one minute of administration of a VEGF antagonist. Where Antagonist A or another pharmaceutically acceptable salt thereof is administered prior to the VEGF antagonist, the VEGF antagonist is administered within a time and in an amount such that the total amount of Antagonist A or another pharmaceutically acceptable salt thereof and VEGF antagonist is effective to treat or prevent an ophthalmological disease or disorder. Where the VEGF antagonist is administered prior to Antagonist A or another pharmaceutically acceptable salt thereof, Antagonist A or another pharmaceutically acceptable salt thereof is administered within a time and in an amount such that the total amount of Antagonist A or another pharmaceutically acceptable salt thereof and VEGF antagonist is effective to treat or prevent an ophthalmological disease or disorder.

In one embodiment, Antagonist A or another pharmaceutically acceptable salt thereof or VEGF antagonist (e.g., ranibizumab, bevacizumab, pegaptanib sodium, ESBA1008 or aflibercept) is administered intravitreally with a 30-gauge or 27-gauge needle. In some embodiments, a 0.5 inch needle is used. In one embodiment, Antagonist A or another pharmaceutically acceptable salt thereof is administered intravitreally with a 30-gauge 0.5 inch needle and a VEGF antagonist (e.g., ranibizumab, bevacizumab, pegaptanib sodium, ESBA 1008 or aflibercept) is administered intravitreally with a 27-gauge needle. In some embodiments, 50µL (1.5 mg in 0.05 mL) of Antagonist A or another pharmaceutically acceptable salt thereof is administered intravitreally with a 30-gauge 0.5 inch needle and 50µL (0.5 mg in 0.05 mL) of a VEGF antagonist (e.g., ranibizumab, bevacizumab, pegaptanib sodium or aflibercept) is administered intravitreally with a 27-gauge needle.

In certain embodiments where Antagonist A or another pharmaceutically acceptable salt thereof such as Antagonist A or another pharmaceutically acceptable salt thereof is used in combination with a VEGF antagonist, such as ranibizumab, bevacizumab, ESBA1008, pegaptanib sodium or aflibercept, one of these two agents is first administered to the subject, and then the other agent is administered to the subject. In particular embodiments, the two agents are both administered to the same eye of the subject. In particular embodiments, the two agents are both administered to both eyes of the subject. The two agents may be administered to an eye in either order, i.e., Antagonist A or another pharmaceutically acceptable salt thereof may be administered first, and then the VEGF antagonist administered, or the VEGF antagonist may be administered first, and then Antagonist A or another pharmaceutically acceptable salt thereof administered. The agent administered second may be administered immediately following administration of the agent administered first, or the agent administered second may be administered after a time period following administration of the agent administered first.

In certain embodiments, the time period from administration of the first agent to administration of the second agent is at least 1 min, at least 5 min, at least 10 min, at least 15 min, at least 30 min, or at least one hour. In certain embodiments, the time period from administration of the first agent to administration of the second agent is between 1 min and 2 hours, between 5 min and 2 hours, between 10 min and 2 hours, between 15 min and 2 hours, between 30 min and 2 hours, between 45 min and 2 hours, between 1 hour and 2 hours, or between 30 min and 1 hour. In certain embodiments, the time period from administration of the first agent to administration of the second agent is about 1 min, about 2 min, about 3 min, about 5 min, about 10 min, about 15 min, about 20 min, about 25 min, about 30 min, about 35 min, about 40 min, about 45 min, about 50 min, about 55 min, about 60 min, about 90 min, or about 120 min.

In certain embodiments, the present invention provides methods for treating or preventing any of the ophthalmological diseases described herein, comprising providing to a subject in need thereof Antagonist A or another pharmaceutically acceptable salt thereof at a first time point, and providing to the subject a VEGF antagonist, e.g., aflibercept, bevacizumab, ranibizumab, ESBA1008, or pegaptanib sodium, at a second time point, wherein the amount of time between the first time point and the second time point is about 1 min, about 2 min, about 5 min, about 10 min, about 15 min, about 20 min, about 25 min, about 30 min, about 40 min, about 50 min, about 60 min, about 90 min, about 2 hours, about 4 hours, about 6 hours, about 8 hours, about 12 hours, about 24 hours, about 36 hours, about 48 hours, about three days, about four days, about five days, about six days, or about seven days.

In certain embodiments, Antagonist A or another pharmaceutically acceptable salt thereof and the VEGF antagonist are administered intravitreally. In certain embodiments, about 1.5 mg or 3.0 mg of Antagonist A or another pharmaceutically acceptable salt thereof to an eye, and about 0.5 mg, about 1.25 mg, about 1.65 mg, or about 2.0 mg of the VEGF antagonist is administered to an eye. In some embodiments, the VEGF antagonist is administered intravitreally about 30 minutes after Antagonist A or another pharmaceutically acceptable salt thereof is administered intravitreally. In some embodiments, Antagonist A or another pharmaceutically acceptable salt thereof is administered intravitreally about 30 minutes after the VEGF antagonist is administered intravitreally.

In one embodiment, a VEGF antagonist is administered to at least one eye of the subject, about 1 hour is allowed to elapse following administration of the VEGF antagonist, and then Antagonist A or another pharmaceutically acceptable salt thereof is administered to the same eye. In one embodiment, Antagonist A or another pharmaceutically acceptable salt thereof is administered to at least one eye of the subject, about 1 hour is allowed to lapse following administration of the PDGF antagonist, and then a VEGF antagonist is administered to the same eye.

In certain embodiments, the PDGF antagonist and the VEGF antagonist are administered to each eye in a total combined volume of less than or about 50 µL, less than or about 60 µL, less than or about 70 µL, less than or about 80 µL, less than or about 90 µL, less than or about 100 µL, less than or about 120 µL, less than or about 150 µL, or less than or about 200 µL.

In certain embodiments, Antagonist A or another pharmaceutically acceptable salt thereof, VEGF antagonist and anti-C5 agent are administered intraocularly, e.g., intravitreally. In particular embodiments, Antagonist A or another pharmaceutically acceptable salt thereof, VEGF antagonist and anti-C5 agent are administered to the mammal via a single injection, e.g., a single intraocular or intravitreal injection. In particular embodiments, Antagonist A or another pharmaceutically acceptable salt thereof, VEGF antagonist and anti-C5 agent are administered sequentially. In certain embodiments, two or more of Antagonist A or another pharmaceutically acceptable salt thereof, a VEGF antagonist and an anti-C5 agent are administered at the same time, e.g., in the same composition. In particular embodiments, one of Antagonist A or another pharmaceutically acceptable salt thereof, a VEGF antagonist and an anti-C5 agent is administered, and within about 30 seconds, one or two of others are subsequently administered. In particular embodiments, all three of Antagonist A or another pharmaceutically acceptable salt thereof, a VEGF antagonist and an anti-C5 agent are administered within about 30 seconds or one minute of each other. In other embodiments, one of Antagonist A or another pharmaceutically acceptable salt thereof, a VEGF antagonist and an anti-C5 agent is administered, and one or both of the others are administered about 1 min, about 2 min, about 5 min, about 10 min, about 15 min, about 20 min, about 25 min, about 30 min, about 40 min, about 50 min, about 60 min, about 90 min, about 2 hours, about 4 hours, about 6 hours, about 8 hours, about 12 hours, about 24 hours, about 36 hours, about 48 hours, about three days, about four days, about five days, about six days, or about seven days later. In other embodiments, one or two of Antagonist A or another pharmaceutically acceptable salt thereof, VEGF antagonist and anti-C5 agent are administered, and the other is administered about 1 min, about 2 min, about 5 min, about 10 min, about 15 min, about 20 min, about 25 min, about 30 min, about 40 min, about 50 min, about 60 min, about 90 min, about 2 hours, about 4 hours, about 6 hours, about 8 hours, about 12 hours, about 24 hours, about 36 hours, about 48 hours, about three days, about four days, about five days, about six days, or about seven days later. In certain embodiments, one of the PDGF antagonist, VEGF antagonist and anti-C5 agent is administered; and another is administered about 1 min, about 2 min, about 5 min, about 10 min, about 15 min, about 20 min, about 25 min, about 30 min, about 40 min, about 50 min, about 60 min, about 90 min, about 2 hours, about 4 hours, about 6 hours, about 8 hours, about 12 hours, about 24 hours, about 36 hours, about 48 hours, about three days, about four days, about five days, about six days, or about seven days later; and the remaining one is administered about 1 min, about 2 min, about 5 min, about 10 min, about 15 min, about 20 min, about 25 min, about 30 min, about 40 min, about 50 min, about 60 min, about 90 min, about 2 hours, about 4 hours, about 6 hours, about 8 hours, about 12 hours, about 24 hours, about 36 hours, about 48 hours, about three days, about four days, about five days, about six days, or about seven days later. In certain embodiments wherein two of Antagonist A or another pharmaceutically acceptable salt thereof, VEGF antagonist and anti-C5 agent are present in the same composition, the composition is administered and the PDGF antagonist, VEGF antagonist or anti-C5 agent that is not present in the composition is administered about 1 min, about 2 min, about 5 min, about 10 min, about 15 min, about 20 min, about 25 min, about 30 min, about 40 min, about 50 min, about 60 min, about 90 min, about 2 hours, about 4 hours, about 6 hours, about 8 hours, about 12 hours, about 24 hours, about 36 hours, about 48 hours, about three days, about four days, about five days, about six days, or about seven days later. In other embodiments wherein two of Antagonist A or another pharmaceutically acceptable salt thereof, VEGF antagonist and anti-C5 agent are present in the same composition, Antagonist A or another pharmaceutically acceptable salt thereof , VEGF antagonist or anti-C5 agent that is not present in the composition is administered, and the composition is administered about 1 min, about 2 min, about 5 min, about 10 min, about 15 min, about 20 min, about 25 min, about 30 min, about 40 min, about 50 min, about 60 min, about 90 min, about 2 hours, about 4 hours, about 6 hours, about 8 hours, about 12 hours, about 24 hours, about 36 hours, about 48 hours, about three days, about four days, about five days, about six days, or about seven days later.

**In** certain embodiments, Antagonist A or another phatmaceutically acceptable salt thereof, e.g., Antagonist A or another pharmaceutically acceptable salt thereof, is administered about every 24 hours for two or more, three or more, four or more, five or more, six or more, or seven or more days, and a VEGF antagonist, e.g., aflibercept, bevacizumab, ESBA1008, pegaptanib sodium or ranimizumab, is administered about 48 hours following the first administration of Antagonist A or another pharmaceutically acceptable salt thereof. In certain embodiments, Antagonist A or another pharmaceutically acceptable salt thereof is administered on each of four successive days, i.e., day 1, day 2, day 3 and day 4, and the VEGF antagonist (e.g., bevacizumab, ranicizumab, ESBA1008, pegaptanib sodium or aflibercept) is administered on the third day, i.e., day 3. In particular embodiments, a composition comprising Antagonist A or another pharmaceutically acceptable salt thereof, e.g., Antagonist A or another pharmaceutically acceptable salt thereof, is administered to a subject, and a composition comprising a VEGF antagonist is administered to the subject about forty-eight hours later.

In one embodiment, about 50 mg/kg of Antagonist A or another pharmaceutically acceptable salt thereof (e.g., Antagonist A or another pharmaceutically acceptable salt thereof) is administered, e.g., intraperitoneally, on day 1, day 2, day 3 and day 4, and about 1 mg/kg of a VEGF antagonist (e.g., bevacizumab, ranibizumab, ESBA 1008, pegaptanib sodium, or aflibercept) is administered on day 3. In one embodiment, about 50 mg/kg of Antagonist A or another pharmaceutically acceptable salt thereof (e.g., Antagonist A or another pharmaceutically acceptable salt thereof) is administered on day 1, day 2, day 3 and day 4, and about 5 mg/kg of a VEGF antagonist (e.g., bevacizumab, ranibizumab, ESBA 1008, pegaptanib sodium, or aflibercept) is administered on day 3.

In one embodiment, about 50 mg/kg of Antagonist A or another pharmaceutically acceptable salt thereof is administered on day 1, day 2, day 3 and day 4, and about 1 mg/kg of aflibercept is administered on day 3. In one embodiment, about 50 mg/kg of Antagonist A or another pharmaceutically acceptable salt thereof is administered on day 1, day 2, day 3 and day 4, and about 5 mg/kg of aflibercept is administered on day 3.

In one embodiment, about 0.03 mg, about 0.3 mg, about 0.5 mg, about 1.0 mg, about 1.5 mg or about 3.0 mg of Antagonist A or another pharmaceutically acceptable salt thereof (e.g., Antagonist A or another pharmaceutically acceptable salt thereof) is administered intravitreally on day 1, day 2, day 3 and day 4, and about 0.5 mg, about 1.0 mg, about 1.5 mg, about 1.65 mg, about 3.0 mg, or about 4.0 mg of a VEGF antagonist (e.g., bevacizumab, ranibizumab, ESBA1008, pegaptanib sodium, or aflibercept) is administered intravitreally on day 3. In one embodiment, about 0.3 mg or about 1.5 mg of Antagonist A or another pharmaceutically acceptable salt thereof is administered intravitreally on day 1, day 2, day 3 and day 4, and about 0.5 mg of ranibizumab is administered intravitreally on day 3. In one embodiment, about 0.3 mg or about 1.5 mg of Antagonist A or another pharmaceutically acceptable salt thereof is administered intravitreally on day 1, day 2, day 3 and day 4, and about 1.25 mg of bevacizumab is administered intravitreally on day 3. In one embodiment, about 0.3 mg or about 1.5 mg of Antagonist A or another pharmaceutically acceptable salt thereof is administered intravitreally on day 1, day 2, day 3 and day 4, and about 2.0 mg of aflibercept is administered intravitreally on day 3. In one embodiment, about 0.3 mg or about 1.5 mg of Antagonist A or another pharmaceutically acceptable salt thereof is administered intravitreally on day 1, day 2, day 3 and day 4, and about 1.65 mg of pegaptanib sodium is administered intravitreally on day 3.

In some embodiments, Antagonist A or another pharmaceutically acceptable salt thereof and VEGF antagonist are administered every four weeks or every 30 days, for six treatments. In some embodiments, the VEGF antagonist is ranibizumab. In some embodiments, 0.3 mg of Antagonist A or another pharmaceutically acceptable salt thereof and 0.5 mg of ranibizumab are administered every four weeks or every 30 days, for six treatments. In some embodiments, 1.5 mg of Antagonist A or another pharmaceutically acceptable salt thereof and 0.5 mg of ranibizumab are administered every four weeks or every 30 days, for six treatments.

In some embodiments, 0.3 mg of Antagonist A or another pharmaceutically acceptable salt thereof and 1.25 mg of bevacizumab, 2.0 mg of aflibercept, or 1.65 mg of pegaptanib sodium are administered every four weeks or every 30 days, for six treatments. In some embodiments, 1.5 mg of Antagonist A or another pharmaceutically acceptable salt thereof and 1.25 mg of bevacizumab, 2.0 mg of aflibercept, or 1.65 mg of pegaptanib sodium are administered every four weeks or every 30 days, for six treatments.

In some embodiments, the methods comprise administering Antagonist A or another pharmaceutically acceptable salt thereof, bevacizumab and aflibercept. In some embodiments, the methods comprise administering Antagonist A or another pharmaceutically acceptable salt thereof, bevacizumab and aflibercept every four weeks or every 30 days, for six treatments. In some embodiments, the methods comprise administering 1.5 mg of Antagonist A or another pharmaceutically acceptable salt thereof, 1.25 mg of bevacizumab, and 2 mg of aflibercept. In some embodiments, the methods comprise administering 1.5 mg of Antagonist A or another pharmaceutically acceptable salt thereof, 1.25 mg of bevacizumab, and 2 mg of aflibercept every four weeks or every 30 days, for six treatments.

In some embodiments, the methods comprise administering to a subject in need thereof (a) Antagonist A or another pharmaceutically acceptable salt thereof and (b) an VEGF antagonist, wherein (a) and (b) are administered in an amount that is effective for treating or preventing an ocular condition (e.g., wet AMD), and wherein the administering occurs once every month, ± about seven days, for 12 consecutive months.

In some embodiments, the methods comprise administering to a subject in need thereof (a) Antagonist A or another pharmaceutically acceptable salt thereof and (b) an VEGF antagonist, wherein: (a) and (b) are administered in an amount that is effective for treating or preventing an ocular condition (e.g., wet AMD); and the administering occurs once every month, ± about seven days, for a first 12 consecutive months, and immediately thereafter once every two months, ± about seven days, for a second 12 consecutive months, commencing on the second month of the second 12 consecutive months.

In some embodiments, the methods comprise administering to a subject in need thereof (a) Antagonist A or another pharmaceutically acceptable salt thereof and (b) an VEGF antagonist, wherein: (a) and (b) are administered in an amount that is effective for treating or preventing an ocular condition (e.g., wet AMD); and the administering occurs once every month, ± about seven days, for 24 consecutive months is also provided herein.

In some embodiments, the methods comprise administering to a subject in need thereof (a) Antagonist A or another pharmaceutically acceptable salt thereof and (b) an VEGF antagonist, wherein: (a) and (b) are administered in an amount that is effective for treating or preventing an ocular condition (e.g., wet AMD); and the administering occurs once every month, ± about seven days, for three consecutive months, and immediately thereafter once every two months, ± about seven days, for 12 consecutive months, commencing on the second month of the 12 consecutive months.

In some embodiments, the methods comprise continuous treatment, continuous and discontinuous treatments, and/or retreatments, e.g., for the treatment or preventing of wet-type AMD or subfoveal neovascular AMD. In some embodiments, continuous treatment comprises administering to Antagonist A or another pharmaceutically acceptable salt thereof and an anti-VEGF agent monthly (± 7 days) for at least 1, 2, 3, 4, 5, 6, 7, 8, 9, 10, 11, or 12 consecutive months. In some embodiments, Antagonist A or a pharmaceutically acceptable salt thereof is administered within about 1 min, about 2 min, about 5 min, about 10 min, about 15 min, about 20 min, about 25 min, about 30 min, about 40 min, about 50 min, about 60 min, about 90 min, about 2 hours, about 4 hours, about 6 hours, about 8 hours, about 12 hours of administration of the VEGF antagonist. In some embodiments, the VEGF antagonist is administered prior to administration of Antagonist A or a pharmaceutically acceptable salt thereof. In other embodiments, Antagonist A or a pharmaceutically acceptable salt thereof is administered prior to administration of the VEGF antagonist. In some embodiments, Antagonist A or a pharmaceutically acceptable salt thereof and a VEGF antagonist are administered as a co-formulation. In some embodiments, the amount of Antagonist A or a pharmaceutically acceptable salt thereof administered is about 1.5 mg/eye and the amount of VEGF antagonist administered is about 0.5 mg/eye (e.g., ranibizumab), about 1.25 mg/eye (e.g., bevacizumab), about 1.65 mg/eye (e.g., pegaptanib sodium), or about 2.0 mg/eye (e.g., aflibercept).

In some embodiments, the methods further comprise measuring the subject's visual acuity. In some embodiments, the subject's visual acuity is measured once every month, ± about seven days. In some embodiments, visual acuity is stable when it is stable for three consecutive months. In some embodiments, visual acuity is stable when at each of the last two of the three consecutive months, visual acuity is within 5 ETDRS letters (better or worse) of the subject's visual acuity at the first of the three consecutive months (i.e., the month immediately preceding the first of the two consecutive following months).

In some embodiments, a subject is administered in accordance with the present methods until the subject's visual acuity is stable. In some embodiments, a subject is administered in accordance with the present methods until the subject's visual acuity is stable for three consecutive months. In some embodiments, a subject is administered in accordance with the present methods until the subject's visual acuity at each of the last two of the three consecutive months is ≤ a five-ETDRS-letter difference from the subject's visual acuity of the first of the three consecutive months. In some embodiments, a subject is administered in accordance with the present methods until the subject experiences no new or significant intraretinal or sub-retinal hemorrhage, or no increase of ≥ 50 µm in foveal intraretinal fluid. In some embodiments, a subject is administered in accordance with the present methods until the subject's visual acuity measured at each of the last two of the three consecutive months is ≤ a five-ETDRS-letter difference from the subject's visual acuity of the first of the three consecutive months, and the subject experiences no new or significant intraretinal or sub-retinal hemorrhage, and no increase of ≥ 50 µm in foveal intraretinal fluid.

In some embodiments, discontinuous treatment is administered after continuous treatment, in which discontinuous treatment is based on a physician's discretion, and the subject has stabilized vision as determined by ≤ a five-ETDRS-letter difference in the subject's visual acuity after continuous and discontinuous treatment.

In some embodiments, subjects with a loss of visual acuity of > 5 ETDRS letters from the previous monthly assessment, new and significant intraretinal or sub-retinal hemorrhage, and/or an increase of ≥ 50 µm. in foveal intraretinal fluid are retreated.

In some embodiments, the continuous method comprises administering Antagonist A or another pharmaceutically acceptable salt thereof and a VEGF antagonist in an amount that is effective for treating or preventing wet AMD, wherein the administering occurs once every month, ± about seven days, for 12 consecutive months. In some embodiments, the methods further comprise measuring the subject's visual acuity at one month, ± about seven days, immediately following the 12 consecutive months, wherein the subject's visual acuity measured on the twelfth of the 12 consecutive months and the one month immediately following the 12 consecutive months is ≤ a five-ETDRS-letter difference in the subject's visual acuity measured on the eleventh of the 12 consecutive months.

In some embodiments, the methods further comprise measuring the subject's visual acuity once every month, ± about seven days, on each of an additional 11 consecutive months. In some embodiments, the subject's visual acuity measured on any two consecutive months of the additional 11 consecutive months is ≤ a five-ETDRS-letter difference in the subject's visual acuity measured on a month immediately preceding the two consecutive months.

In some embodiments, the subject's visual acuity measured on the twelfth of the 12 consecutive months and the one month immediately following the 12 consecutive months is not ≤ a five-ETDRS-letter difference in the subject's visual acuity measured on the eleventh of the 12 consecutive months and the subject is retreated. In some embodiments, retreatment comprises administering to the patient on the one month immediately following the 12 consecutive months Antagonist A or another pharmaceutically acceptable salt thereof and a VEGF antagonist in an amount that is effective for treating or preventing wet AMD, measuring the patient's visual acuity on a month, ± about seven days, immediately following the one month immediately following the 12 consecutive months, and administering to the subject on each immediately following month Antagonist A or another pharmaceutically acceptable salt thereof and a VEGF antagonist in an amount that is effective for treating or preventing wet AMD, until the subject's visual acuity on any two consecutive following months is ≤ a five-ETDRS-letter difference in the subject's visual acuity measured on a month immediately preceding the first of the two consecutive following months. In some embodiments, the total number of months does not exceed 24.

In some embodiments, wherein the subject's visual acuity measured on the one month immediately following the 12 consecutive months is not ≤ a five-ETDRS-letter difference in the subject's visual acuity measured on the twelfth of the 12 consecutive months and is not solely attributable to newly diagnosed foveal atrophy or worsening ocular media opacity, the method further comprises administering to the subject on the one month immediately following the 12 consecutive months Antagonist A or another pharmaceutically acceptable salt thereof and a VEGF antagonist in an amount that is effective for treating or preventing wet AMD; and administering to the subject on each immediately following month (a) and (b) in an amount that is effective for treating or preventing wet AMD, until the subject's visual acuity measured on any two consecutive following months is ≤ a five-ETDRS-letter difference in the subject's visual acuity measured on a month immediately preceding the first of the two consecutive following months. In some embodiments, the total number of months does not exceed 24.

In some embodiments, wherein the subject presents intraretinal or sub-retinal hemorrhage or a ≥ 50 µm increase in foveal intraretinal fluid at one month, ± about seven days, immediately following the 12 consecutive months, the method further comprises administering to the subject on the one month immediately following the 12 consecutive months Antagonist A or another pharmaceutically acceptable salt thereof an a VEGF antagonist in an amount that is effective for treating or preventing wet AMD; and administering to the subject on each immediately following month (a) and (b) in an amount that is effective for treating or preventing wet AMD, until the subject's visual acuity measured on any two consecutive following months is ≤ a five-ETDRS-letter difference in the subject's visual acuity measured on a month immediately preceding the first of the two consecutive following months. In some embodiments, the total number of months does not exceed 24.

Also provided herein is a method comprising administering Antagonist A or another pharmaceutically acceptable salt thereof and a VEGF antagonist intravitreally once every month, ± about seven days, for a first 12 consecutive months, and immediately thereafter once every two months, ± about seven days, for a second 12 consecutive months, commencing on the second month of the second 12 consecutive months. In some embodiments, Antagonist A or a pharmaceutically acceptable salt thereof is administered within about 1 min, about 2 min, about 5 min, about 10 min, about 15 min, about 20 min, about 25 min, about 30 min, about 40 min, about 50 min, about 60 min, about 90 min, about 2 hours, about 4 hours, about 6 hours, about 8 hours, about 12 hours of administration of the VEGF antagonist. In some embodiments, the VEGF antagonist is administered prior to administration of Antagonist A or a pharmaceutically acceptable salt thereof. In other embodiments, Antagonist A or a pharmaceutically acceptable salt thereof is administered prior to administration of the VEGF antagonist. In some embodiments, Antagonist A or a pharmaceutically acceptable salt thereof and a VEGF antagonist are administered as a co-formulation. In some embodiments, the amount of Antagonist A or a pharmaceutically acceptable salt thereof administered is about 1.5 mg/eye and the amount of VEGF antagonist administered is about 0.5 mg/eye (e.g., ranibizumab), about 1.25 mg/eye (e.g., bevacizumab), about 1.65 mg/eye (e.g., pegaptanib sodium), or about 2.0 mg/eye (e.g., aflibercept).

In some embodiments, the method further comprises measuring the subject's visual acuity once every month, ± about seven days, during the first 12 consecutive months and second 12 consecutive months. In some embodiments, the subject's visual acuity measured on any one of the first, third, fifth, seven, ninth and eleventh months of the second consecutive 12 months decreased at least five ETDRS letters relative to the patient's visual acuity measured on the month immediately preceding the first, third, fifth, seven, ninth or eleventh month of the second consecutive 12 months.

In some embodiments, the methods further comprises administering to the subject an amount of Antagonist A or a pharmaceutically acceptable salt thereof and a VEGF antagonist effective for treating or preventing wet AMD on the month in which the subject's visual acuity measured the decrease of at least five ETDRS letters relative to the patient's visual acuity measured on the immediately preceding month.

In some embodiments, the method further comprises administering Antagonist A or a pharmaceutically acceptable salt thereof and a VEGF antagonist on any one of the first, third, fifth, seven, ninth and eleventh months of the second consecutive 12 months.

In some embodiments, the decrease in visual acuity is attributed to solely newly diagnosed foveal atrophy or opacified ocular media.

In some embodiments, the subject presents intraretinal or sub-retinal hemorrhage or a ≥ 50 µm increase in foveal intraretinal fluid on any one of the first, third, fifth, seven, ninth and eleventh months of the second consecutive 12 months.

In some embodiments, the method further comprises administering Antagonist A or a pharmaceutically acceptable salt thereof and a VEGF antagonist on month in which the subject presents intraretinal or sub-retinal hemorrhage or a ≥ 50 µm increase in foveal intraretinal fluid.

Also provided herein is a method comprising administering Antagonist A or another pharmaceutically acceptable salt thereof and a VEGF antagonist intravitreally once every month, ± about seven days, for 24 consecutive months. In other embodiments, Antagonist A or another pharmaceutically acceptable salt thereof and a VEGF antagonist are administered intravitreally once a month for three months and then every other month for the next 21 months. In some embodiments, Antagonist A or a pharmaceutically acceptable salt thereof is administered within about 1 min, about 2 min, about 5 min, about 10 min, about 15 min, about 20 min, about 25 min, about 30 min, about 40 min, about 50 min, about 60 min, about 90 min, about 2 hours, about 4 hours, about 6 hours, about 8 hours, about 12 hours of administration of the VEGF antagonist. In some embodiments, the VEGF antagonist is administered prior to administration of Antagonist A or a pharmaceutically acceptable salt thereof. In other embodiments, Antagonist A or a pharmaceutically acceptable salt thereof is administered prior to administration of the VEGF antagonist. In some embodiments, Antagonist A or a pharmaceutically acceptable salt thereof and a VEGF antagonist are administered as a co-formulation. In some embodiments, the amount of Antagonist A or a pharmaceutically acceptable salt thereof administered is about 1.5 mg/eye and the amount of VEGF antagonist administered is about 0.5 mg/eye (e.g., ranibizumab), about 1.25 mg/eye (e.g., bevacizumab), about 1.65 mg/eye (e.g., pegaptanib sodium), or about 2.0 mg/eye (e.g., aflibercept).

In some embodiments, the methods comprise administering to a subject in need thereof (a) Antagonist A or another pharmaceutically acceptable salt thereof and (b) an VEGF antagonist, wherein (a) and (b) are administered in an amount that is effective for treating or preventing an ophthalmological disease or disorder (e.g., wet AMD), and wherein the administering occurs once every month, ± about seven days, for a first administration period of at least 3 consecutive months, followed by administering (a) and (b) for a second administration period at a frequency of at least every other month ± about seven days beginning at two months ± about seven days after the day of the last month of the first administration period on which (a) and (b) are administered. In some embodiments, the first administration period is for at least 6 consecutive months. In some embodiments, the VEGF antagonist is ranibizumab or bevacizumab, wherein (a) and (b) are administered at a frequency of once every month ± about seven days during the second administration period and wherein the second administration period is at least about nine months.

In some embodiments, the methods further comprise measuring the subject's visual acuity on a day that is prior to and within about one month of administration of (a) and (b). In some embodiments, the methods further comprise administering to the subject (a) and (b) in an amount that is effective for treating or preventing an an ophthalmological disease or disorder (e.g., wet AMD), until the subject's visual acuity on any two consecutive following months is ≤ a five-ETDRS-letter difference in the subject's visual acuity measured on a month immediately preceding the first of the two consecutive following months.

In some embodiments, the method further comprise administering to the subject (a) and (b) every other month in an amount that is effective for treating or preventing an an ophthalmological disease or disorder (e.g., wet AMD), until the subject's visual acuity on any two consecutive visual acuity assessments is not ≤ a five-ETDRS-letter difference in the subject's visual acuity measured on a visual acuity assessment immediately preceding the first of the two consecutive visual acuity assessments.

In other embodiments, the methods further comprise administering to the subject (a) and (b) every month in an amount that is effective for treating or preventing an an ophthalmological disease or disorder (e.g., wet AMD), until the subject's visual acuity on any two consecutive following months is ≤ a five-ETDRS-letter difference in the subject's visual acuity measured on a month immediately preceding the first of the two consecutive following months.

In some embodiments, the methods comprise administering to a subject in need thereof (a) Antagonist A or another pharmaceutically acceptable salt thereof and (b) aflibercept, wherein (a) and (b) are administered in an amount that is effective for treating or preventing an ophthalmological disease or disorder (e.g., wet AMD), and wherein the administering occurs once every month, ± about seven days, for a first administration period of at least 3 consecutive months, followed by administering (a) and (b) for a second administration period at a frequency of at least every other month ± about seven days beginning at two months ± about seven days after the day of the last month of the first administration period on which (a) and (b) are administered.

In some embodiments, the subject has intraretinal or sub-retinal hemorrhage or a ≥ 50 µm increase in foveal intraretinal fluid at one month, ± about seven days, immediately following the second administration period. In some embodiments, the methods further comprise administering to the subject on each month ± about seven days, beginning on the month that immediately follows the second administration period (a) and (b) in an amount that is effective for treating or preventing wet AMD, until the subject's visual acuity measured on any two consecutive months that follow the 12 consecutive months is ≤ a five-ETDRS-letter difference in the subject's visual acuity measured on a month immediately preceding the first of the two consecutive months.

In some embodiments, the total number of months of treatment does not exceed 24.

Pharmaceutical compositions according to the invention may be formulated to release Antagonist A or another pharmaceutically acceptable salt thereof, a VEGF antagonist, or an anti-C5 agent, substantially immediately upon administration or at any predetermined time period after administration, using controlled release formulations. For example, a pharmaceutical composition can be provided in sustained-release form. The use of immediate or sustained release compositions depends on the nature of the condition being treated. If the condition consists of an acute disorder, treatment with an immediate release form can be utilized over a prolonged release composition. For certain preventative or long-term treatments, a sustained released composition can also be appropriate.

Administration of one or both of the antagonists of, or an anti-C5 agent, in controlled release formulations can be useful where the antagonist, either alone or in combination, has (i) a narrow therapeutic index (*e.g*., the difference between the plasma concentration leading to harmful side effects or toxic reactions and the plasma concentration leading to a therapeutic effect is small; generally, the therapeutic index, TI, is defined as the ratio of median lethal dose (LD₅₀) to median effective dose (ED₅₀)); (ii) a narrow absorption window in the gastro-intestinal tract; or (iii) a short biological half-life, so that frequent dosing during a day is required in order to sustain the plasma level at a therapeutic level.

Many strategies can be pursued to obtain controlled release in which the rate of release outweighs the rate of degradation or metabolism of the therapeutic antagonist. For example, controlled release can be obtained by the appropriate selection of formulation parameters and ingredients, including, e.g., appropriate controlled release compositions and coatings. Examples include single or multiple unit tablet or capsule compositions, oil solutions, suspensions, emulsions, microcapsules, microspheres, nanoparticles, patches, and liposomes. Methods for preparing such sustained or controlled release formulations are well known in the art.

Antagonist A or another pharmaceutically acceptable salt thereof, the VEGF antagonist, or the anti-C5 agent can also be delivered using a drug-delivery device such as an implant. Such implants can be biodegradable and/or biocompatible, or can be non-biodegradable. The implants can be permeable to Antagonist A or another pharmaceutically acceptable salt thereof, the VEGF antagonist, or the anti-C5 agent. Ophthalmic drug delivery devices can be inserted into a chamber of the eye, such as the anterior or posterior chamber or can be implanted in or on the sclera, choroidal space, or an avascularized region exterior to the vitreous. In one embodiment, the implant can be positioned over an avascular region, such as on the sclera, so as to allow for transcleral diffusion of Antagonist A or another pharmaceutically acceptable salt thereof, the VEGF antagonist, or the anti-C5 agent to the desired site of treatment, e.g., the intraocular space and macula of the eye. Furthermore, the site of transcleral diffusion can be proximal to a site of neovascularization such as a site proximal to the macula. Suitable drug delivery devices are described, for example, in U.S. Publication Nos. 2008/0286334; 2008/0145406; 2007/0184089; 2006/0233860; 2005/0244500; 2005/0244471; and 2005/0244462, and U.S. Pat. Nos. 6,808,719 and 5,322,691, the contents of each of which is herein incorporated by reference in its entirety.

In one embodiment, the implant comprises Antagonist A or another pharmaceutically acceptable salt thereof and/or VEGF antagonist dispersed in a biodegradable polymer matrix. The matrix can comprise PLGA (polylactic acid-polyglycolic acid copolymer), an ester-end capped polymer, an acid end-capped polymer, or a mixture thereof. In another embodiment, the implant comprises Antagonist A or another pharmaceutically acceptable salt thereof and/or a VEGF antagonist, a surfactant, and lipophilic compound. The lipophilic compound can be present in an amount of about 80-99% by weight of the implant. Suitable lipophilic compounds include, but are not limited to, glyceryl palmitostearate, diethylene glycol monostearate, propylene glycol monostearate, glyceryl monostearate, glyceryl monolinoleate, glyceryl monooleate, glyceryl monopalmitate, glyceryl monolaurate, glyceryl dilaurate, glyceryl monomyristate, glyceryl dimyristate, glyceryl monopalmitate, glyceryl dipalmitate, glyceryl monostearate, glyceryl distearate, glyceryl monooleate, glyceryl dioleate, glyceryl monolinoleate, glyceryl dilinoleate, glyceryl monoarachidate, glyceryl diarachidate, glyceryl monobehenate, glyceryl dibehenate, and mixtures thereof. In another embodiment, the implant comprises Antagonist A or another pharmaceutically acceptable salt thereof and/or a VEGF antagonist housed within a hollow sleeve. The PDGF antagonist or VEGF antagonist, or both, are delivered to the eye by inserting the sleeve into the eye, releasing the implant from the sleeve into the eye, and then removing the sleeve from the eye. An example of this delivery device is described in U.S. Publication No. 2005/0244462, which is hereby incorporated by reference in its entirety.

In one embodiment, the implant is a flexible ocular insert device adapted for the controlled sustained release of Antagonist A or another pharmaceutically acceptable salt thereof and/or a VEGF antagonist into the eye. In one embodiment, the device includes an elongated body of a polymeric material in the form of a rod or tube containing Antagonist A or another pharmaceutically acceptable salt thereof, VEGF antagonist or both, and with at least two anchoring protrusions extending radially outwardly from the body. The device may have a length of at least 8 mm and the diameter of its body portion including the protrusions does not exceed 1.9 mm. The sustained release mechanism can, for example, be by diffusion or by osmosis or bioerosion. The insert device can be inserted into the upper or lower formix of the eye so as to be independent of movement of the eye by virtue of the formix anatomy. The protrusions can be of various shapes such as, for example, ribs, screw threads, dimples or bumps, truncated cone-shaped segments or winding braid segments. In a further embodiment, the polymeric material for the body is selected as one which swells in a liquid environment. Thus a device of smaller initial size can be employed. The insert device can be of a size and configuration such that, upon insertion into the upper or lower formix, the device remains out of the field of vision so as to be well retained in place and imperceptible by a recipient over a prolonged period of use. The device can be retained in the upper or lower formix for 7 to 14 days or longer. An example of this device is described in U.S. Pat. No. 5,322,691, which is hereby incorporated by reference in its entirety.

### Kits

The invention relates to kits comprising one or more pharmaceutical compositions and instructions for use. At least two antagonists can be formulated together or in separate compositions and in individual dosage amounts. The antagonists are also useful when formulated as pharmaceutically acceptable salts. In one embodiment, the kits comprise a composition comprising Antagonist A or another pharmaceutically acceptable salt thereof and a pharmaceutically acceptable carrier or vehicle and another composition comprising a VEGF antagonist and a pharmaceutically acceptable carrier or vehicle. In another embodiment, the kits comprise a composition comprising a VEGF antagonist, Antagonist A or another pharmaceutically acceptable salt thereof and a pharmaceutically acceptable carrier or vehicle. Each of the kits' compositions can be contained in a container. In some embodiments, the kits comprise an anti-C5 agent.

The kits can comprise (1) an amount of Antagonist A or another pharmaceutically acceptable salt thereof and a pharmaceutically acceptable carrier, vehicle, or diluent in a first unit dosage form; (2) an amount of a VEGF antagonist and a pharmaceutically acceptable carrier, vehicle, or diluent in a second unit dosage form; and (3) a container. The container can be used to separate components and include, for example, a divided bottle or a divided foil packet. The separate antagonist compositions may also, if desired, be contained within a single, undivided container. In some embodiments, the kits comprise an anti-C5 agent.

The kits can also comprise directions for the administration of the antagonists. The kits are particularly advantageous when the separate components are administered in different dosage forms, are administered at different dosage levels, or when titration of the individual antagonists is desired.

### EXAMPLES

### Example 1: Antagonist A and Ranibizumab Combination Therapy for Treating Subfoveal Neovascular Lesions Secondary to Neovascular Age-Related Macular Degeneration (NVAMD)

In this study, 449 subjects with subfoveal neovascular lesions secondary to NVAMD received six monthly intravitreous injections of Antagonist A given in combination with ranibizumab (administered as Lucentis^{®}, commercially available from Genentech, South San Francisco, CA). Antagonist A was injected as the formulation shown in Table 12. The primary efficacy endpoint in the study was the mean change in visual acuity from baseline at the week 24 visit. As pre-specified in the analysis plan, the Hochberg procedure (Hochberg, Y. (1988). A sharper Bonferroni procedure for multiple tests of significance. Biometrika. 75, 800-802) was employed to account for multiple dose comparisons.

The subjects were randomized in a 1:1:1 ratio to the groups shown in Table 13.

**Table 12- Antagonist A Formulation**

| | | | **30 mg/mL** | |
|---|---|---|---|---|
| **Name of Ingredient** | **Reference to Standards** | Function | **Solution Composition** | **Percent (w/v)** |
| Antagonist A | In-house standard | Drug substance | 30.0 mg | 3% |
| Monobasic Sodium Phosphate Monohydrate | USP/Ph. Eur | pH buffering agent | 0.3 mg | 0.03% |
| Dibasic Sodium Phosphate Heptahydrate | USP/Ph. Eur | pH buffering agent | 2.1 mg | 0.2% |
| Sodium Chloride | USP/Ph. Eur | Tonicity adjuster | 9.0 mg | 0.9% |
| Hydrochloric Acid | NF/Ph. Eur | pH adjuster | As needed | |
| Sodium Hydroxide | NF/Ph. Eur | pH adjuster | As needed | |
| Water for Injection | USP/Ph. Eur | Diluent | q.s. | 95.9% |
| Nitrogen | NF/Ph. Eur | Inert gas overlay | --- | --- |
| Total Volume | | | 1 ml | |
| Volume in Final Drug Product Presentation | | | 230 microliters | |

**Table 13- Antagonist A and Ranibizumab Combination Therapy for Subfoveal Neovascular Lesions Secondary to NVAMD Treatment Groups**

| **Group No.** | **Group Name** | **Treatment Regimen** |
|---|---|---|
| 1 | Combination Therapy (0.3 mg) | Subjects were administered 0.3 mg/eye of Antagonist A and 0.5 mg/eye of Lucentis^{®} |
| 2 | Combination Therapy (1.5 mg) | Subjects were administered 1.5 mg/eye of Antagonist A and 0.5 mg/eye of Lucentis^{®} |
| 3 | Ranibizumab Monotherapy | Subjects were administered Antagonist A Sham and 0.5 mg/eye of Lucentis^{®} |

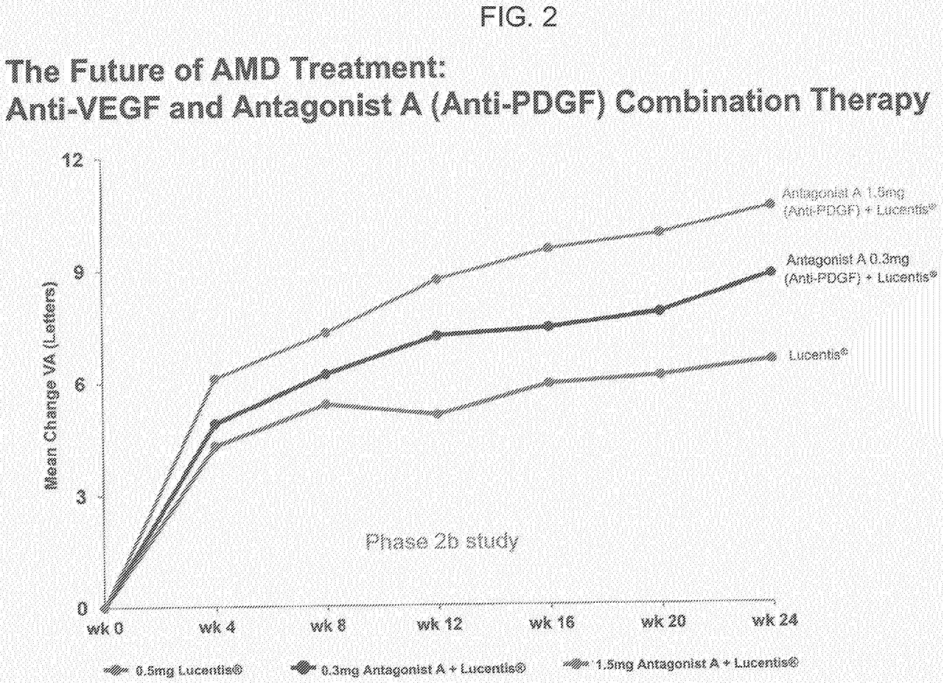

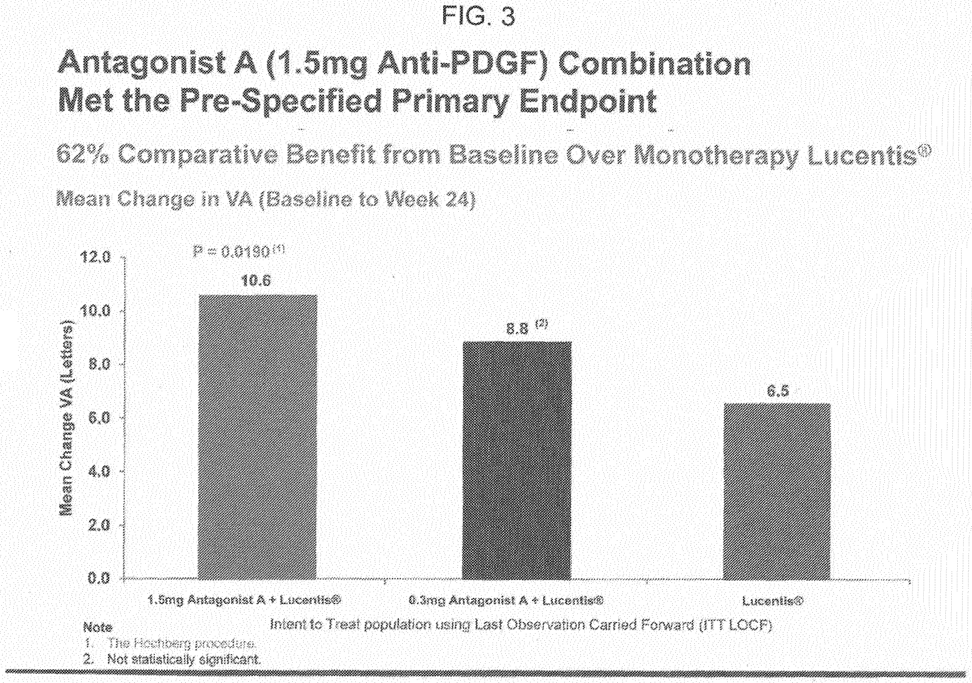

Combination therapy proved superior in terms of mean visual gain when compared to eyes that were treated with anti-VEGF monotherapy. Subjects treated with Lucentis^{®} and either 1.5 mg/eye or 0.3 mg/eye Antagonist A showed an increase in visual acuity compared with those treated with Lucentis^{®} alone **(****FIG. 2****).** The combination of 1.5 mg/eye of Antagonist A and 0.5 mg of Lucentis^{®} met the pre-specified, alpha protected primary endpoint of superiority in mean change of visual acuity gain compared to ranibizumab monotherapy from baseline to 24 weeks (10.6 ETDRS letters at week 24, compared to 6.5 letters, p=0.019, representing a 62% additional benefit). **(****FIG. 3****)** Subjects treated with Lucentis^{®} and either 1.5mg or 0.3 mg Antagonist A showed a 62% comparative benefit from baseline compared to treatment with Lucentis^{®} alone.

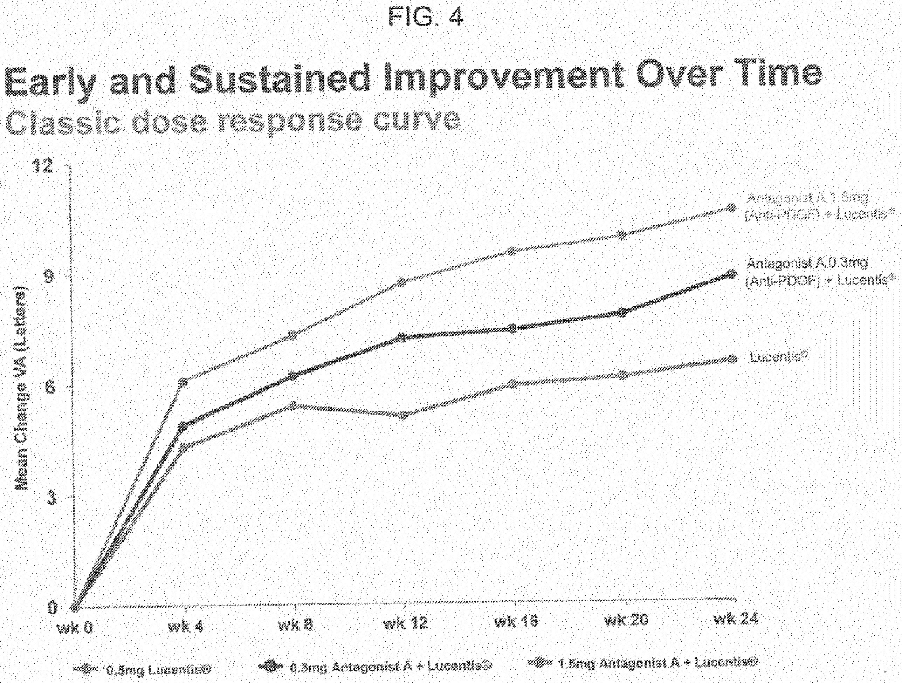

In addition, the mean change in vision over time demonstrated the benefit of combination therapy at each measured time point over 24 weeks. **(****FIG. 4****)** That benefit was sustained during the study and demonstrated increasing differentiation of the curves at study closure.

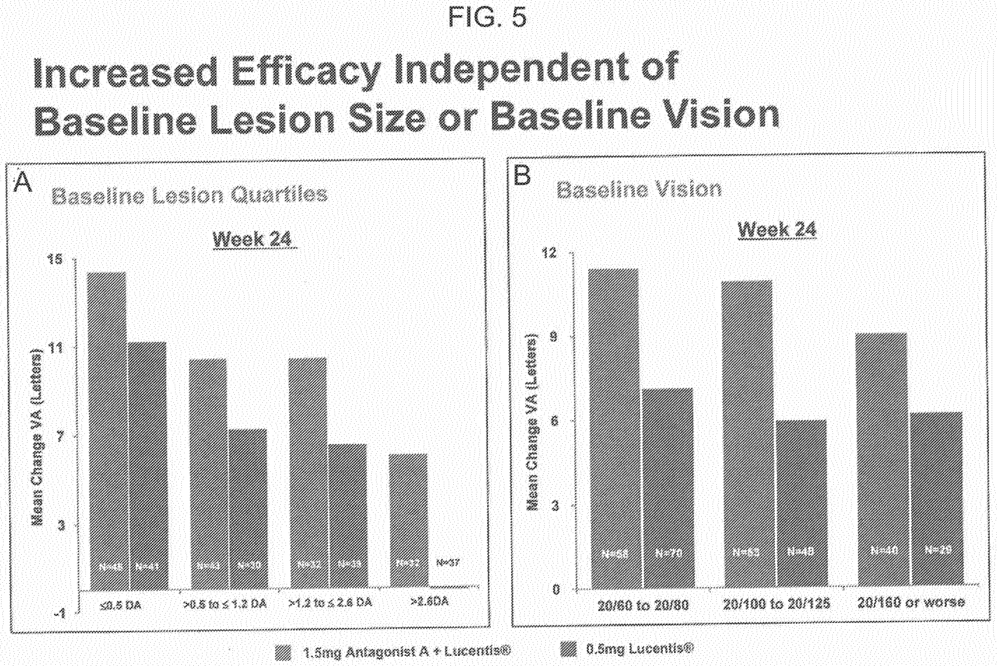

Treatment with 0.5 mg of Lucentis^{®} and either 1.5 mg or 0.3 mg Antagonist A in wet AMD patients also had increased efficacy as compared to patients treated with Lucentis^{®} alone, independent of baseline lesion size or vision. **(****FIGS. 5A and 5B****)**

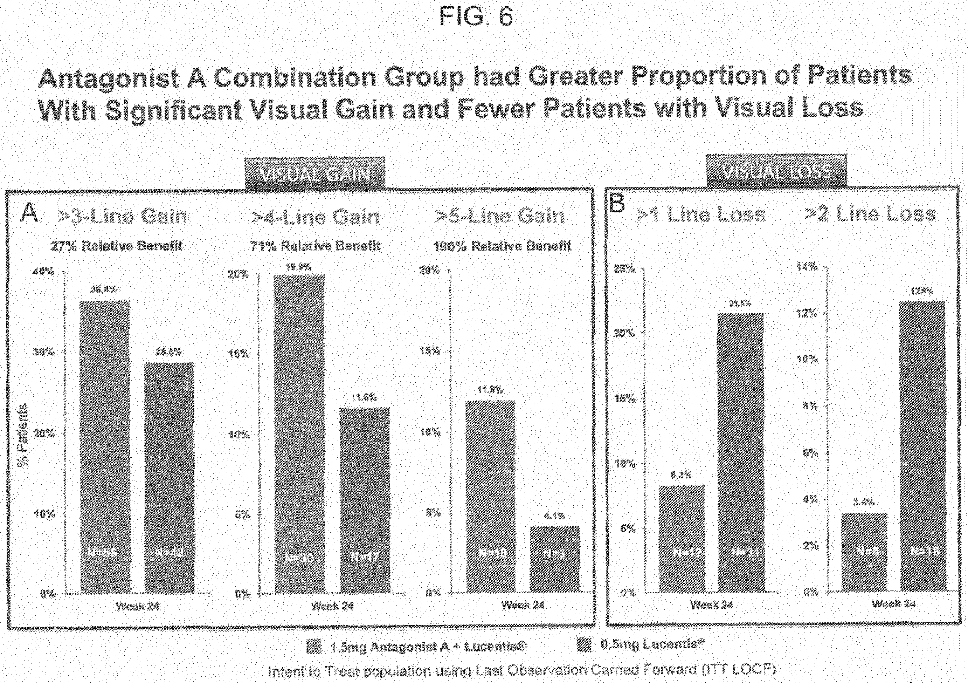

A greater percentage of subjects in the Combination Therapy (1.5mg) group achieved enhanced visual outcomes compared to those in the Ranibizumab Monotherapy group with respect to multiple treatment endpoints at week 24, as shown in **FIG. 6A****,** and Table 14.

**Table 14- Percentage of Subjects in the Combination Therapy (1.5 mg) Group and Ranibizumab Monotherapy Group with Visual Acuity Improvement**

| | **Percentage of Patients** | |
|---|---|---|
| **Treatment Endpoint** | Combination Therapy (1.5 mg) | Ranibizumab Monotherapy |
| >3-lines of visual acuity improvement | 36.4% | 28.6% |
| >4-lines of visual acuity improvement | 19.9% | 11.6% |
| >5-lines of visual acuity improvement | 11.9% | 4.1% |
| ≥ 20/40 vision after treatment | 37.0% | 31.9% |
| ≥ 20/25 vision after treatment | 12.3% | 5.6% |

Moreover, fewer subjects in the Combination Therapy (1.5 mg) group demonstrated a loss of visual acuity as compared to the number of subjects in the Ranibizumab Monotherapy group at week 24, as shown in **FIG. 6B** and Table 15.

**Table 15- Percentage of Subjects in the Combination Therapy (1.5 mg) Group and Ranibizumab Monotherapy Group with Visual Acuity Loss**

| | **Percentage of Patients** | |
|---|---|---|
| **Treatment Endpoint** | Combination Therapy (1.5 mg) | Ranibizumab Monotherapy |
| ≥1-lines of visual acuity loss | 8.3% | 21.5% |
| ≥2-lines of visual acuity loss | 3.4% | 12.5% |
| ≤ 20/125 vision after treatment | 19.2 % | 27.8% |
| ≤ 20/200 vision after treatment | 10.3% | 13.9% |

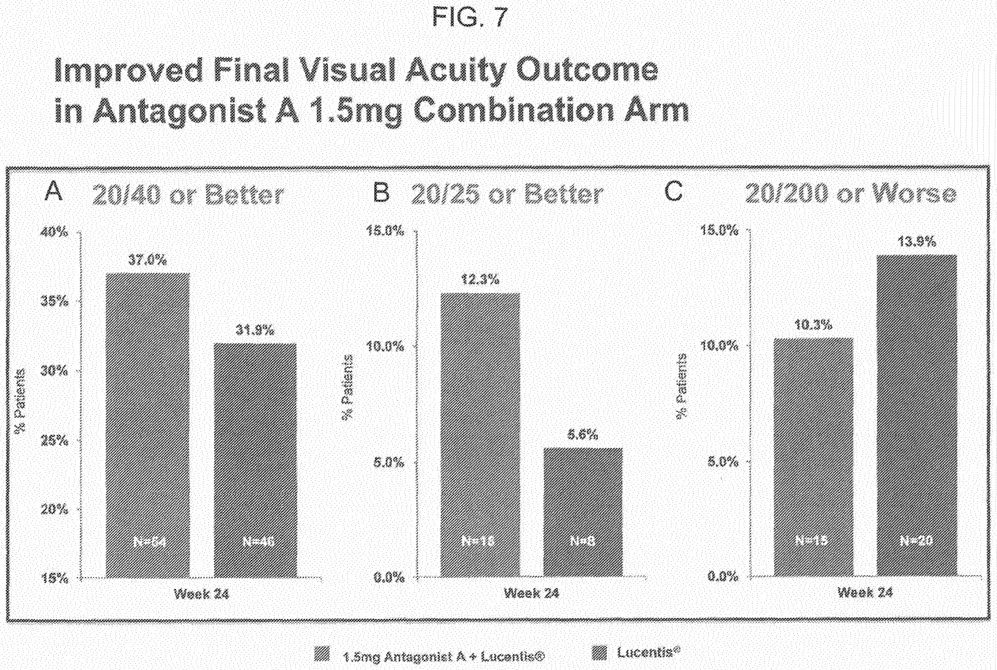

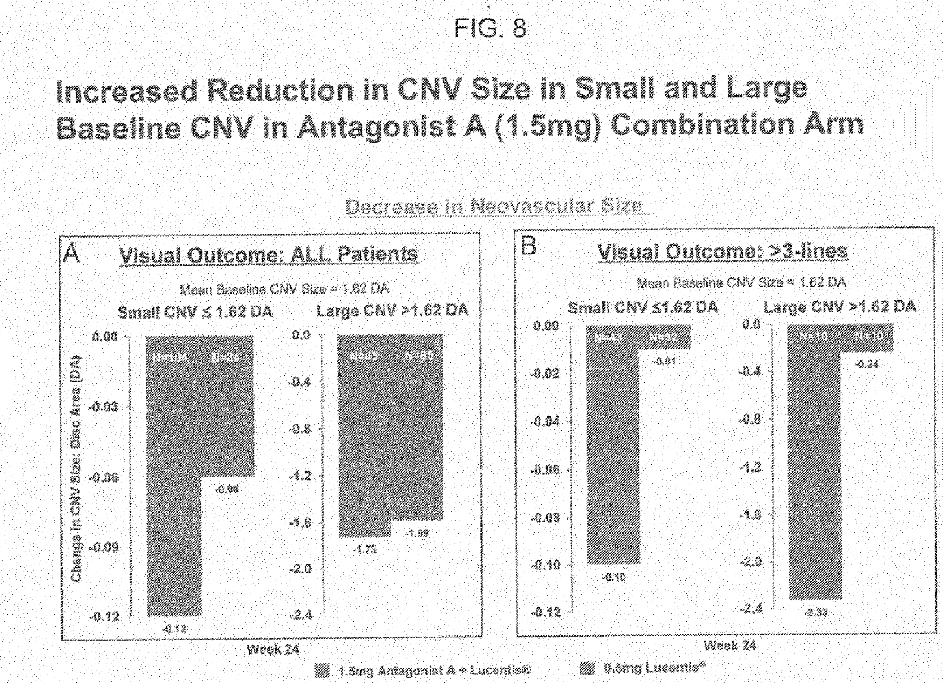

Subjects treated with Lucentis^{®} and 1.5 mg Antagonist A showed improved final visual acuity compared to patients treated with Lucentis^{®} monotherapy. **(****FIG. 7****)** Subjects in the Combination Therapy (1.5 mg) group also showed increased reduction in CNV size in small and large baseline CNV as compared to subjects in the Ranibizumab Monotherapy group **(****FIGS. 8A and 8B****).**

Combination therapy was well tolerated. There were no events of endophthalmitis, retinal detachment, retinal tear or iatrogenic traumatic cataract after a total of 4431 intravitreal injections (1776 administrations of Antagonist A and 2655 administrations of Lucentis^{®}). As expected, mean intraocular pressure (IOP) increased after each intravitreal injection consistent with a volume effect. However, mean IOP in all arms returned to pre-injection levels at the next visit, including at the end of the study. The systemic safety profile of combination therapy was similar to that of ranibizumab monotherapy.

The results of the trial show statistically significant superior efficacy of the combination treatment with Antagonist A and ranibizumab over Lucentis^{®} (ranibizumab) monotherapy for the treatment of wet AMD.

### Example 2: ARC1905 for the Treatment of Wet AMD

Forty-three patients with subfoveal neovascular AMD received six monthly administrations of ARC1905 (0.3 mg/eye, 1 mg/eye or 2 mg/eye) in combination with Lucentis. The mean change in visual acuity at week 24 was an increase of +13.6, +11.7 and +15.3 letters at the doses of 0.3 mg, 1 mg and 2 mg, respectively. Furthermore, 46%, 47% and 60% of patients gained 3 or more lines of visual acuity at the doses of 0.3 mg, 1 mg, and 2 mg, respectively.

### Example 3: ARC1905 for the Treatment and Prevention of Dry AMD

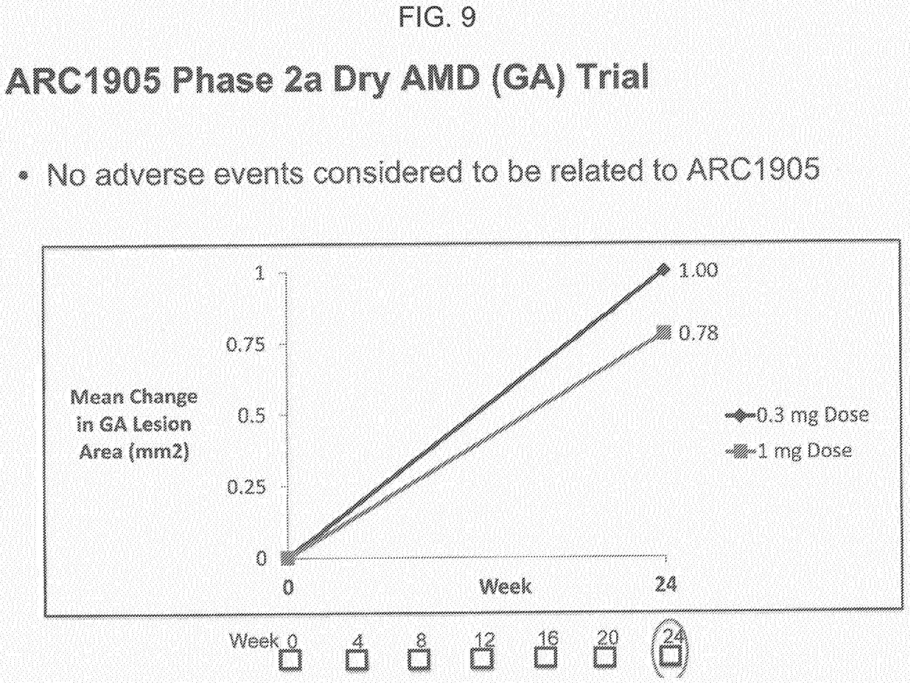

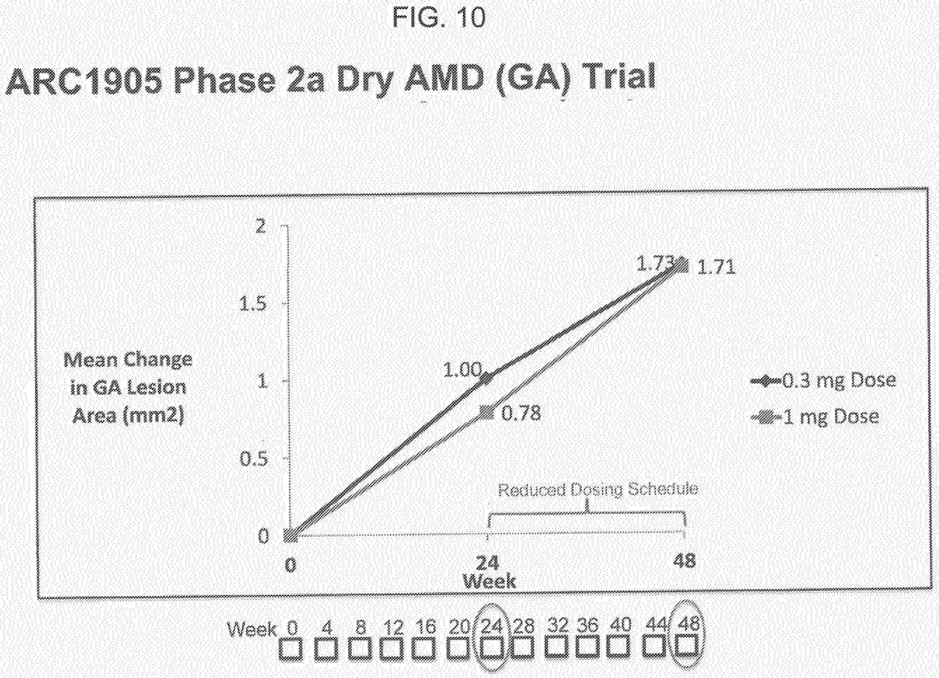

Forty-seven patients with dry AMD were enrolled to receive five intravitreal injections of either 0.3 mg/eye or 1.0 mg/eye of ARC1905 over a 36-week treatment period. **FIG. 9** shows the mean change in geographic atrophy (GA) lesion area in dry AMD patients measured at week 24 in patients treated with either 0.3 mg or 1.0 mg doses of ARC1905 at weeks 0, 4, and 8, **FIG. 10** shows the mean change in GA lesion in dry AMD patients measured at week 24 and week 48 in patients treated with either 0.3 mg or 1.0 mg doses of ARC 1905 at weeks 0, 4, 8, 24, and 36. The results show a dose-dependent reduction in growth of the GA lesion, indicating ARC 1905 can slow the progression of GA in non-exudative type AMD patients

### Example 4: Visual Acuity Testing using ETDRS Chart

Best-corrected visual acuity is measured using standard charts, lighting, and procedures. Best correction is determined by careful refraction at that visit.

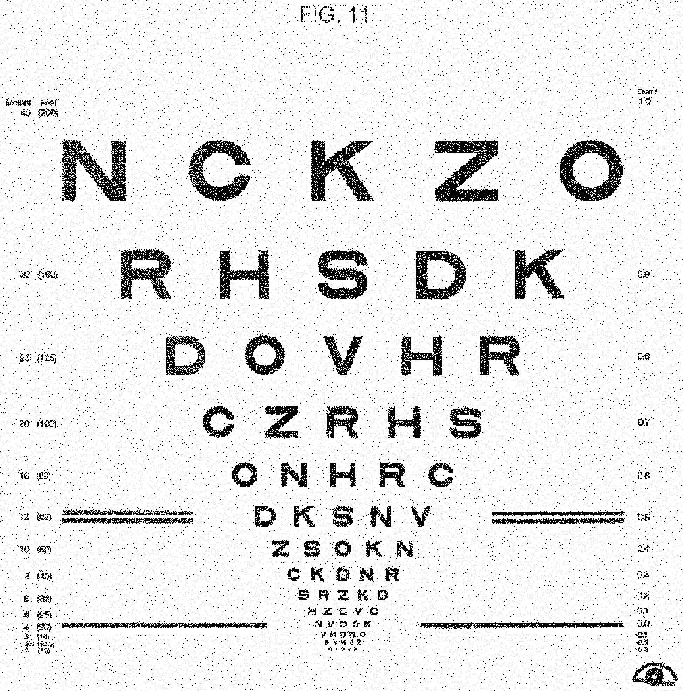

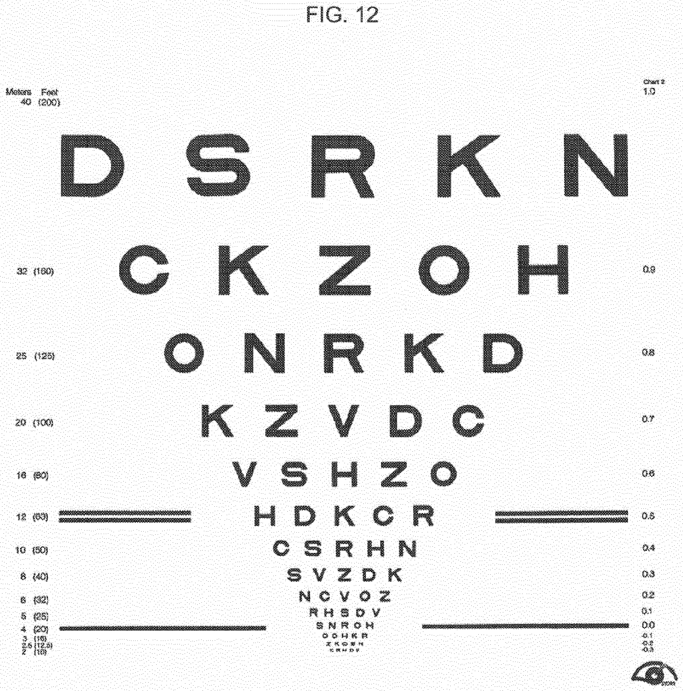

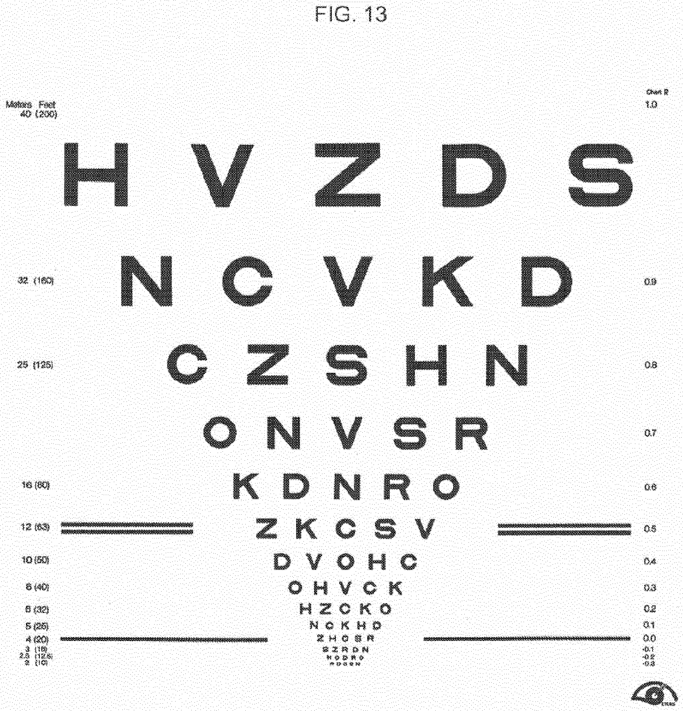

Chart 1 **(****FIG. 11****)** is used for testing the visual acuity of the right eye. Chart 2 **(****FIG. 12****)** is used for testing the left eye. Chart R **(****FIG. 13****)** is used for testing refraction. Subjects do not see any of the charts before the examination.

A distance of 4 meters is between the subject's eyes and the visual acuity chart. With the box light off, not more than 15 foot-candles of light (161.4 Lux) fall on the center of the chart. To measure the amount of light, the room is set up for visual acuity testing, but with the box light off. The light meter is placed at the fourth line from the top of the chart, with its back against the chart and the reading is taken. If more than one lane is available for testing visual acuity, the visual acuity of an individual subject should be measured in the same lane at each visit. If different lanes are used to test visual acuity, they each meet the same standards.

Retroilluminated ETDRS charts are used. The illuminator box is either wall-mounted or mounted on a stand (available from Lighthouse Low Vision Services). The light box is mounted at a height such that the top of the third row letter is 49 ± 2 inches from the floor.

The visual acuity light box is equipped with two 20-watt fluorescent tubes (available from General Electric Cool Daylight) and a ballast which partially covers the tubes. Because the illumination of fluorescent tubes generally diminishes by 5 percent during the first 100 hours and by another 5 percent during the next 2000 hours, new tubes are kept on for 4 days (96 hours) continuously, and replaced once a year.

A sticker is placed on the back of the light box, indicating the date on which the present tubes were installed. A spare set of burned in bulbs is available.

Each tube is partly covered by a 14-inch fenestrated sleeve, which is open in the back. This serves as a baffle to reduce illumination. Each sleeve is centered on the tube with the opening towards the back.

All eyes are tested at 4 meters first, even if the refraction was performed at 1 meter. The subject is seated comfortably directly in front of the chart so that the eyes remain at the 4 meter distance. Testing begins with the right eye. The subject's left eye is occluded. A folded tissue or eye pad lightly taped over the eye behind the trial frame serves as an effective occluder that allows eccentric fixation without inadvertent use of the covered eye. After testing the right eye, occlusion of the right eye is done before Chart 2 is put up for testing the left eye.

The lens correction from the subjective refraction is in the trial frame worn by the subject.

The subject is asked to read the letters slowly, approximately one letter per second. The subject is told that only one chance is given to read each letter on the chart. If the subject is unsure about the identity of the letter, then the subject is encouraged to guess.

The subject begins by reading the top line of the chart and continue reading every letter on each smaller line, from left to right on each line. The examiner circles every correct letter read and totals each line and the whole column (0 if no letters are correct) on the data collection form. An X is put through letters read incorrectly. Letters, for which no guess was attempted, are not circled. When a subject reaches a level where he/she cannot guess, the examiner may stop the test provided that the subject has made errors on previous guesses, which is a clear indication that the best visual acuity has been obtained.

When a subject cannot read at least 20 letters on the chart at 4.0 meters, the subject is tested at 1.0 meter. The distance from the subject to the chart should be measured again using the rigid one meter stick. The distance is measured from the outer canthus to the center of the fourth letter (right eye) or the second letter (left eye) of the third line of the chart. The spherical correction in the trial frame should be changed by adding +0.75 to correct for the closer test distance. The subject may fixate eccentrically or turn or shake his/her head to improve visual acuity. If this is done, the examiner ensures that the fellow eye remains occluded both centrally and peripherally and that the subject does not move forward in the chair. Particular care should be taken to ensure the subject does not move forward when testing at 1 meter. The subject is reminded to blink.

The examiner does not tell the subject if a letter was identified correctly. The subject may be encouraged by neutral comments, such as "good", "next", and "OK".

The examiner does not stand close to the chart during testing. The examiner's attention is focused on the subject and the data collection form. If the subject has difficulty locating the next line to read, the examiner may go up to the chart and point to the next line to be read, and then moves away from the chart.

When it is possible to measure the visual acuity of the eye at 4.0 meters (i.e., 20 or more letters read at 4 meters), the visual acuity score for that eye is recorded as the number of letters correct plus 30. The subject gets credit for the 30 1M letters even though they did not have to read them. Otherwise, the visual acuity score is the number of letters read correctly at 1.0 meter plus the number, if any, read at 4M. If no letters are read correctly at either 4.0 meters or 1 meter, then the visual acuity score is recorded as 0.

### INCORPORATION BY REFERENCE

All publications and patent applications disclosed in this specification are herein incorporated by reference to the same extent as if each individual publication or patent application was specifically and individually indicated to be incorporated by reference.
Further disclosures are provided by way of the following numbered embodiments:
1. A method for treating or preventing wet age-related macular degeneration (wet AMD), comprising administering to a subject in need thereof (a) Antagonist A or another pharmaceutically acceptable salt thereof and (b) an VEGF antagonist, wherein (a) and (b) are administered in an amount that is effective for treating or preventing wet AMD, and wherein the administering occurs once every month, ± about seven days, for a first administration period of at least 3 consecutive months, followed by administering (a) and (b) for a second administration period at a frequency of at least every other month ± about seven days beginning at two months ± about seven days after the day of the last month of the first administration period on which (a) and (b) are administered.
2. The method of embodiment 1, wherein (a) and (b) are administered within about 60 minutes of each other.
3. The method of embodiment 1, wherein the VEGF antagonist is ranibizumab, bevacizumab, pegaptanib sodium, ESBA 1008 or aflibercept.
4. The method of embodiment 1, wherein the VEGF antagonist is ranibizumab or bevacizumab, wherein (a) and (b) are administered at a frequency of once every month ± about seven days during the second administration period and wherein the second administration period is at least about nine months.
5. The method of embodiment 4, further comprising measuring the subject's visual acuity.
6. The method of embodiment 5, further comprising administering to the subject (a) and (b) in an amount that is effective for treating or preventing wet AMD, until the subject's visual acuity on the last two of any three consecutive months is ≤ a five-ETDRS-letter difference from the subject's visual acuity on the first of the three consecutive months.
7. The method of embodiment 5, further comprising administering to the subject (a) and (b) every other month in an amount that is effective for treating or preventing wet AMD, wherein the subject's visual acuity on the last two of any three consecutive months is ≤ a five-ETDRS-letter difference from the subject's visual acuity on the first of the three consecutive months.
8. The method of embodiment 7, further comprising administering to the subject (a) and (b) every month in an amount that is effective for treating or preventing wet AMD, until the subject's visual acuity on the last two of any three consecutive months is ≤ a five-ETDRS-letter difference from the subject's visual acuity on the first of the three consecutive months.
9. The method of embodiment 1, wherein the VEGF antagonist is aflibercept.
10. The method of embodiment 1, wherein the total number of months does not exceed 24.
11. The method of embodiment 4, wherein the subject has intraretinal or sub-retinal hemorrhage or a ≥ 50 µm increase in foveal intraretinal fluid at one month, ± about seven days, immediately following the second administration period.
12. The method of embodiment 11, further comprising: administering to the subject on each month ± about seven days, beginning on the month that immediately follows the second administration period (a) and (b) in an amount that is effective for treating or preventing wet AMD, until the subject's visual acuity on the last two of any three consecutive months that follow the 12 consecutive months is ≤ a five-ETDRS-letter difference from the subject's visual acuity on the first of the three consecutive months.
13. The method of embodiment 12, wherein the total number of months does not exceed 24.
14. The method of embodiment 1, wherein Antagonist A or another pharmaceutically acceptable salt thereof is administered intravitreally in an amount of about 1.5 mg/eye.
15. The method of embodiment 4, wherein the VEGF antagonist is bevacizumab and is administered intravitreally in an amount of about 1.25 mg/eye.
16. The method of embodiment 9, wherein the VEGF antagonist is administered intravitreally in an amount of about 2 mg/eye.
17. The method of embodiment 4, wherein the VEGF antagonist is ranibizumab and is administered intravitreally in an amount of about 0.5 mg/eye.
18. The method of embodiment 1, further comprising administering an anti-C5 agent.
19. The method of embodiment 1, further comprising administering (a) and (b) on a month in which the subject has intraretinal or sub-retinal hemorrhage or a ≥ 50 µm increase in foveal intraretinal fluid.
20. A method for treating or preventing sub-retinal fibrosis, comprising administering to a subject in need thereof (a) Antagonist A or another pharmaceutically acceptable salt thereof in an amount that is effective for treating or preventing sub-retinal fibrosis.
21. The method of embodiment 20, further comprising administering to the subject (b) a VEGF antagonist, wherein (a) and (b) are administered in an amount that is effective for treating or preventing sub-retinal fibrosis.
22. The method of embodiment 20, wherein the subject has wet age-related macular degeneration (wet AMD).
23. The method of embodiment 22, wherein the sub-retinal fibrosis is associated with the wet AMD.
24. The method of embodiment 20, wherein administering Antagonist A or another pharmacetically acceptable salt thereof results in a decrease in the size of sub-retinal hyper-reflective material (SHRM) as evidenced by spectral domain optical coherence tomography (SD-OCT) or results in stabilization of the subject's vision.
25. The method of embodiment 20, wherein Antagonist A or another pharmaceutically acceptable salt thereof is administered intravitreally in an amount of about 1.5 mg/eye.
26. The method of embodiment 21, wherein the VEGF antagonist is bevacizumab, ranibizumab, aflibercept, pegaptanib sodium or ESBA1008.
27. The method of embodiment 26, wherein the VEGF antagonist is bevacizumab and is administered intravitreally in an amount of about 1.25 mg/eye.
28. The method of embodiment 26, wherein the VEGF antagonist is aflibercept and is administered intravitreally in an amount of about 2 mg/eye.
29. The method of embodiment 26, wherein the VEGF antagonist is ranibizumab and is administered intravitreally in an amount of about 0.5 mg/eye.
30. The method of embodiment 21, further comprising administering an anti-C5 agent.
31. A method for treating or preventing von Hippel-Lindau (VHL) disease, comprising administering to a subject in need thereof Antagonist A or another pharmaceutically acceptable salt thereof in an amount that is effective for treating or preventing VHL disease.
32. The method of embodiment 31, further comprising administering a VEGF antagonist.
33. The method of embodiment 31, wherein Antagonist A or another pharmaceutically acceptable salt thereof is administered intravitreally in an amount of about 1.5 mg/eye.
34. The method of embodiment 32, wherein the VEGF antagonist is bevacizumab and is administered intravitreally in an amount of about 1.25 mg/eye.
35. The method of embodiment 32, wherein the VEGF antagonist is aflibercept and is administered intravitreally in an amount of about 2 mg/eye.
36. The method of embodiment 32, wherein the VEGF antagonist is ranibizumab and is administered intravitreally in an amount of about 0.5 mg/eye.
37. The method of embodiment 32, further comprising administering an anti-C5 agent.

## Claims

1. An anti-C5 agent for use in reducing the growth of a geographic atrophy (GA) lesion in a subject in need thereof, wherein the anti-C5 agent is a C5 specific aptamer comprising the nucleotide sequence of SEQ ID NO: 26 conjugated to a polyethylene glycol moiety via a linker, or a pharmaceutically acceptable salt thereof,
wherein "reducing the growth of a GA lesion" is reducing the growth of a GA lesion over time as compared to that in a subject not receiving the anti-C5 agent, or the pharmaceutically acceptable salt thereof.

2. The anti-C5 agent for use according to claim 1, wherein the C5 specific aptamer has the following structure: 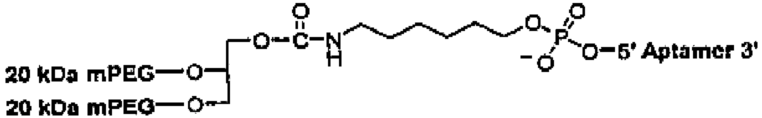
or a pharmaceutically acceptable salt thereof,
where Aptamer = fCmGfCfCGfCmGmGfUfCfUfCmAmGmGfCGfCfUmGmAmGfUfCfUmGmAmGfUfUfUA fCfCfUmGfCmG-3T (SEQ ID NO: 26), wherein fC and fU = 2' fluoro nucleotides, mG and mA = 2'-OMe nucleotides, all other nucleotides are 2'-OH, and 3T indicates an inverted deoxythymidine.

3. The anti-C5 agent for use according to claim 2, wherein the C5 specific aptamer has the following structure: 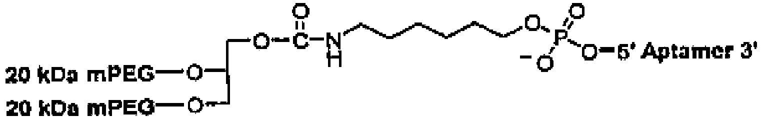 where Aptamer = fCmGfCfCGfCmGmGfUfCfUfCmAmGmGfCGfCfUmGmAmGfUfCfUmGmAmGfUfUfUA fCfCfUmGfCmG-3T (SEQ ID NO: 26), wherein fC and fU = 2' fluoro nucleotides, mG and mA = 2'-OMe nucleotides, all other nucleotides are 2'-OH, and 3T indicates an inverted deoxythymidine.

4. The anti-C5 agent for use according to any preceding claim, wherein the anti-C5 agent is administered to the subject weekly, biweekly, monthly or quarterly in an amount of 2 mg/eye.

5. The anti-C5 agent for use according to any preceding claim, wherein the anti-C5 agent is administered intraocularly to the subject via intravitreal injection into the eye.

6. The anti-C5 agent for use according to any preceding claim, wherein the anti-C5 agent is administered monthly.

7. The anti-C5 agent for use according to any preceding claim, wherein the growth of the GA lesion in the subject in need thereof is reduced by at least 20% as compared to a subject who is not administered the anti-C5 agent.

8. The anti-C5 agent for use according to any preceding claim, wherein the subject is human.
